(19) Europäisches Patentamt
European Patent Office
Office européen des brevets

(11) EP 0 474 561 B1

(12) FASCICULE DE BREVET EUROPEEN

(45) Date de publication et mention de la délivrance du brevet:
**09.12.1998 Bulletin 1998/50**

(21) Numéro de dépôt: **91402382.5**

(22) Date de dépôt: **05.09.1991**

(51) Int Cl.6: **C07D 295/13**, A61K 31/445, C07D 409/04, C07D 401/06, C07D 333/38, C07D 417/12, C07D 401/12, C07D 405/12, C07D 239/42, C07D 409/06, C07D 409/12, C07D 211/58, C07D 211/64, C07D 211/52, C07D 211/48, C07D 211/26, C07D 211/22, C07D 211/14

(54) **Arylalkylamines, procédé pour leur préparation et compositions pharmaceutiques les contenant**

Arylalkylamine, Verfahren zur Herstellung und diese enthaltende Arzneimittel

Arylalkylamines, process for their preparation and pharmaceutical compositions containing them

(84) Etats contractants désignés:
**AT BE CH DE DK ES FR GB GR IT LI LU NL SE**

(30) Priorité: **05.09.1990 FR 9011039**
**25.06.1991 FR 9107824**

(43) Date de publication de la demande:
**11.03.1992 Bulletin 1992/11**

(73) Titulaire: **SANOFI**
**75008 Paris (FR)**

(72) Inventeurs:
- **Emonds-Alt, Xavier**
  **F-34980 Combaillaux (FR)**
- **Goulaouic, Pierre**
  **F-34000 Montpellier (FR)**
- **Proietto, Vincenzo**
  **F-34680 Saint George d'Orques (FR)**
- **van Broeck, Didier**
  **F-34570 Murviel les Montpellier (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 261 842**
**EP-A- 288 352**
**EP-A- 325 406**
**EP-A- 428 434**
**WO-A-88/02362**
**BE-A- 701 994**
**DE-B- 1 112 514**

- **JOURNAL OF MEDICINAL CHEMISTRY vol. 13, no. 4, 1970, pages 747 - 748; A. DONETTI, E. MARAZZI-UBERTI: 'CENTRAL NERVOUS SYSTEM ACTIVITY OF ETHYL 1-NAPHTHYLALKYLCARBAMATES'**
- **Pharmacologie, R. Schorderet, pages 519,675**
- **Decision making in drug research, pages 173-187**

EP 0 474 561 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouveaux dérivés aromatiques substitués par un groupement aminé et par diverses fonctions esters, amines ou amides.

La présente invention concerne également l'utilisation des composés selon l'invention dans des compositions à usage thérapeutique et plus particulièrement dans les phénomènes pathologiques qui impliquent le système des neurokinines.

Des ligands endogènes aux récepteurs des neurokinines ont été décrits, telles la substance P (SP), la neurokinine A (NKA) (S.J. BAILEY et al., 1983, Substance P, P. Skrabanck ed., 16-17 Boole Press, Dublin) et la neurokinine B (NKB) (S.P. WATSON, Life Sciences, 1983, 25, 797-808).

Les récepteurs aux neurokinines ont été reconnus sur de nombreuses préparations et sont actuellement classés en trois types : $NK_1$, $NK_2$ et $NK_3$. Alors que la plupart des préparations étudiées jusqu'à maintenant présentent plusieurs types de récepteurs, tel l'iléon de cobaye ($NK_1$, $NK_2$ et $NK_3$), certaines d'entre elles n'en posséderaient qu'un seul, telles l'artère carotide de chien ($NK_1$), l'artère pulmonaire de lapin dépourvue d'endothélium ($NK_2$) et la veine porte de rat ($NK_3$) (D. REGOLI et al., Trends Pharmacol. Sci., 1988, 9, 290-295 et Pharmacology, 1989, 38, 1-15).

Une caractérisation plus précise des différents récepteurs est rendue possible par la synthèse récente d'agonistes sélectifs. Ainsi, la [$Sar^9$, Met-$(O_2)^{11}$] SP, la [$Nle^{10}$] $NKA_{4-10}$, et la [Me $Phe^7$] -NKB présenteraient une sélectivité respective pour les récepteurs $NK_1$, $NK_2$ et $NK_3$ (cf. D. REGOLI, 1988 et 1989 précédemment cité).

On a maintenant trouvé que certains dérivés aromatiques aminés et diversement substitués possèdent des propriétés pharmacologiques intéressantes, en tant qu'antagonistes des récepteurs de la neurokinine A et sont notamment utiles pour le traitement de toute pathologie neurokinine A dépendante.

Le récepteur $NK_2$ et la neurokinine A sont, par exemple, impliqués dans les inflammations neurogéniques des voies respiratoires (P.J. BARNES, Arch. Int. Pharmacodyn., 1990, 303, 67-82 et G.F. JOOS et al., Arch. Int. Pharmacodyn., 1990, 303, 132-146).

A ce jour, seuls des antagonistes peptidiques des récepteurs $NK_2$ ont été décrits. Une publication de C.A. MAGGI et al., Br. J. Pharmacol., 1990, 100, 588-592, décrit des peptides qui sont des antagonistes sélectifs des récepteurs $NK_2$.

La demande de brevet européen 0 347 802 décrit des dérivés peptidiques antagonistes de la neurokinine A utiles en tant qu'immunosuppresseurs, dans le traitement de l'arthrite, de l'asthme, de la douleur de l'inflammation, de l'hypermotilité gastrointestinale, de la maladie de Huntington, de psychoses, de l'hypertension, des migraines, de l'urticaire etc.

La demande de brevet européen 0 336 230 décrit également des dérivés peptidiques antagonistes de la substance P et de la neurokinine A utiles pour le traitement et la prévention de l'asthme.

Enfin la demande de brevet EP 0 428 434 décrits des dérivés non peptidiques antagonistes des récepteurs des neurokinines utiles pour le traitement de toute pathologie substance P et neurokine dépendante.

Ainsi, selon un de ses aspects, la présente invention concerne des composés aromatiques aminés diversement substitués de formule :

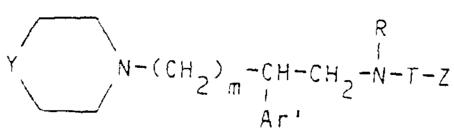

(I)

dans laquelle :

- Y représente - soit un groupe Cy-N dans lequel
  - . Cy représente un phényle non substitué ou substitué une ou plusieurs fois par l'un des substituants choisis parmi : l'hydrogène, un atome d'halogène, un hydroxyle, un alcoxy en $C_1$-$C_4$, un alkyle en $C_1$-$C_4$, un trifluorométhyle, lesdits substituants étant identiques ou différents, un groupe cycloalkyle en $C_3$-$C_7$ ; un groupe pyrimidinyle ou un groupe pyridyle ;
  - soit un groupe

$$Ar-(CH_2)_x-\overset{\overset{X}{|}}{C}$$

dans lequel

- . Ar représente un phényle non substitué ou substitué une ou plusieurs fois par l'un des substituants choisis parmi : l'hydrogène, un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;
- . x est zéro ou un ;
- . X représente un hydroxyle, un alcoxy en $C_1$-$C_4$ ; un hydroxyalkyle dans lequel l'alkyle est en $C_1$-$C_3$ ; un acyloxy en $C_1$-$C_4$ ; un phénacyloxy ; un carboxy ; un carbalcoxy en $C_1$-$C_4$ ; un cyano ; un aminoalkylène dans lequel l'alkylène est en $C_1$-$C_3$ ; un groupe -N-$(X_1)_2$ dans lequel les groupes $X_1$ représentent indépendamment l'hydrogène, un alkyle en $C_1$-$C_4$ ; un groupe

$$-NH-\underset{\underset{O}{\|}}{C}-Alk$$

dans lequel Alk représente un alkyle en $C_1$-$C_6$ ; un groupe

$$-Alk_1-NH-\underset{\underset{O}{\|}}{C}-Alk'_1$$

dans lequel $Alk_1$ est un alkylène en $C_1$-$C_3$
et $Alk'_1$ est un alkyle en $C_1$-$C_3$ ;
un acyle en $C_1$-$C_4$ ; un groupe -S-$X_2$ dans lequel $X_2$ représente l'hydrogène ou un groupe alkyle en $C_1$-$C_4$ ; ou bien X forme avec l'atome de carbone auquel il est lié et avec l'atome de carbone voisin dans l'hétérocycle une double liaison ;

- m est 2 ou 3 ;
- Ar' représente un phényle non substitué ou substitué une ou plusieurs fois par l'un des substituants choisis parmi : l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle, un indolyle ; un indolyle N substitué par un alkyle en $C_1$-$C_3$ ;
- R représente l'hydrogène, un alkyle en $C_1$-$C_6$;
- T représente un groupe choisi parmi

$$-\overset{\overset{O}{\|}}{C}- \qquad \text{et} \qquad -\overset{\overset{W}{\|}}{C}-NH-$$

W étant un atome d'oxygène ou de soufre, et

- Z représente soit l'hydrogène, soit M ou OM lorsque T représente le groupe

$$-\overset{\overset{O}{\|}}{C}-,$$

soit M lorsque T représente le groupe

$$-\overset{\overset{W}{\|}}{C}-NH\ ;$$

M représente un alkyle en $C_1$-$C_6$ ; un phénylalkyle dans lequel l'alkyle est en $C_1$-$C_3$, éventuellement substitué sur le cycle aromatique par un halogène, un trifluorométhyle, un alkyle en $C_1$-$C_4$, un hydroxyle, un alcoxy en $C_1$-$C_4$ ; un pyridylalkyle dans lequel l'alkyle est en $C_1$-$C_3$ ; un naphtylalkyle dans lequel l'alkyle est en $C_1$-$C_3$, éventuellement substitué sur le cycle naphtyle par un halogène, un trifluorométhyle, un alkyle en $C_1$-$C_4$, un hydroxyle, un alcoxy en $C_1$-$C_4$ ; un pyridylthioalkyle dans lequel l'alkyle est en $C_1$-$C_3$ ; un styryle ; un groupe aromatique ou hétéroaromatique mono-, di- ou tricyclique éventuellement substitué ;

ou un de ses sels avec des acides minéraux ou organiques.

Dans la présente description les groupes alkyle ou les groupes alcoxy sont droits ou ramifiés.

Les sels des composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate, le glycolate, le gluconate, le citrate, l'iséthionate.

De façon particulière, dans la formule (I), Z représente un groupe aromatique ou hétéroaromatique mono-, di- ou tricyclique, pouvant porter un ou plusieurs substituants, dont un atome de carbone du carbocycle aromatique ou de l'hétérocycle aromatique est directement lié au groupe T.

Plus particulièrement, le radical Z peut être un groupe phényle, qui peut être non substitué ou éventuellement contenir un ou plusieurs substituants.

Lorsque Z est un groupe phényle, celui-ci peut être de préférence mono- ou disubstitué, notamment en position 2,4, mais aussi par exemple en position 2,3 ; 4,5 ; 3,4 ou 3,5 ; il peut aussi être trisubstitué, notamment en position 2,4,6, mais aussi par exemple en 2,3,4 ; 2,3,5 ou 2,4,5 ; 3,4,5 ; tétrasubstitué, par exemple en 2,3,4,5 ; ou pentasubstitué. Les substituants du groupe phényle peuvent être : F ; Cl ; Br ; I ; CN ; OH ; $NH_2$ ; NH-CO-$NH_2$ ; $NO_2$, $CONH_2$ ; $CF_3$ ; alkyle en $C_1$-$C_{10}$, de préférence en $C_1$-$C_4$, méthyle ou éthyle étant préférés, ainsi que par exemple n-propyle, isopropyle, n-butyle, isobutyle, sec-butyle, tert.-butyle, pentyle ou n-pentyle, hexyle ou n-hexyle, heptyle ou n-heptyle, octyle ou n-octyle, nonyle ou n-nonyle ainsi que décyle ou n-décyle ; alcényle contenant 2 à 10, de préférence 2-4 atomes de carbone, par exemple vinyle, allyle, 1-propényle, isopropényle, butényle ou 1-butén-1-, -2-, -3- ou -4-yle, 2-butén-1-yle, 2-butén-2-yle, pentényle, hexényle ou décényle ; alcynyle contenant 2 à 10, de préférence 2-4 atomes de carbone, par exemple éthynyle, 1-propyn-1-yle, propargyle, butynyle ou 2-butyn-1-yle, pentynyle, décynyle ; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle ; bicycloalkyle contenant 4 à 11, de préférence 7 atomes de carbone, exo ou endo 2-norbornyle étant préférés, ainsi que par exemple 2-isobornyle ou 5-camphyle ; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle; alcoxy contenant 1 à 10, de préférence 1-4 atomes de carbone, méthoxy ou éthoxy étant préférés, ainsi que par exemple n-propoxy, isopropoxy, n-butoxy, isobutoxy, sec-butoxy, tert-butoxy, pentyloxy, hexyloxy, heptyloxy, octyloxy, nonyloxy ou décyloxy ; alcoxyalkyle contenant 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle ; alcoxyalcoxyalkyle contenant jusqu'à 10, de préférence de 4 à 7 atomes de carbone, par exemple alcoxyalcoxyméthyle, par exemple 2-méthoxyéthoxyméthyle, 2-éthoxyéthoxyméthyle ou 2-isopropoxyéthoxyméthyle, alcoxyalcoxyéthyle par exemple 2-(2-méthoxyéthoxy)éthyle ou 2-(2-éthoxyéthoxy)éthyle ; alcoxyalcoxy contenant de 2 à 10, de préférence de 3 à 6 atomes de carbone, par exemple 2-méthoxyéthoxy, 2-éthoxyéthoxy ou 2-n-butoxyéthoxy ; alcényloxy contenant 2 à 10, de préférence 2 à 4 atomes de carbone, allyloxy étant préféré, ainsi que par exemple vinyloxy, propényloxy, isopropényloxy, butényloxy tel que 1-butén-1-, -2-, -3- ou -4-yloxy, 2-butén-1-yloxy, 2-butén-2-yloxy, pentényloxy, hexényloxy ou décényloxy ; alcényloxyalkyle avec jusqu'à 10, de préférence 3-6 atomes de carbone, par exemple allyloxyméthyle ; alcynyloxy contenant de 2 à 10, de préférence 2 à 4 atomes de carbone, propargyloxy étant préféré, ainsi que par exemple éthynyloxy, 1-propyn-1-yloxy, butynyloxy ou 2-butyn-1-yloxy, pentynyloxy ou décynyloxy ; alcynyloxyalkyle contenant de 3 à 10, de préférence 3 à 6 atomes de carbone, par exemple éthynyloxyméthyle, propargyloxyméthyle ou 2-(2-butyn-1-yloxy)éthyle ; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy ; alkylthio contenant de 1 à

10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio ; alkylthioalkyle contenant de 2 à 10, de préférence 2 à 6 atomes de carbone, par exemple méthylthiométhyle, 2-méthylthioéthyle ou 2-n-butylthioéthyle ; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi qu'aroylamino ou benzoylamino ; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylaminopropyle, formylaminobutyle, acétylaminobutyle, ainsi que propionylaminobutyle, butyrylaminobutyle ; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy ; alcoxycarbonyle contenant de 2 à 5, de préférence 2 et 3 atomes de carbone, méthoxycarbonyle et éthoxycarbonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle ; cycloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle ; alkylaminocarbonylamino contenant de 2 à 4 atomes de carbone, tel que méthylaminocarbonylamino, éthylaminocarbonylamino, propylaminocarbonylamino ; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino ; (pyrrolidino-1)-carbonylamino ; (pipéridino-1)carbonylamino ; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cycloheptylaminocarbonylamino ; alkylaminocarbonylaminoalkyle contenant de 3 à 9, de préférence 4 à 7 atomes de carbone, méthylaminocarbonylaminoéthyle, éthylaminocarbonylaminoéthyle, éthylaminocarbonylaminopropyle, éthylaminocarbonylaminobutyle étant préférés, ainsi que par exemple méthylaminocarbonylaminométhyle, n-propylaminocarbonylaminobutyle, n-butylaminocarbonylaminobutyle ; dialkylaminocarbonylaminoalkyle contenant de 4 à 11 atomes de carbone, par exemple diméthylaminocarbonylaminoéthyle, diéthylaminocarbonylaminoéthyle, diéthylaminocarbonylaminopropyle, diéthylaminocarbonylaminobutyle ; (pyrrolidino-1)carbonylaminoéthyle ; (pipéridino-1)-carbonylaminoéthyle ; cycloalkylaminocarbonylaminoalkyle contenant de 5 à 12, de préférence 8 à 11 atomes de carbone, cyclopentylaminocarbonylaminoéthyle, cyclopentylaminocarbonylaminopropyle, cyclopentylaminocarbonylaminobutyle, cyclohexylaminocarbonylaminoéthyle, cyclohexylaminocarbonylaminopropyle et cyclohexylaminocarbonylaminobutyle étant préférés, ainsi que par exemple cyclopropylaminocarbonylaminométhyle, cycloheptylaminocarbonylaminoéthyle ; alcoxycarbonylaminoalkyle contenant de 3 à 12, de préférence 4 à 9 atomes de carbone, méthoxycarbonylaminoéthyle, éthoxycarbonylaminoéthyle, n-propoxycarbonylaminoéthyle, isopropoxycarbonylaminoéthyle, n-butoxycarbonylaminoéthyle, isobutoxycarbonylaminoéthyle, sec-butoxycarbonylaminoéthyle, tert-butoxycarbonylaminoéthyle, éthoxycarbonylaminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopropoxycarbonylaminobutyle ; cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopentyloxycarbonylaminobutyle, cyclohexyloxycarbonylaminoéthyle, cyclohexyloxycarbonylaminopropyle, cyclohexyloxycarbonylaminobutyle étant préférés, ainsi que par exemple cyclopropyloxycarbonylaminométhyle, cycloheptyloxycarbonylaminoéthyle ; carbamoylalkyle contenant de 2 à 5, de préférence 2 atomes de carbone, de préférence carbamoylméthyle, ainsi que carbamoyléthyle, carbamoylpropyle, carbamoylbutyle ; alkylaminocarbonylalkyle contenant de 3 à 9, de préférence 3 à 6 atomes de carbone, méthylaminocarbonyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminocarbonylméthyle, tert-butylaminocarbonylméthyle étant préférés, ainsi que par exemple éthylaminocarbonyléthyle, éthylaminocarbonylpropyle, éthylaminocarbonylbutyle, n-propylaminocarbonylbutyle, n-butylaminocarbonylbutyle ; dialkylaminocarbonylalkyle contenant de 4 à 11, de préférence 4 à 8 atomes de carbone, diméthylaminocarbonylméthyle, diéthylaminocarbonylméthyle, di-n-propylaminocarbonylméthyle, (pyrrolidino-1)carbonylméthyle, (pipéridino-1)carbonylméthyle étant préférés, ainsi que par exemple diéthylaminocarbonyléthyle, (pipéridino-1)carbonyléthyle, diéthylaminocarbonylpropyle, diéthylaminocarbonylbutyle ; cycloalkylaminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutylaminocarbonylméthyle, cycloheptylaminocarbonylméthyle, cyclohexylaminocarbonyléthyle, cyclohexylaminocarbonylpropyle, cyclohexylaminocarbonylbutyle ; alkylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy ; dialkylaminocarbonylalcoxy contenant de 4 à 10, de préférence 4 à 7 atomes de carbone, tel que diméthylaminocarbonylméthoxy, diéthylaminocarbonyléthoxy, (pipéridinyl-1)carbonylméthoxy ; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le radical Z peut également représenter un groupe aromatique bicyclique tel que le 1- ou 2-naphtyle ; 1-, 2-, 3-, 4-, 5-, 6-, 7-indényle, dont une ou plusieurs liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$.

Le radical Z peut être aussi un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en $C_1$-$C_4$.

Le groupe Z est de préférence un groupe phényle, éventuellement disubstitué par un halogène, tel que le chlore ou un groupe thiényle ; le groupe T est de préférence -C = O et le groupe R est de préférence un méthyle.

Un groupe de composés préférés de l'invention est constitué par les composés de formule I dans laquelle Ar', R, T, Z et m sont tels que définis précédemment et Y est le groupe de formule :

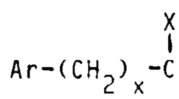

dans laquelle Ar et x sont tels que définis précédemment et X est un hydroxyle, un acétoxy ou un groupe de formule

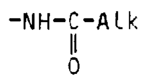

dans laquelle Alk représente un alkyle en $C_1$-$C_6$.

Le groupe Ar' est de préférence le groupe dichloro-3,4 phényle.

Un composé particulièrement préféré est le N-méthyl N[(phényl-4 acétylamino-4 pipéridinyl)-4 dichloro-3,4 phényl)-2 butyl] benzamide sous la forme du racémate ou de l'un de ses énantiomères (+) ou (-) ainsi que ses sels avec des acides minéraux ou organiques.

Selon un autre de ses aspects, la présente invention concerne un procédé pour la préparation des composés de formule (I) et de ses sels, caractérisé en ce que :

- a) on traite une amine libre de formule :

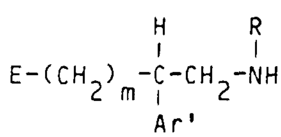

(II)

dans laquelle m, Ar' et R sont tels que définis précédemment et E représente un groupe O-protecteur tel que par exemple tétrahydropyrannyl-2-oxy ou un groupe :

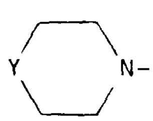

dans lequel Y est tel que défini précédemment étant entendu que :

lorsque Y représente le groupe

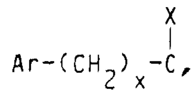

où X est un hydroxyle, cet hydroxyle est protégé ;

- soit avec un dérivé fonctionnel d'un acide de formule :

HO - CO - Z (III)

dans laquelle Z est tel que défini précédemment, lorsqu'on doit préparer un composé de formule (I) où T est -CO-,

- soit avec un iso(thio)cyanate de formule :

W = C = N - Z (III')

dans laquelle W et Z sont tels que définis précédemment, lorsqu'on doit préparer un composé de formule (I) où T est -C(W)-NH-, pour former le composé de formule :

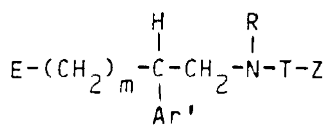

(IV)

- b) puis, lorsque E représente le tétrahydropyrannyloxy, on élimine le groupe tétrahydropyrannyle par action d'un acide,
- c) on traite l'alcanolamine N-substituée ainsi obtenue de formule :

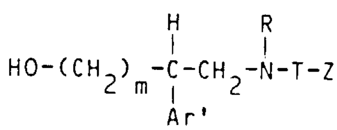

(V)

avec le chlorure de méthanesulfonyle ;

- d) on fait réagir le mésylate ainsi obtenu de formule :

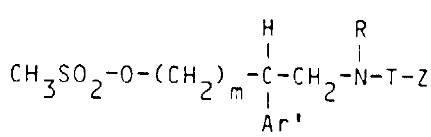

(VI)

avec une amine secondaire de formule :

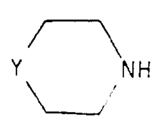

(VII)

dans laquelle Y est tel que défini précédemment ; et

- e) après déprotection éventuelle de l'hydroxyle représenté par X, on transforme éventuellement le produit ainsi

obtenu en un de ses sels.

Comme dérivé fonctionnel de l'acide (III), on utilise l'acide lui-même, convenablement activé par exemple par le cyclohexylcarbodiimide ou par l'hexafluorophosphate de benzotriazolyl N-oxytrisdiméthylaminophosphonium (BOP), ou bien un des dérivés fonctionnels qui réagissent avec les amines, par exemple un anhydride, un anhydride mixte, le chlorure d'acide ou un ester activé. Lorsque Z est un groupe OM, l'acide concerné est l'acide carbonique et, comme dérivé fonctionnel, on utilise le monochlorure, à savoir un chloroformiate Cl-CO-OM.

Lorsque comme produit de départ on utilise un composé de formule (II) où E représente un groupe :

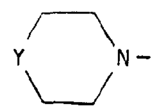

le procédé de la présente invention peut être représenté et illustré en détail par le Schéma 1 ci-après :

SCHEMA 1

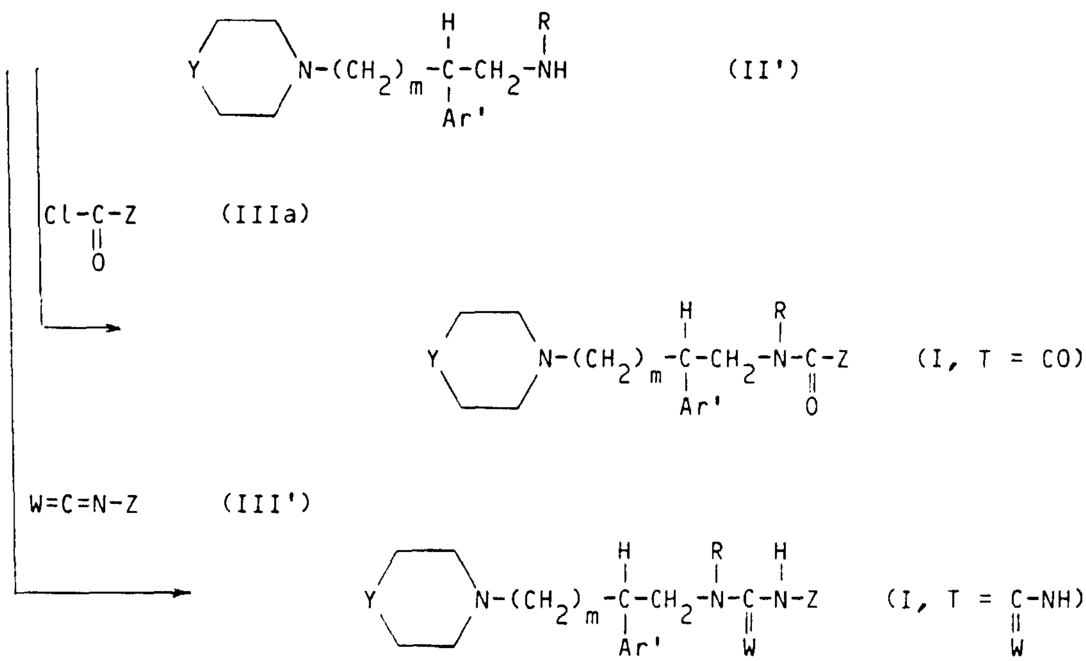

Dans la formule (IIIa) ci-dessus, on considère le chlorure d'acide comme dérivé fonctionnel réactif de l'acide (III). On peut cependant utiliser un autre dérivé fonctionnel ou on peut partir de l'acide libre (III) en réalisant un couplage de (II') avec le BOP (hexafluorophosphate de benzotriazolyl N-oxytrisdiméthylamino phosphonium), puis, en additionnant l'acide (III) en présence d'une base organique comme par exemple la triéthylamine, dans un solvant comme le dichlorométhane ou le diméthylformamide, à température ambiante, les composés (I) obtenus sont isolés et purifiés selon les méthodes habituelles, comme par exemple la chromatographie ou la recristallisation.

On peut faire aussi réagir (II') avec un iso(thio)cyanate W = C = N-Z (III') dans un solvant inerte anhydre tel que par exemple le benzène, pendant une nuit à température ambiante puis traiter le mélange réactionnel selon les méthodes habituelles pour obtenir les composés (I).

Lorsque comme produit de départ on utilise un composé de formule (II) où E représente un groupe tétrahydropyrannyloxy, le procédé de la présente invention peut être représenté et illustré à partir du Schéma 2.

Les réactions du composé (II") avec les réactifs (IIIa) et (III') se déroulent comme décrit ci-dessus pour le Schéma 1, le chlorure d'acide (IIIa) pouvant être remplaçé par un autre dérivé fonctionnel ou par l'acide libre activé par exemple par le BOP.

L'intermédiaire (IV') ainsi obtenu est déprotégé par hydrolyse acide douce pour conduire au composé hydroxylé libre (V). La déprotection par hydrolyse du groupe tétrahydropyranyloxy peut être effectuée directement sur le composé (II"). On obtient ainsi le composé hydroxylé (II''') qui est mis en réaction directement avec les réactifs (IIIa) et (III') comme décrit dans le schéma 2 ci-après pour fournir le composé (V).

A partir du composé (V), on prépare le mésylate (VI) pour le substituer par une amine secondaire de formule (VII) pour obtenir finalement les composés (I) selon l'invention.

SCHEMA 2

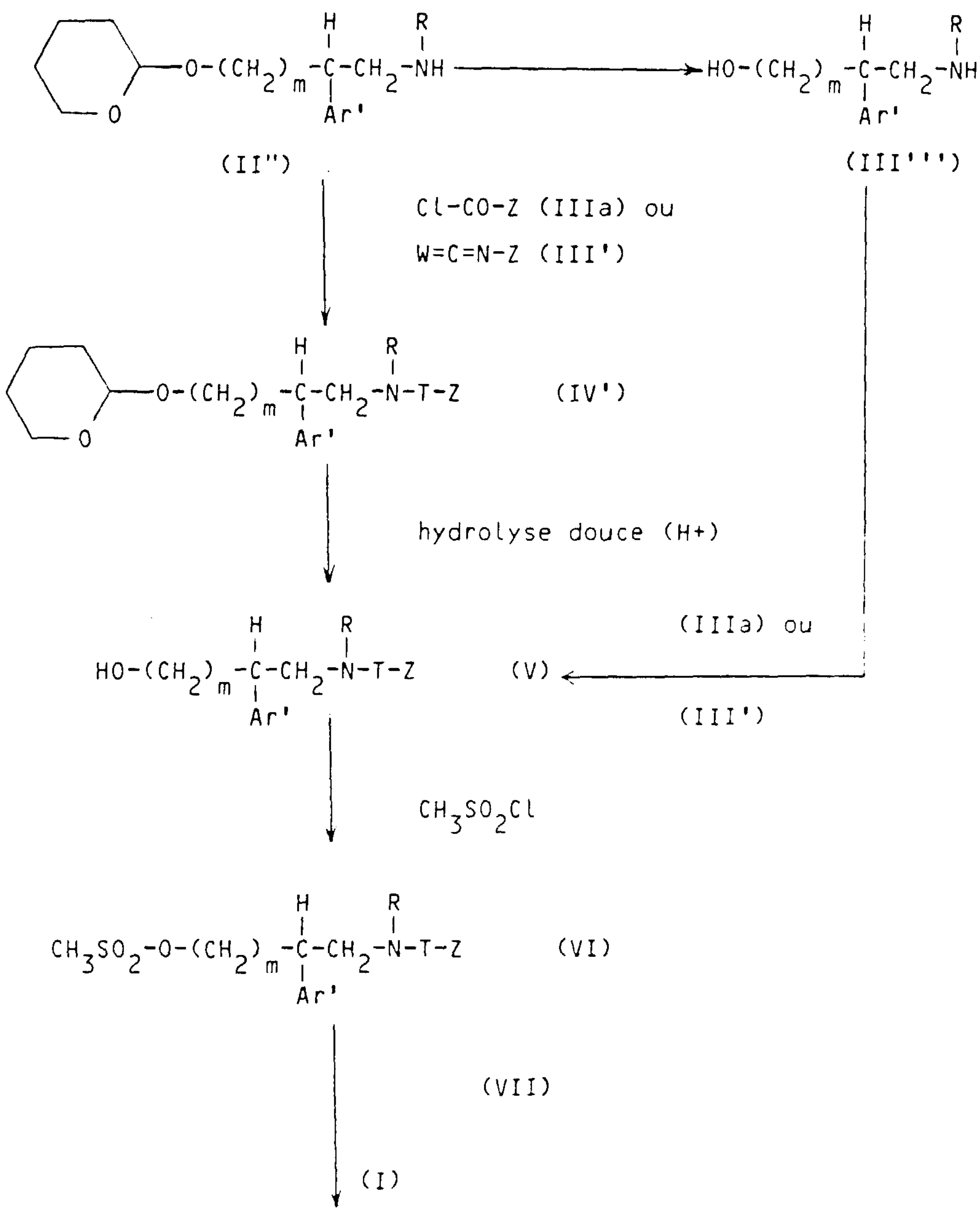


Les produits de formule (I) ainsi obtenus sont isolés, sous forme de base libre ou de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel

que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, les composés de formule (I) peuvent être isolés sous forme d'un de leurs sels, par exemple le chlorhydrate ou l'oxalate ; dans ce cas, si nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

La résolution des mélanges racémiques et éventuellement des mélanges de diastéréoisomères (I) permet d'isoler les énantiomères ou les diastéréoisomères qui font partie de l'invention.

La résolution peut également être faite sur l'un des composés (II") et (II"') du schéma 2 ci-dessus, les réactions suivantes, indiquées dans le même schéma, ne provoquant pas de racémisation. De façon avantageuse, la résolution est effectuée sur un composé de formule (II"') dans laquelle R est tel que défini ci-dessus, de préférence l'hydrogène. La séparation est effectuée selon des méthodes connues, par formation d'un sel avec un acide optiquement actif, par exemple l'acide (+)- ou (-)-tartrique, par séparation des sels diastéréoisomères et hydrolyse. Un composé particulièrement adapté à la résolution a la formule (II"'), dans laquelle R est l'hydrogène et Ar' est 2,4- ou, de préférence, 3,4-dichlorophényle.

Les composés de départ de formule (II) sont préparés à partir de nitriles de formule :

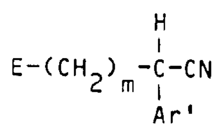

(VIII)

dans laquelle m, E et Ar' sont tels que définis ci-dessus, par réduction du nitrile.

Pour la préparation des composés de formule (II) où R est l'hydrogène, les nitriles de départ de formule (VIII) sont soumis à une hydrogénation dans un alcanol tel que l'éthanol, en présence d'un catalyseur tel que le nickel de Raney, et l'amine libre primaire peut être isolée selon les méthodes classiques.

Lorsqu'on souhaite préparer les composés de formule (II) où R est le groupe méthyle, on traite l'amine libre, obtenue par hydrogénation du nitrile (VIII) comme décrit ci-dessus, avec un chloroformiate, par exemple avec le chloroformiate de formule Cl-CO-$OR_1$, où $R_1$ est un alkyle en $C_1$-$C_6$, pour obtenir les carbamates de formule :

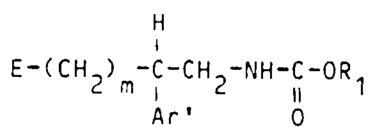

qui sont ensuite réduits par des moyens connus, tels que l'action d'un agent réducteur comme par exemple un hydrure métallique, tel que l'hydrure de sodium et d'aluminium, l'hydrure de lithium et d'aluminium ou par un hydrure de bore, tel que le diméthylsulfure de borane. La réduction est réalisée dans un solvant, tel que l'éther, le toluène ou le tétrahydrofuranne, à une température comprise entre la température ambiante et 60°C. L'amine ainsi obtenue de formule :

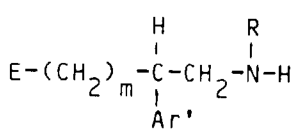

(II, R = $CH_3$)

est isolée selon les méthodes habituelles.

On peut aussi traiter le composé de formule :

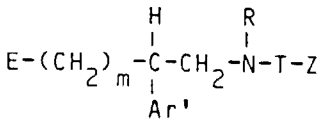

(IV, R = H)

dans laquelle m, E, Ar', T et Z sont tels que définis précédemment par un halogénure d'alkyle en présence d'une base forte telle que par exemple un hydrure métallique comme par exemple l'hydrure de sodium, dans un solvant inerte tel que le tétrahydrofuranne chauffé au reflux pour préparer les composés (IV) dans lesquels R est autre que l'hydrogène.

Les nitriles de formule (VIII) sont préparés à partir de nitriles de formule :

$$Ar'-CH_2-CN \qquad (XI)$$

qui par alkylation avec un composé de formule :

$$E-(CH_2)_m-G \qquad (XII)$$

dans laquelle m et E sont tels que définis ci-dessus et G est un atome d'halogène, par exemple de brome ou un groupe hydroxy protégé, donnent les composés (VIII) désirés.

La synthèse des nitriles de formule (VIII) où E est un groupe tétrahydropyrannyloxy est réalisée à partir d'un dérivé tétrahydropyrannyloxy (THP-O) obtenu par réaction entre un alcanol de formule $Br-(CH_2)_m-OH$ avec m tel que défini précédemment et le dihydropyranne pour conduire au composé :

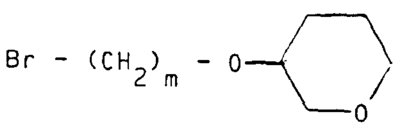

(XII, E = THP-O-, G = Br)

qui est ensuite additionné, en présence d'hydrure alcalin sur le dérivé acétonitrile (XI) pour préparer l'intermédiaire,

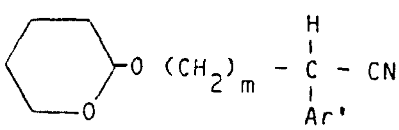

(VIII, E = THP-O-)

La synthèse des nitriles de formule (VIII) où E représente un groupe :

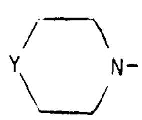

où Y est tel que défini précédemment, est effectuée selon des méthodes connues par addition sur des dérivés chlorés de formule :

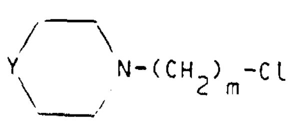

(XIII)

d'un dérivé nitrile de formule :

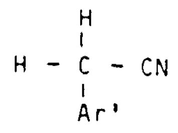

(XIV)

en présence d'amidure de sodium dans un solvant tel que le toluène à des températures comprises entre 30 et 80°C.

Le dérivé chloré (XIII) est préparé par action d'un réactif chlorant tel que le chlorure de thionyle sur le dérivé hydroxylé de formule :

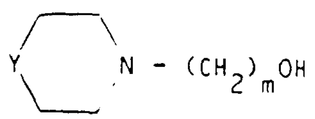

(XV)

lui-même préparé à partir de l'amine de formule (VII) dans laquelle, si X = OH, alors le groupe hydroxyle est éventuellement protégé par un groupe O-protecteur selon les méthodes habituelles,

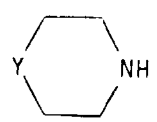

(VII)

amine sur laquelle on fait réagir, si m = 2, l'oxyde d'éthylène et, si m = 3, un halogéno-3 propanol.

Les composés selon l'invention ont fait l'objet d'essais biochimiques et pharmacologiques.

Les propriétés antagonistes de la liaison aux récepteurs $NK_2$ ont été mises en évidence par des essais réalisés sur des membranes de duodénum de rat selon L. BERGSTOM et al., Mol. Pharmacol., 1987, 32, 764-771.

Des essais ont aussi été réalisés sur l'artère pulmonaire de lapin dépourvue d'endothélium, qui possède des récepteurs $NK_2$ dont l'activation conduit à une contraction musculaire. Les essais sur différents organes isolés ont été réalisés selon D. REGOLI et al., Trends Pharmacol. Sci., 1988, 9, 290-295 et Pharmacology, 1989, 38, 1-15.

Des essais sur le bronchospasme chez le cobaye induit par un agoniste $NK_2$ ont été réalisés selon H. KONZETT et al., Arch. Exp. Path. Pharm., 1940, 195, 71-4.

Les composés selon l'invention déplacent la [2-$^{125}$I histidyl]-neurokinine A de son récepteur avec une Ki de l'ordre de 3 à 0,50 nM.

Les mêmes composés, dans les essais réalisés sur l'artère pulmonaire de lapin ont montré une $pA_2$ de 10,4 à 9.

Les mêmes composés, dans les essais réalisés sur le bronchospasme du cobaye ont montré une activité antagoniste de la [Nle$^{10}$]-neurokinine A par voie i.v. à une dose de 200 µg/kg.

Compte tenu des propriétés antagonistes de la neurokinine A dont sont pourvus les composés selon l'invention, ils peuvent être utiles dans toute pathologie neurokinine A dépendante et plus particulièrement dans les inflammations neurogéniques des voies respiratoires comme par exemple l'asthme ou la bronchoconstriction.

Les composés de la présente invention sont peu toxiques ; notamment, leur toxicité aigüe est compatible avec leur utilisation comme médicament. Pour une telle utilisation, on administre aux mammifères une quantité efficace d'un composé de formule (I) ou d'un de ses sels pharmaceutiquement acceptables.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Dans les compositions pharmaceutiques de la présente invention pour l'administration orale, sublinguale, sous-cutanée, intramusculaire, intraveineuse, transdermique, locale ou rectale, les ingrédients actifs peuvent être administrés sous formes unitaires d'aministration, en mélange avec des supports pharmaceutiques classiques, aux animaux

et aux être humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire, les formes d'administration rectale, les formes d'administration par inhalation ou par application sur la membrane des muqueuses comme celles du nez, de la gorge ou des bronches, par exemple à l'aide d'un aérosol contenant le principe actif sous forme d'un spray ou d'une poudre sèche.

Afin d'obtenir l'effet désiré, la dose de principe actif peut varier entre 0,25 et 1 000 mg par jour, de préférence entre 2 et 250 mg.

Chaque dose unitaire peut contenir de 0,25 à 250 mg de principe actif, de préférence de 1 à 125 mg, en combinaison avec un support pharmaceutique. Cette dose unitaire peut être administrée 1 à 4 fois par jour.

Lorsque l'on prépare une composition solide sous forme de comprimés, on mélange l'ingrédient actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou l'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorodifluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Les compositions susdites peuvent également renfermer d'autres produits actifs tels que, par exemple, des bronchodilatateurs, des antitussifs ou des antihistaminiques.

Les exemples suivants illustrent l'invention.

Dans les exemples qui suivent, les abréviations suivantes ont été utilisées.

F : point de fusion instantané exprimé en degrés Celcius
Ac : acétyl
AcO : acétoxy.

Les spectres de RMN ont été effectués à 200 MHz dans le diméthylsulfoxyde deutéré.

m : massif,
s : singulet,
se : singulet élargi,
t : triplet,
Mult : multiplet.

EXEMPLE 1

Chlorhydrate de N-[(dichloro-3,4 phényl)-2 (hydroxy-4 phényl-4 pipéridinyl)-4 butyl] dichloro-2,4 benzamide.

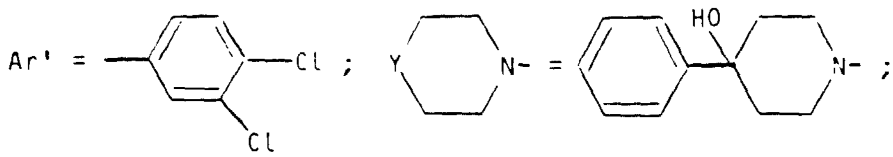

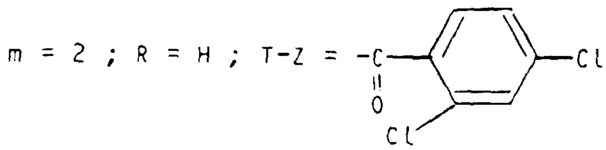

a) (Tétrahydropyrannyl-2 oxyéthyl)-α dichloro-3,4 benzèneacétonitrile.

16,5 g d'hydrure de sodium à 80 % dans l'huile sont mis en suspension dans 200 ml de tétrahydrofuranne sec. On ajoute goutte à goutte à 20°C, en 30 min, une solution de 100 g de dichloro-3,4 phénylacétonitrile dans 500 ml de tétrahydrofuranne puis on agite le mélange réactionnel à température ambiante pendant 2 h. Le mélange est refroidi à -20°C et on ajoute une solution de 118 g de bromo-1 tétrahydropyrannyl-2 oxyéthane dans 100 ml de tétrahydrofuranne, on laisse revenir le mélange à température ambiante et après 2 h on ajoute une solution de 50 g de chlorure d'ammonium dans 3 l d'eau. On extrait avec 1,5 l d'éther, lave avec une solution saturée de chlorure de sodium, décante, sèche sur $MgSO_4$ et concentre sous vide.

Le résidu est chromatographié sur gel de silice, éluant : dichlorométhane puis dichlorométhane-acétate d'éthyle 95-5 (v/v). Les fractions de produit pur sont concentrées sous vide pour fournir 118 g d'une huile.

b) (Tétrahydropyrannyl-2 oxyéthyl)-β dichloro-3,4 benzèneéthanamine.

118 g du nitrile obtenu précédemment sont mis en solution dans 700 ml d'éthanol absolu. On ajoute 300 ml d'ammoniaque concentrée puis, sous balayage d'azote, on ajoute du nickel de Raney (10 % de la quantité de nitrile de départ). On hydrogène ensuite sous atmosphère d'hydrogène à température ambiante et pression ordinaire.

16 l d'hydrogène sont absorbés en 4 h. Le catalyseur est séparé par filtration sur célite, le filtrat est concentré sous vide, le résidu est repris dans une solution saturée de chlorure de sodium. Après extraction à l'éther et séchage sur $MgSO_4$, on obtient 112 g d'une huile.

c) N-[(Dichloro-3,4 phényl)-2 tétrahydropyrannyl-2 oxy)-4 butyl] dichloro-2,4 benzamide.

80 g de l'amine obtenue précédemment sont mis en solution dans 800 ml de dichlorométhane. La solution est refroidie à 0°C, on ajoute 38,4 ml de triéthylamine puis 55 g de chlorure de l'acide dichloro-2,4 benzoïque. Le mélange réactionnel est alors agité à température ambiante pendant 1 h, puis lavé à l'eau. La phase organique est décantée, séchée sur $MgSO_4$ et concentrée sous vide pour fournir 120 g d'une huile.

d) N-[(dichloro-3,4 phényl)-2 hydroxy-4 butyl] dichloro-2,4 benzamide.

120 g du produit obtenu précédemment sont mis en solution dans 1 l de méthanol en présence de 12 g d'acide paratoluènesulfonique. Le mélange réactionnel est agité pendant 18 h à température ambiante puis concentré sous vide. Le résidu est repris dans le dichlorométhane et lavé avec une solution à 10 % de carbonate de sodium. La phase organique est décantée et séchée sur $MgSO_4$ pour fournir 106 g d'une huile.

e) N-[(Méthanesulfonyloxy-4 (dichloro-3,4 phényl)-2 butyl] dichloro-2,4 benzamide.

106 g de l'alcool obtenu précédemment sont mis en solution dans 1 l de dichlorométhane puis on ajoute à la solution refroidie à 0°C, 44 ml de triéthylamine et 24,2 ml de chlorure de méthanesulfonyle. Le mélange réactionnel est agité à 0°C pendant 45 min, lavé 3 fois à l'eau glacée, décanté, séché sur $MgSO_4$ et concentré sous vide.
Le résidu est recristallisé de l'éther isopropylique.
m = 95 g

f) Composé 1

Un mélange de 1 g du produit obtenu précédemment, de 0,8 g d'hydroxy-4 phényl-4 pipéridine et de 1 ml de diméthylformamide, est chauffé à 60°C pendant 2 h. Après refroidissement, on dilue avec de l'éther, lave avec une solution d'hydroxyde de sodium diluée puis à l'eau. Après séchage sur $MgSO_4$, on évapore les solvants et chromatographie le résidu sur 40 g de silice, élution : dichlorométhane puis dichlorométhane/méthanol 90/10, (v/v). La concentration des fractions pures fournit 0,9 g du produit dont on fait le chlorhydrate dans le dichlorométhane en additionnant de l'éther chlorhydrique jusqu'à pH = 1. Le précipité est séparé par filtration puis concrétisé dans l'éther.
m = 0,95 g.
RMN :
8,75 (t, 1 H) ; 7,7-7 (m, 11 H) ; 5,4 (s, 1 H) ; 3,6-2,6 (m, 9 H) ; 2,6-1,6 (m, 6 H).

EXEMPLE 2

Chlorhydrate de N-[(dichloro-3,4 phényl)-2 (hydroxy-4 phényl-4 pipéridinyl)-4 butyl] acétamide.

Ar' = 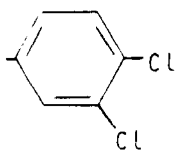 ; 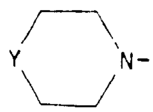 = 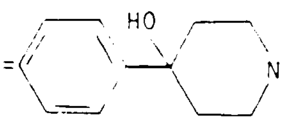 ;

m = 2 ; R = H ; T-Z = 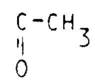

On prépare le N-[dichloro-3,4 phényl)-2 mésyloxy-4 butyl] acétamide en procédant comme il est décrit dans l'exemple 1 étapes a) b) c) d) et e) en remplaçant à l'étape c) le chlorure de l'acide dichloro-2,4 benzoïque par le chlorure d'acétyle.

Composé 2

On chauffe à 60°C un mélange de 6,5 g du produit précédemment préparé, de 6,8 g d'hydroxy-4 phényl-4 pipéridine et de 10 ml de diméthylformamide.

Après 1 h, le mélange réactionnel est versé dans l'eau et extrait à l'acétate d'éthyle. La phase organique est décantée, séchée sur $Na_2SO_4$, filtrée et concentrée sous vide. Le résidu est purifié par chromatographie sur gel de silice ; éluant : méthanol/ dichlorométhane 10/90 (v/v). La concentration des fractions de produit pur fournit un résidu qui est salifié avec de l'éther chlorhydrique et on recueille 6 g de chlorhydrate.
RMN :
7,95 (t, 1 H) ; 7,7-7,0 (m, 8 H) ; 3,6-2,6 (m, 9 H) ; 2,6-1,3 (m, 9 H).

EXEMPLE 3

Chlorhydrate de N-éthyl N-[(dichloro-3,4 phényl)-2 (hydroxy-4 phényl-4 pipéridinyl)-4 butyl] thiophène-2 carboxamide.

Ar' = 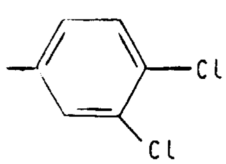 ; 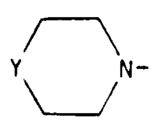 = 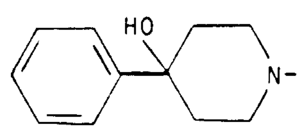 ;

m = 2 ; R = $-CH_2CH_3$ ; T-Z = 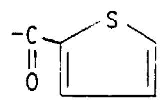

a) Chlorhydrate de N-éthyl (hydroxy-4 phényl-4 pipéridinyl) éthyl-β dichloro-3,4 benzèneéthanamine.

A une suspension de 0,96 g d'hydrure de lithium aluminium dans 10 ml de tétrahydrofuranne on ajoute une solution de 5,5 g du composé 2, obtenu précédemment à l'exemple 2, dans 20 ml de tétrahydrofuranne puis le mélange réactionnel est chauffé à reflux pendant 2 h. On refroidit alors et hydrolyse avec 4 ml d'une solution d'hydroxyde de sodium 4 N, filtre l'alumine, rince au tétrahydrofuranne et évapore. Après salification avec une solution d'éther chlorhydrique, le chlorhydrate est concrétisé dans un mélange isopropanol/éther isopropylique ; on en recueille 4,7 g.

b) Composé 3

A une solution de 2,45 g, du produit précédemment obtenu, dans le dichlorométhane à 0°C, on ajoute 2,75 ml de triéthylamine puis 0,8 g de chlorure de thénoyle-2. Après hydrolyse avec une solution d'hydroxyde de sodium 0,1 N et extraction au dichlorométhane, le produit est purifié par chromatographie sur silice H ; éluant : méthanol/dichlorométhane : 2,5/97,5 (v/v). Le produit pur est salifié avec une solution d'éther chlorhydrique et on recueille finalement 1,0 g de chlorhydrate.
RMN :
7,75-6,9 (m, 11 H) ; 5,35 (s, 1 H) ; 3,9-2,55 (m, 11 H) ; 2,55-1,5 (m, 6 H) ; 0,95 (t, 3 H).

EXEMPLE 4

Chlorhydrate de N-éthyl-N-[(dichloro-3,4 phényl)-2 (hydroxy-4 phényl-4 pipéridinyl)-4 butyl] méthoxy-4 benzamide.

En procédant selon l'exemple 3 étape b), à partir du composé obtenu à l'étape a) et en remplaçant le chlorure de thénoyle-2 par le chlorure de l'acide méthoxy-4 benzoïque, on obtient le composé 4.
F = 165°C.

EXEMPLE 5

Dichlorhydrate de N-[(hydroxy-4 (pyridinyl-2 méthyl)-4 pipéridinyl)-4 (dichloro-3,4 phényl)-2 butyl] dichloro-2,4 benzamide.

Ar' = 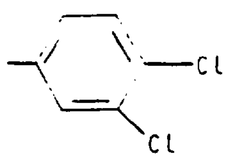 ; 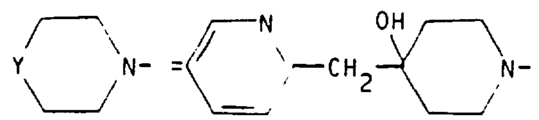 ;

m = 2; R = H ; T-Z = 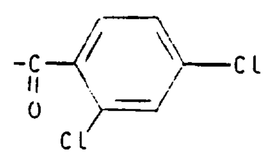

a) N-[(dichloro-3,4 phényl)-2 éthylènedioxy-4,4 pipéridinyl)-4 butyl] dichloro-2,4 benzamide.

On chauffe à 100°C pendant 15 min un mélange de 12,1 g du mésylate obtenu à l'exemple 1 e) et de 8,6 g d'éthylènedioxy-4,4-pipéridine. Le mélange est refroidi, repris dans le dichlorométhane, lavé à l'eau. La phase organique est décantée, séchée sur $Na_2SO_4$, filtrée et concentrée sous vide. Le résidu est chromatographié sur gel de silice ; éluant : méthanol/dichlorométhane : 2/98 (v/v).

La concentration des fractions pures fournit 12,15 g de produit attendu.

b) N-[(dichloro-3,4 phényl)-2 (oxo-4 pipéridinyl)-4 butyl] dichloro-2,4 benzamide.

On dissout le produit précédemment obtenu dans 100 ml d'acétone puis on ajoute 100 ml d'acide chlorhydrique 6 N. Après 2 h, on ajoute 1 l d'eau et 1 l d'éther et on récupère la phase aqueuse. Celle-ci est ensuite amenée à pH = 10 par addition d'hydroxyde de sodium puis extraite avec 1 l d'éther. Après séchage et évaporation de la phase organique, on obtient 9,7 g de produit pur.

c) Composé 5

A une solution de 1 g de produit précédemment obtenu dans 5 ml de tétrahydrofuranne à 25°C sous azote, on ajoute une solution de picolyl-2 lithium 2,15 M dans tétrahydrofuranne jusqu'à persistance de la coloration rouge (voir synthesis page 43, 1974). Après hydrolyse et extraction à l'éther, le résidu est chromatographié sur gel de silice ; éluant : méthanol/dichlorométhane : 15/85 (v/v). Après évaporation des fractions pures et salification du résidu avec une solution d'éther chlorhydrique, on obtient 400 mg d'une mousse blanche.
RMN :
8,8-7,15 (m, 11 H) ; 5,3 (s large, 1 H) ; 4-2,55 (m, 11 H) ; 2,35-1,4 (m, 6 H).

<u>EXEMPLE 6</u>

Chlorhydrate de N-[(benzyl-4 hydroxy-4 pipéridinyl)-4 (dichloro-3,4 phényl)-2 butyl] N'-napht-1-yl urée.

Ar' = 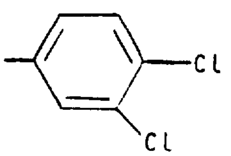 ; 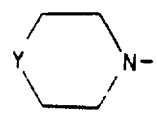 = 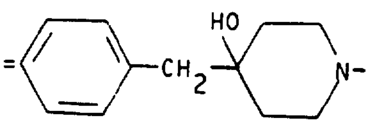 ;

m = 2 ; R = H ; T-Z = 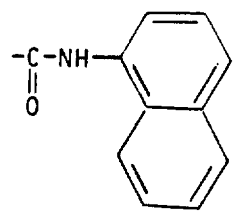

a) N-[(tétrahydropyrannyl-2 oxy)-4 (dichloro-3,4 phényl)-2 butyl] N'-napht-1-yl urée.

A une solution de 7,6 g de napht-1-yl isocyanate dans 50 ml de toluène, on ajoute 12 g de (dichloro-3,4 phényl)-2 (tétrahydropyrannyl-2 oxy)-4 butanamine obtenue selon l'exemple 1 b) dans 50 ml de toluène. Après avoir agité le mélange réactionnel pendant 10 min, on ajoute 50 ml de méthanol et concentre sous vide.

b) N-[hydroxy-4 (dichloro-3,4 phényl)-2 butyl-4] N'-napht-1-yl urée.

A une solution du produit précédemment obtenu, on ajoute 2 g d'acide p-toluènesulfonique et on chauffe le mélange à reflux pendant 10 min. La solution est lavée avec du bicarbonate de sodium et concentrée à sec. Après purification du résidu par chromatographie sur gel de silice en éluant avec de l'acétate d'éthyle, on obtient 13,1 g d'une huile incolore.

c) N-[(dichloro-3,4 phényl)-2 mésyloxy-4 butyl] N'-napht-1-yl urée.

A une solution de 13,1 g du produit précédemment obtenu dans 100 ml de dichlorométhane, on ajoute à 0°C 5,37 ml de triéthylamine puis 2,77 ml de chlorure de mésyle. La solution est alors lavée avec 3 fois 100 ml d'eau glacée puis la phase organique est séchée et évaporée. Le résidu est alors recristallisé dans l'isopropanol puis filtré et rincé

à l'éther isopropylique.
m = 8 g
F = 120°C

d) Composé 6

On chauffe à 100°C pendant 20 min un mélange de 4 g de produit précédemment obtenu, 4 g d'hydroxy-4 benzyl-4 pipéridine et 4 ml de diméthylformamide. Le tout est ensuite versé sur l'eau et extrait au dichlorométhane. Le résidu est alors purifié par chromatographie sur gel de silice en éluant avec un mélange méthanol/ dichlorométhane : 4/96 (v/v). Après salification avec une solution d'éther chlorhydrique, on recueille 1,0 g de chlorhydrate pur.
RMN :
8,7 (s, 1 H) ; 8,2-6,8 (m, 16 H) ; 3,5-2,5 (m, 11 H) ; 2,3-1,3 (m, 6 H).

Les composés décrits dans les tableaux 1, 2 et 3 ci-dessous ont été synthétisés selon les exemples 1 à 6. Ces composés sont tous des chlorhydrates.

TABLEAU 1

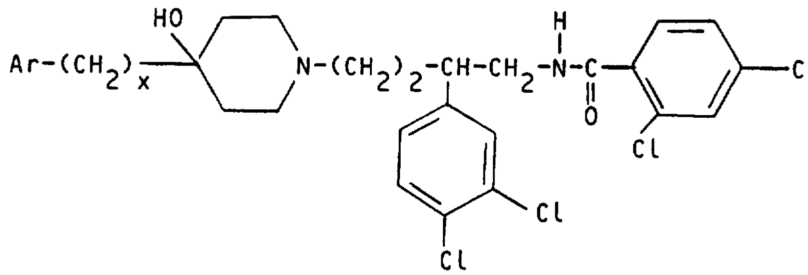

, HCl

| exemple n° | Ar | x | Spectre de RMN |
|---|---|---|---|
| 7 | 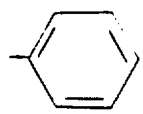 | 1 | 8,54 (t, 1 H) ; 7,7-7 (m, 11 H) ; 4,8 (s large 1 H) ; 3,8-2,55 (m, 11 H) ; 2,4-1,25 (m, 6 H). |
| 8 | 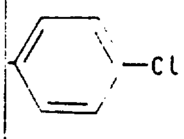 | 1 | 8,6 (t, 1 H) ; 7,8-7,2 (m, 10 H) ; 4,9 (s, 1 H) ; 3,7-2,6 (m, 11 H) ; 2,2-1,4 (m, 6 H). |
| 9 | 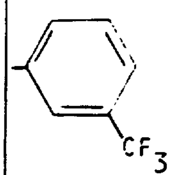 | 0 | 8,55 (t, 1 H) ; 7,8-7,2 (m, 10 H) ; 5,70 (s, 1 H) ; 3,6-2,6 (m, 9 H); 2,6-1,6 (m, 6 H). |
| 10 | 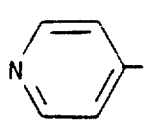 | 1 | 8,7 (d, 2 H) ; 8,45 (t, 1 H) ; 7,85 (d, 2 H) ; 7,6-7 (m, 6 H) ; 5,3 (s large, 1 H) ; 4,0-2,3 (m, 11 H) ; 2,3-0,6 (m, 6 H). |

TABLEAU 2

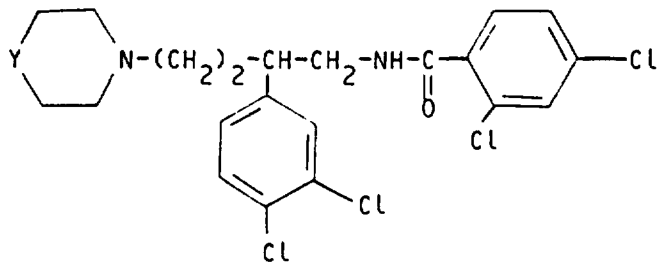

, HCl

| exemple n° | 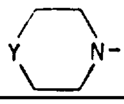 | Spectre de RMN |
|---|---|---|
| 11 | 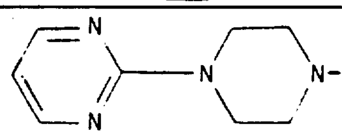 | 8,4-8,8 (m, 3 H) ; 7,2-7,8 7,2-7,8 (m, 6 H) ; 6,8 (t, 1 H) ;<br>4,7 (d, 2 H) ; 2,6-3,8 (m, 11 H) ;<br>2,2 (Mult., 2 H). |

TABLEAU 3

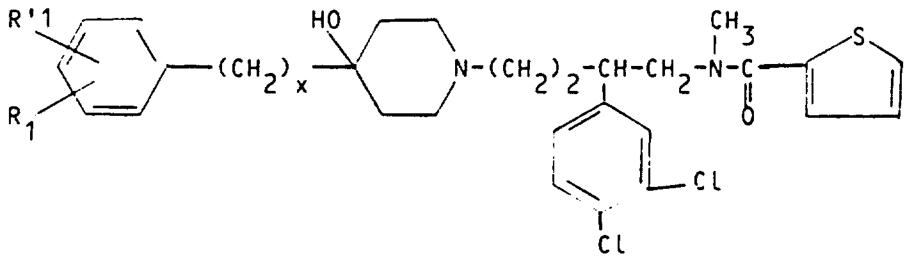

, HCl

| exemple n$^{O}$ | $R_1$ | $R'_1$ | x | Spectre de RMN |
|---|---|---|---|---|
| 12 | H | H | 1 | 7,8-6,8 (m, 11 H) ; 4,75 (s, 1 H) ; 4,0-2,5 (m, 14 H) ; 2,3-1,3 (m, 6 H). |
| 13 | 4-$OCH_3$ | H | 0 | 6,9-7,4 (m, 5 H) ; 3,9-4,9 (m, 1 H) ; 0,6-2,6 (m, 11 H). |

EXEMPLE 14

Chlorhydrate de N-méthyl N-[(dichloro-3,4 phényl)-2 (hydroxy-4 phényl-4 pipéridinyl)-4 butyl] benzamide.

Ar' = 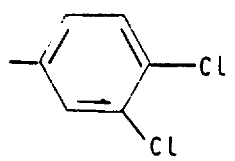 ; 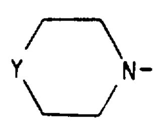 = 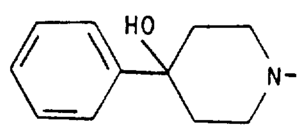 ;

m = 2 ; R = $-CH_3$ ; T-Z = 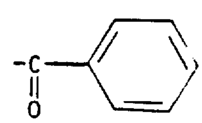

a) N-[(dichloro-3,4 phényl)-2 (tétrahydropyrannyl-2 oxy)-4 butyl] carbamate d'éthyle.

A une solution refroidie à -20°C de 80 g d'amine obtenue à l'exemple 1 b), et de 39 ml de triéthylamine, dans 800 ml de dichlorométhane, on ajoute goutte à goutte 26,4 ml de chloroformiate d'éthyle. Après 30 min, on lave deux fois à l'eau puis avec une solution tampon pH = 2.

On décante la phase organique et sèche sur $MgSO_4$ puis concentre sous vide pour obtenir 98 g de produit sous forme d'huile.

b) N-(méthyl) (tétrahydropyrannyl-2 oxy) éthyl-β dichloro-3,4 benzèneéthanamine.

Dans un tricol de 2 l balayé à l'azote, on introduit 20 g d'hydrure d'aluminium en suspension dans 200 ml de tétrahydrofuranne. On ajoute à 20°C une solution de 98 g du carbamate obtenu précédemment, dans 800 ml de tétrahydrofuranne.

On chauffe avec précaution jusqu'au reflux et on maintient celui-ci pendant 18 h.

On refroidit à 0°C et hydrolyse, avec 35 ml d'eau puis avec un mélange de 17 ml d'une solution d'hydroxyde de sodium concentrée et 150 ml d'eau. On sépare les minéraux par filtration puis concentre sous vide pour obtenir 80,5 g de produit sous forme d'huile.

c) Chlorhydrate de N-méthyl hydroxyéthyl-β dichloro-3,4 benzène-éthanamine.

A 50 g d'aminoalcool protégé obtenu précédemment en solution dans 500 ml d'éthanol, on ajoute 20 ml d'acide chlorhydrique concentré. Après 2 h 30, on concentre sous vide, dissout le résidu dans 200 ml d'acétonitrile puis ajoute lentement 350 ml d'éther. On agite pendant 1 h, filtre les cristaux, rince à l'éther.
m = 32,8 g
F = 152°C.

d) N-méthyl N-[(dichloro-3,4 phényl)-2 hydroxy-4 butyl] carbamate de tert-butyle.

A 32,8 g de chlorhydrate du produit précédemment en solution dans 300 ml de dioxanne et 30 ml d'eau, on ajoute 20 ml de triéthylamine. On additionne alors 27 g de $Boc_2O$ (di-tert-butyldicarbonate) puis on agite à température ambiante pendant 15 min. On chauffe à 60°C pendant 30 min. Après concentration à sec, on reprend à l'éther, lave à l'eau puis avec une solution tampon pH = 2, puis encore à l'eau. On sèche sur $MgSO_4$, concentre sous vide pour obtenir 40 g d'huile.

e) N-méthyl N-[(dichloro-3,4 phényl)-2 méthanesulfonyloxy-4 butyl] carbamate de tert-butyle.

A 40 g d'alcool obtenu précédemment en solution dans 400 ml de dichlorométhane, on ajoute 17 ml de triéthylamine. On refroidit à 0°C et ajoute goutte à goutte 9,3 ml de chlorure de mésyle. Après 15 min, on lave 2 fois à l'eau, sèche sur $MgSO_4$, concentre à sec pour obtenir 49 g d'huile.

f) Chlorhydrate de N-méthyl N-[(dichloro-3,4 phényl)-2 (hydroxy-4 phényl-4 pipéridinyl)-4 butyl] carbamate de tert-butyle.

On chauffe un mélange de 20 g du produit obtenu précédemment et de 18 g d'hydroxy-4 phényl-4 pipéridine dans 40 ml de diméthylformamide à 70°C pendant 1 h 30. On verse ensuite la solution sur 300 ml d'eau glacée, filtre le précipité et rince à l'eau. Le solide est ensuite repris dans l'éther, séché sur $MgSO_4$ et évaporé. Le produit brut est purifié par chromatographie sur gel de silice en éluant avec un gradient de méthanol dans le dichlorométhane (jusqu'à 5 %). On obtient 22 g de produit pur.

g) Dichlorhydrate de N-méthyl (hydroxy-4 phényl-4 pipéridinyl) éthyl-β dichloro-3,4 benzèneméthanamine.

A une solution de 22 g du dérivé précédemment obtenu dans 100 ml de méthanol, on ajoute 100 ml d'acide chlorhydrique concentré centré et 20 ml d'eau. Après 1 h, le mélange réactionnel est concentré sous vide. On obtient une mousse qui est triturée dans l'éther puis séchée.
m = 20,7 g.

h) Composé 14

A une solution de 2 g du produit précédemment obtenu et de 2 ml de triéthylamine dans 20 ml de dichlorométhane à -78°C sous azote, on ajoute 0,51 ml de chlorure de benzoyle et on laisse agiter pendant 10 min. Après hydrolyse avec une solution d'hydroxyde de sodium 0,1 N et extraction au dichlorométhane, le produit est purifié par chromatographie, éluant méthanol/dichlorométhane 10/90 (v/v). On récupère ainsi 1,37 g de produit pur dont on fait le chlorhydrate en additionnant de l'éther chlorhydrique jusqu'à pH = 1. On obtient finalement 1,40 g de chlorhydrate sous forme de mousse.
RMN :
7,7-6,6 (m, 13 H) ; 5,35 (s, 1 H) ; 3,8-2,5 (m, 12 H) ; 2,5-1,5 (m, 6 H).

EXEMPLE 15

Chlorhydrate de N-méthyl N-[(dichloro-3,4 phényl)-2 (hydroxy-4 phényl-4 pipéridiny)-4 butyl] N'-benzylurée.

Ar' = 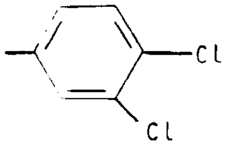 ; 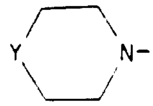 = 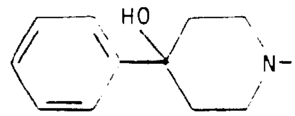 ;

m = 2 ; R = $CH_3$ ; T-Z = 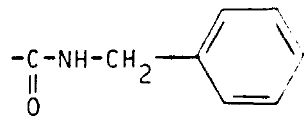

A une solution de 2 g du produit obtenu selon l'exemple 14 g) et de 1,2 ml de triéthylamine dans 20 ml de dichlorométhane à 0°C sous azote, on ajoute 0,60 ml d'isocyanate de benzyle et on laisse agiter le mélange pendant 1 h. Après lavage avec une solution d'hydroxyde de sodium 0,1 N, le produit est purifié par chromatographie sur gel de silice ; éluant méthanol/dichlorométhane 6/94 (v/v). Le produit est ensuite salifié avec une solution d'éther chlorhydrique et on obtient 1,8 g de chlorhydrate.
RMN :
7,7-6,9 (m, 13 H) ; 6,75 (t, 1 H) ; 5,4 (s large, 1 H) ; 4,1 (m, 2 H) ; 3,7-2,5 (m, 12 H) ; 2,5-1,4 (m, 6 H).

EXEMPLE 16

Chlorhydrate de N-méthyl N-[(dichloro-3,4 phényl)-2 (hydroxy-4 phényl-4 pipéridinyl)-4 butyl] carbamate d'éthyle.

Ar'. = 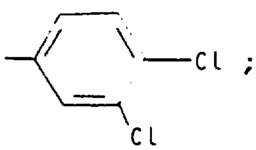 ; 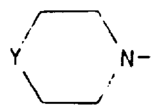 = 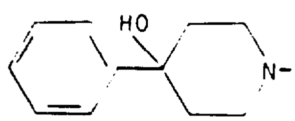 ;

m = 2 ; R = $-CH_3$ ; T-Z = $-\underset{\underset{O}{\|}}{C}-OCH_2CH_3$

A une solution de 2 g de produit obtenu précédemment selon l'exemple 14 g) et 2 ml de triéthylamine dans 20 ml de dichlorométhane à -78°C sous azote, on ajoute 0,44 ml de chloroformiate d'éthyle. Après 5 min, on hydrolyse avec une solution d'hydroxyde de sodium 0,1 N et extrait au dichlorométhane. Le produit est ensuite purifié par chromatographie sur gel de silice ; éluant méthanol/dichlorométhane : 8/92 (v/v). Les fractions pures sont concentrées sous vide et l'addition d'éther chlorhydrique permet d'obtenir 1,1 g de chlorhydrate sous la forme d'une mousse blanche.
RMN :
7,7-7,1 (m, 8 H) ; 5,45 (s, 1 H) ; 4,1-2,6 (m, 14 H) ; 2,6-1,6 (m, 6 H) ; 1,1 (t, 3 H).

Les composés décrits dans les tableaux 4 et 5 ont été synthétisés selon les exemples 14 à 16. Ces composés sont des chlorhydrates.

TABLEAU 4

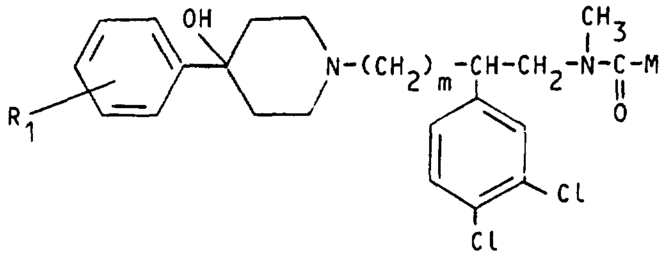

, HCl

| exemple n° | M | $R_1$ | m | Spectre de RMN ou F ; °C |
|---|---|---|---|---|
| 17 | $-CH_3$ | H | 2 | 7,8-7,1 (m, 8 H) ; 3,7-2,65 (m, 12 H) ; 2,65-1,6 (m, 9 H). |
| 18 | $-CH_2-CH_2-CH_3$ | H | 2 | 7,7-7,0 (m, 8 H) ; 3,7-2,55 (m, 12 H) ; 2,55-0,5 (m, 13 H). |
| 19 | 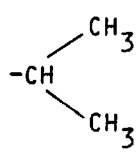 | H | 2 | 7,8-7,1 (m, 8 H) ; 5,5 (s, large, 1H) ; 3,9-2,65 (m, 13 H) ; 2,65-0,5 (m, 12 H). |
| 20 | $-CH_2-$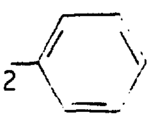 | H | 2 | 7,7-6,8 (m, 13 H) ; 5,35 (s, 1H) ; 3,7-2,5 (m, 14 H) ; 2,5-1,5 (m, 6 H). |
| 21 | 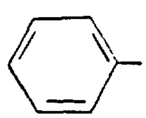 | 4-Cl | 2 | 7,8-6,9 (m, 12 H) ; 5,6 (s, 1 H) ; 3,9-2,6 (m, 12 H) ; 2,6-1,6 (m, 6 H). |

TABLEAU 4 (suite)

| exemple n° | M | $R_1$ | m | Spectre de RMN ou F ; °C |
|---|---|---|---|---|
| 22 | 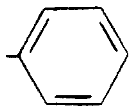 | H | 3 | 148 |
| 23 | 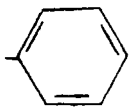 | H | 2 | 198-200 |
| 24 | 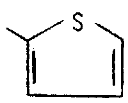 | 4-$CH_3$ | 2 | 7,9-7,0 (m, 10) ; 5,40 (m, 1 H) ; 3,9-2,6 (m, 12 H) ; 2,6-1,6 (m, 9 H). |
| 25 | 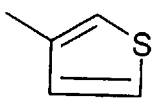 | H | 2 | 7,9-6,5 (m, 11 H) ; 5,45 (s, 1 H ) ; 3,9-2,6 (m, 12 H) ; 2,6-1,6 (m, 6 H). |
| 26 | 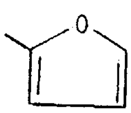 | H | 2 | 7,8-6,4 (m, 11 H) ; 5,3 (s, 1 H) ; 3,9-2,6 (m, 12 H) ; 2,4-1,6 (m, 6 H). |

TABLEAU 4 (suite)

| exemple n° | M | $R_1$ | m | Spectre de RMN ou F ; °C |
|---|---|---|---|---|
| 27 | 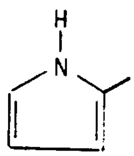 | H | 2 | 7,6-7,2 (m, 8H) ; 6,8 (s, 1H) ; 6,4 (s, 1H) ; 6,05 (s, 1 H) ; 5,4 (s, 1 H); 3,7 (m, 2 H) ; 3,4-2,6 (m, 7 H) ; 3,0 (s, 3 H) ; 2,4 (m, 2 H) ; 2,1 (m, 2 H); 1,7 (m, 2 H). |
| 28 | 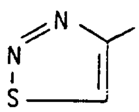 | H | 2 | 203 |
| 29 | 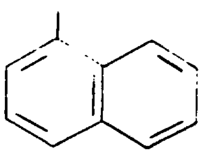 | H | 2 | 198-200 |
| 30 | 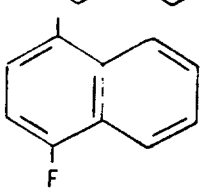 | H | 2 | 180 (décomposition) |

TABLEAU 5

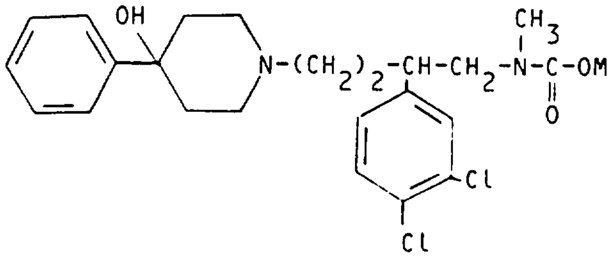

, HCl

| exemple n° | M | Spectre de RMN |
|---|---|---|
| 31 | 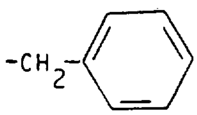 | 7,6-6,9 (m, 13 H) ; 5,35 (s large, 1 H) ; 5,1-4,6 (m, 2 H) ; 3,7-2,5 (m, 12 H) ; 2,5-1,5 (m, 6 H). |
| 32 | 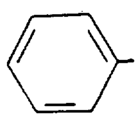 | 7,7-7,0 (m, 11 H) ; 6,85 (d, J = 8 Hz, 1 H) ; 6,75 (d, J = 8 Hz, 1 H) ; 5,35 (s, 1 H) ; 3,8-2,6 (m, 12 H) ; 2,4 (m, 2 H) ; 2,1 (m, 2 H) ; 2,1 (m, 2 H) ; 1,7 (m, 2 H). |

EXEMPLE 33

Chlorhydrate de N-[(dichloro-3,4 phényl)-2 (hydroxy-4 phényl-4 pipéridinyl)-4 butyl] N-méthyl thiophène-2 carboxamide.

Ar' = 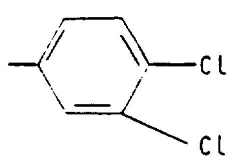 ; 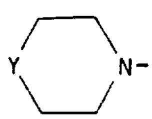 = 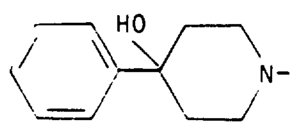 ;

m = 2 ; R = $CH_3$ ; -T-Z = 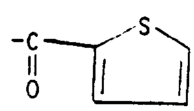

a) N-[(dichloro-3,4 phényl)-2 (tétrahydropyrannyl-2 oxy)-4 butyl] thiophène-2 carboxamide.

On agite à température ambiante un mélange de 4,77 g d'amine obtenue selon l'exemple 1 b) et de 1,7 g de triéthylamine dans 50 ml de dichlorométhane. On ajoute alors goutte à goutte, à température ambiante, 2,19 g de chlorure de thénoyle-2 en solution dans 20 ml de dichlorométhane et laisse le mélange réactionnel à température ambiante pendant une nuit. Le solvant est concentré sous vide, le résidu est lavé à l'eau, extrait à l'éther, lavé avec une solution à 5 % de bicarbonate de sodium puis avec une solution saturée de chlorure de sodium ; la phase organique est décantée, séchée sur $Na_2SO_4$, filtrée et concentrée sous vide. Le résidu est chromatographié sur gel de silice ; éluant : méthanol/dichlorométhane 98/2 (v/v). Les fractions pures sont recueillies et concentrées sous vide. Le résidu est lavé avec une solution d'hydroxyde de sodium à 5 %, extrait à l'éther et séché. On obtient 4,6 g d'huile incolore.

b) N-[(dichloro-3,4 phényl)-2 (tétrahydropyrannyl-2 oxy)-4 butyl] N-méthyl thiophène-2 carboxamide.

On agite à température ambiante un mélange de 3,4 g de l'amide obtenu précédemment et 0,45 g d'hydrure de sodium à 55 % dans 10 ml de tétrahydrofuranne. Le mélange réactionnel devient orange limpide. On ajoute ensuite 1,23 g d'iodométhane dans 10 ml de tétrahydrofuranne et agite ensuite pendant 1 h à température ambiante et chauffe à reflux pendant 1 h. On concentre le tétrahydrofuranne, reprend le résidu dans l'eau, extrait à l'éther, lave une deuxième fois à l'eau avec une solution de chlorure de sodium et concentre sous vide.
m = 3,4 g.

c) N-[(dichloro-3,4 phényl)-2 hydroxy-4 butyl] N-méthyl thiophène-2 carboxamide.

3,5 g du produit obtenu précédemment sont mis en solution dans 30 ml de méthanol en présence de 0,35 g de résine (Amberlyst H-15 Aldrich, résine sulfonique sèche acide) et le mélange est chauffé à reflux pendant 1 heure 30.

Le mélange est filtré sur célite, le filtrat est concentré sous vide, le résidu est lavé à l'hexane puis repris dans un mélange éther/hexane. On obtient 2,6 g de cristaux blancs.
F = 107-109°C.

d) N-[(dichloro-3,4 phényl)-2 méthanesulfonyloxy-4 butyl] N-méthyl thiophène-2 carboxamide.

On agite à température ambiante 2 g d'alcool obtenu précédemment et 0,65 g de triéthylamine dans 30 ml de dichlorométhane. On ajoute ensuite goutte à goutte une solution de 0,69 g de chlorure de mésyle dans 10 ml de dichlorométhane. A la fin de l'addition on chauffe à reflux pendant 30 min. On évapore le dichlorométhane sous vide, reprend le résidu à l'eau, extrait à l'acétate d'éthyle, lave avec une solution de bicarbonate de sodium à 5 %, puis avec une solution saturée de chlorure de sodium, sèche sur $Na_2SO_4$, évapore le solvant.
m = 1,2 g.

e) Composé 33

Un mélange de 1 g du produit obtenu précédemment, de 1 g d'hydroxy-4 phényl-4 pipéridine et de 2 ml de diméthylformamide est chauffé à 60°C pendant 2 h.

Après refroidissement, on dilue avec de l'éther, lave à l'eau puis avec une solution diluée d'hydroxyde de sodium. On sèche sur $MgSO_4$ puis évapore les solvants. Le résidu est chromatographié sur gel de silice ; éluant dichlorométhane jusqu'à dichlorométhane additionné de 2,5 % de méthanol. On obtient 0,7 g de produit dont on fait le chlorhydrate ; après dissolution dans du dichlorométhane, on ajoute de l'éther chlorhydrique jusqu'à pH = 1, puis on concentre sous vide. Le chlorhydrate est concrétisé dans l'éther.
m = 0,74 g.
RMN :
7,8-6,8 (m, 11 H) ; 5,3 (s, 1 H) ; 3,8-2,5 (m, 12 H) ; 2,5-1,4 (m, 6 H).

Les composés décrits dans le tableau 6 ont été préparés selon l'exemple 33. Ces composés sont tous des chlorhydrates.

<u>EXEMPLE 34</u>

Chlorhydrate de N-méthyl N-[(dichloro-3,4 phényl)-2 (hydroxy-4 (hydroxy-4 phényl -4 pipéridinyl)-4butyl)] thiophène-2 carboxamide.

Ar' = 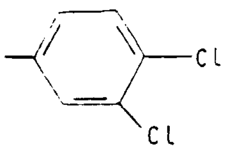 ; 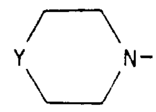 = 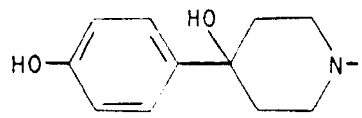 ;

m = 2 ; R = $-CH_3$ ; T-Z = 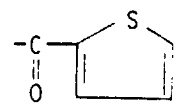

a) Préparation de l'amine : hydroxy-4 (hydroxy-4 phényl)-4 pipéridine.

Etape 1 : benzyloxy-4 bromobenzène.

32,6 g de bromo-4 phénol, 34,2 g de bromure de benzyle et 42 g de carbonate de potassium dans 150 ml de diméthylformamide sont agités à 40°C pendant 2 h.

La solution est concentrée sous vide, le résidu est repris dans l'eau puis on extrait à l'éther, lave à l'eau, décante, sèche sur $MgSO_4$, et concentre sous vide.

Le résidu est recristallisé dans l'isopropanol.

m = 30 g
F = 61°C.

Etape 2 : benzyl-1 (benzyloxy-4 phényl)-4 hydroxy-4 pipéridine.

14 g du produit précédemment préparé sont dissous dans 100 ml de tétrahydrofuranne et additionnés à 1,2 g de magnésium recouverts de 20 ml de tétrahydrofuranne à 60°C. A la fin de l'addition on maintient la température à 60°C pendant 2 h et refroidit ensuite à -10°C. On ajoute alors goutte à goutte une solution de 10 g de benzyl-4 pipéridone et laisse revenir le mélange à température ambiante. Le mélange est versé sur une solution saturée de chlorure d'ammonium, on extrait à l'éther, lave à l'eau, décante, sèche sur $MgSO_4$, et concentre sous vide. Le résidu est chromatographié sur gel de silice, éluant : dichlorométhane/méthanol 97,5/2,5 (v/v). La concentration des fractions pures fournit 9 g du produit attendu.
F = 104-107°C.

Etape 3 : hydroxy-4 (hydroxy-4 phényl)-4 pipéridine.

6 g du produit obtenu précédemment en solution dans 200 ml d'éthanol sont hydrogénés à température ambiante et pression atmosphérique en présence de palladium sur charbon à 10 %. Quand le volume théorique d'hydrogène est absorbé, on filtre le catalyseur, concentre le filtrat sous vide, reprend le résidu à l'éther et filtre les cristaux.
m = 1,1 g
F = 232-235°C.

b) Composé 34

2,1 g du produit précédemment obtenu selon l'exemple 33 d), 1 g de l'amine obtenue à l'étape 3 ci-dessus et 1,1 g de triéthylamine sont mis en solution dans 5 ml de diméthylformamide et sont chauffés à 80°C pendant 2 h. On concentre le mélange sous vide, reprend le résidu dans l'eau et acidifie à pH = 3 avec une solution d'acide chlorhydrique 6 N, extrait à l'acétate d'éthyle, décante, sèche sur $MgSO_4$ et concentre sous vide.

Le résidu est repris dans l'acétone et concrétisé dans l'éther.

m = 0,7 g
RMN :
9,3 (s, 1 H) ; 6,6-8 (m, 10 H) ; 5,2 (s, 1 H) ; 2,6-4 (m, 12 H) ; 1,6-2,4 (m, 6 H).

TABLEAU 6

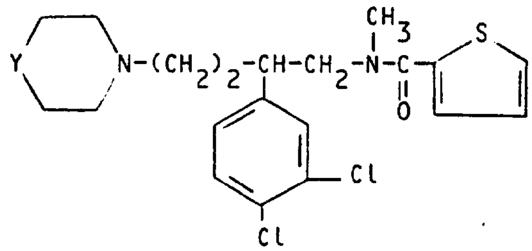

, HCl

| exemple n° | 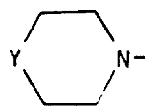 | | Spectre de RMN |
|---|---|---|---|
| 35 | 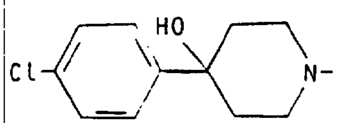 | | 7,8-6,8 (m, 10 H) ; 5,5 (s, 1 H) ; 4,0-2,6 (m, 12 H) ; 2,6-1,5 (m, 6 H). |
| 36 | 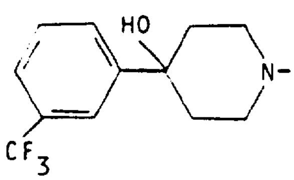 | | 8-7 (m, 10 H) ; 5,8 (s, 1 H) ; 4-2,7 (m, 12 H) ; 2,7-1,7 (m, 6 H). |
| 37 | 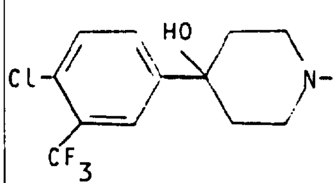 | | 7,85 (s, 1 H) ; 7,75-7,2 (m, 7 H) ; 7,05 (s, 1 H) ; 5,8 (s, 1 H) ; 3,8-2,6 (m, 12 H) ; 2,6-1,6 (m, 6 H). |
| 38 | 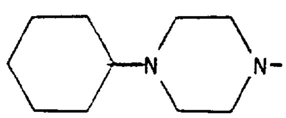 | | 6,8-7,8 (m, 6 H) ; 2,6-4 (m, 17 H) ; 0,8-2,2 (m, 12 H). |
| 39 | 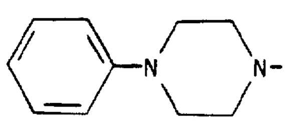 | | 8,2 (s, 2 H) ; 6,7-7,8 (m, 11 H) ; 2-4 m, 18 H). |
| 40 | 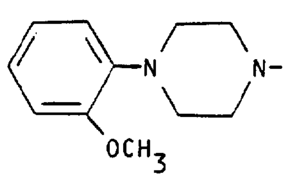 | | 6,7-7,8 (m, 10 H) ; 1,8-4 (m, 10 H). |

TABLEAU 6 (suite)

| exemple n° | 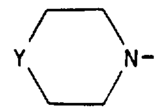 | Spectre de RMN |
| --- | --- | --- |
| 41 | 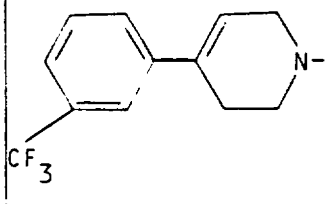 | 7-8 (m, 10 H) ; 6,35 (s, 1 H) ; 2-4,2 (m, 16 H). |

EXEMPLE 42

Chlorhydrate de N-[(benzyl-4 acétoxy-4 pipéridinyl)-4 (dichloro-3,4 phényl)-2 butyl] dichloro-2,4 benzamide.

Ar' = 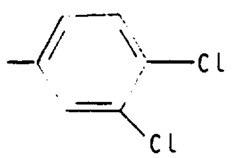 ; 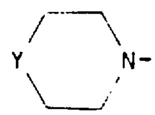 = 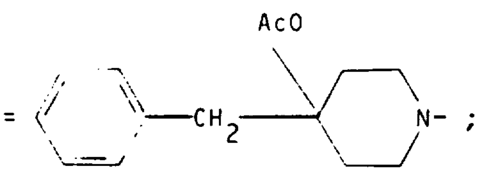 ;

m = 2 ; R = H ; T-Z = 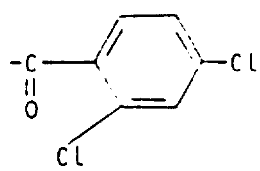

A 0,4 g de chlorhydrate de N-[(benzyl-4 hydroxy-4 pipéridinyl)-4 (dichloro-3,4 phényl)-2 butyl] dichloro-2,4 benzamide (Composé 7 obtenu selon l'exemple 1), en solution dans 10 ml de dichlorométhane en présence de deux équivalents de triéthylamine, on ajoute 0,12 g de chlorure d'acétyle.

Après 1 h d'agitation à température ambiante, on lave à l'eau, décante la phase organique, sèche sur $MgSO_4$, et concentre sous vide. Le résidu est chromatographié sur gel de silice éluant : dichlorométhane puis dichlorométhane/méthanol 95/5 (v/v). Les fractions de produit pur sont concentrées sous vide puis on ajoute de l'éther chlorhydrique jusqu'à pH = 1 et concentre l'éther sous vide.

Le chlorhydrate est concrétisé dans l'éther.

m = 0,26 g

RMN :

8,5 (t, 1 H) ; 7,7-6,98 (m, 11 H) ; 3,6-2,6 (m, 11 H) ; 2,4-1,8 (m, 9 H).

Les composés décrits dans le tableau 7 ont été préparés selon les exemples 1 à 42.

TABLEAU 7

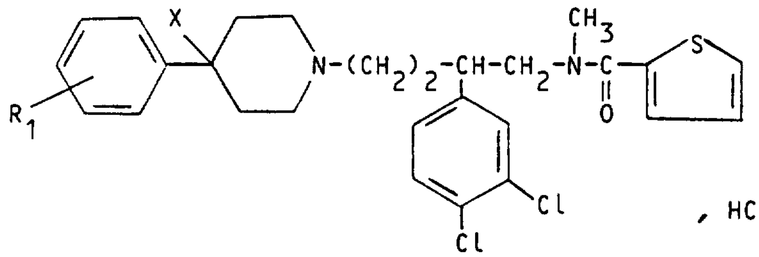

, HCl

| exemple n° | $R_1$ | X | Spectre de RMN |
|---|---|---|---|
| 43 | H | $-O-\underset{\underset{O}{\Vert}}{C}-CH_3$ | 7,9-6,9 (m, 11 H) ; 4,0-2,65 (m, 12 H) ; 2,65-1,8 (m, 9 H). |
| 44 | 4-Cl | $-O-\underset{\underset{O}{\Vert}}{C}-CH_3$ | 7,7-6,9 (m, 10 H) ; 3,8-2,5 (m, 12 H) ; 2,5-2,1 (m, 6 H) ; 2,0 (s, 3 H). |
| 45 | H | -CN | 7-7,9 (m, 11 H) ; 2-4,1 (m, 18 H). |
| 46 | H | $-\underset{\underset{O}{\Vert}}{C}-CH_3$ | 7-7,8 (m, 11 H) ; 1,8-4 (m, 21 H). |
| 47 | H | $-\underset{\underset{O}{\Vert}}{C}-OCH_2CH_3$ | 7-7,9 (m, 11 H) ; 2-4,3 (m, 20 H) ; 1,15 (t, 3 H). |

TABLEAU 8

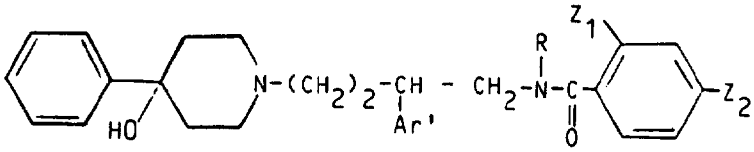

| Exemple n° | Ar' | -R | $Z_1$ | $Z_2$ | Spectre de RMN ou F ; °C |
|---|---|---|---|---|---|
| 48 | 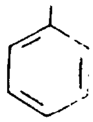 | $CH_3$ | H | H | 186 |
| 49 | 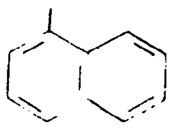 | H | H | H | 148-152 |
| 50 | 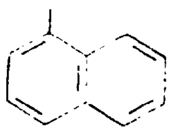 | $CH_3$ | H | H | 144-146 |
| 51 | 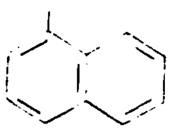 | H | $OCH_3$ | $OCH_3$ | 8,4 (d, J = 8 Hz, 1 H) ; 8,0-7,7 (m, 4 H) ; 7,7-7,2 (m, 8 H) ; 6,6 (m, 2 H) ; 5,4 (s, 1 H); 4,1-2,8 (m, 9 H) ; 3,8 (s, 3 H) ; 3,6 (s, 3 H); 2,5-2,2 (m, 4 H) ; 1,8 (m, 2 H). |
| 52 | 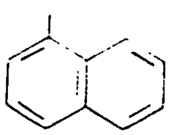 | $CH_3$ | $OCH_3$ | $OCH_3$ | 140-145 (décomposition) |
| 53 | 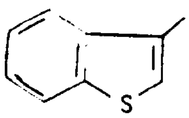 | $CH_3$ | H | H | 118 |
| 54 | 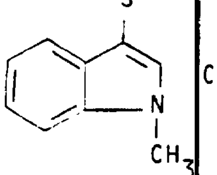 | $CH_3$ | H | H | 7,7-6,7 (m, 15 H) ; 5,4 (m, 1 H) ; 4,0-2,6 (m, 15 H) ; 2,4-1,6 (m, 6 H). |

TABLEAU 9

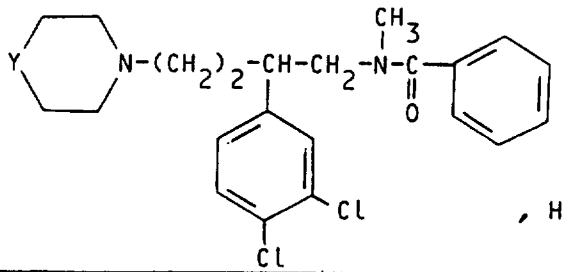

, HCl

| Exemple n° | 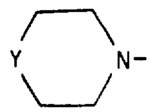 | Spectre de RMN ou F ; °C |
|---|---|---|
| 55 | 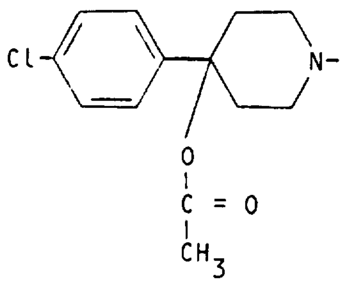 | 7,8-6,9 (m, 12 H) ; 3,9-2,7 (m, 12 H) ; 2,7-1,8 (m, 9 H). |
| 56 | 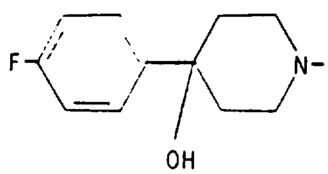 | 200 |
| 57 | 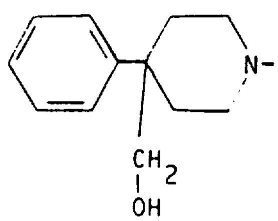 | 7,8-6,8 (m, 13 H) ; 4,6 (m, 1 H) ; 3,8-1,8 (m, 20 H). |
| 58 | 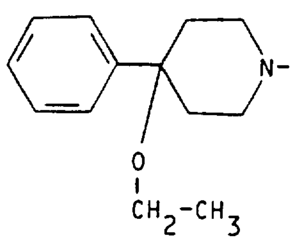 | 7,8-6,9 (m, 13 H) ; 3,8-2,6 (m, 14 H) ; 2,5-1,9 (m, 6 H) ; 1,1 (t, J = 6 Hz, 3 H). |

TABLEAU 9 (suite)

| Exemple n° | 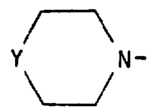 | Spectre de RMN ou F ; °C |
|---|---|---|
| 59 | 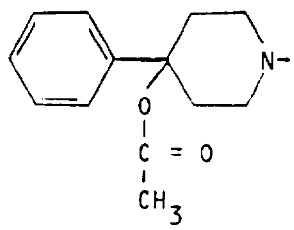 | 203 |
| 60 | 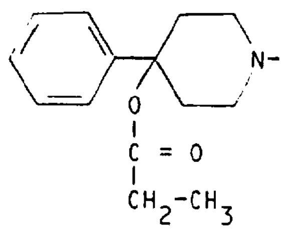 | 198 |
| 61 | 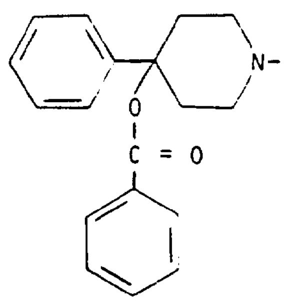 | 140 |
| 62 | 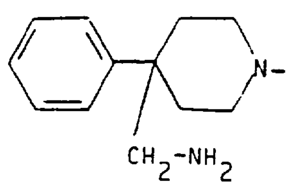 | 163 |
| 63 | 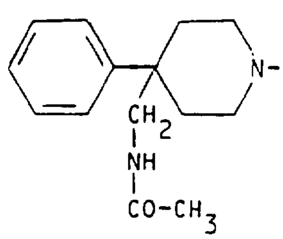 | 144 |

TABLEAU 9 (suite)

| Exemple n° | 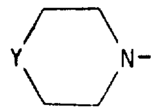 | Spectre de RMN ou F ; °C |
|---|---|---|
| 64 | 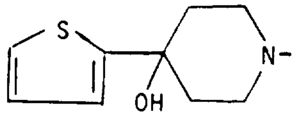 | 188-190 |
| 65 | 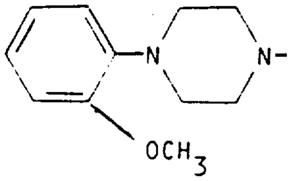 | 134 |
| 66 | 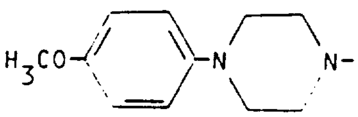 | 114-116 |
| 67 | 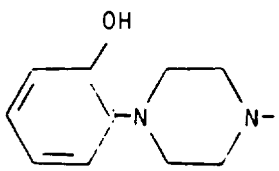 | 128-130 |

TABLEAU 10

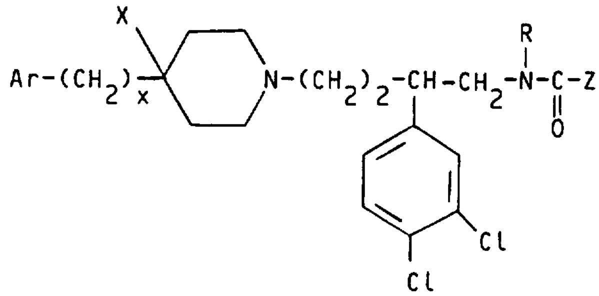

, HCl

| Exemple n° | 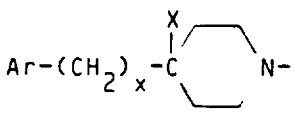 | R | Z | Spectre de RMN ou F ; °C |
|---|---|---|---|---|
| 68 | 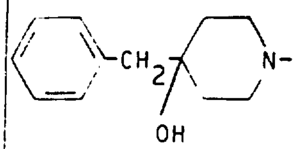 | $CH_3$ | 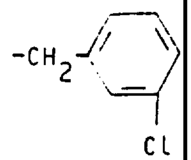 | 7,7-6,8 (m, 12 H) ; 4,7 (s, 1 H) ; 3,6-2,4 (m, 16 H) ; 2,2-1,4 (m, 6 H). |
| 69 | 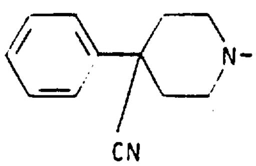 | $CH_3$ | 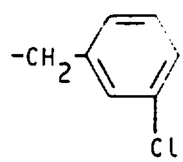 | 7,7-6,8 (m, 22 H) ; 3,7-2,5 (m, 14 H) ; 2,6-2,0 (m, 6 H). |
| 70 | 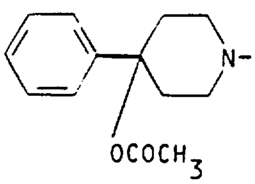 | $CH_3$ | 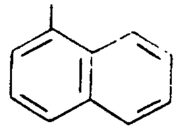 | 232 |

EXEMPLE 71

Chlorhydrate de N-méthyl N-[(phényl-4 acétylamino-4 pipéridinyl)-4 (dichloro-3,4 phényl)-2 butyl] benzamide.

A) Préparation de l'amine :

Chlorhydrate d'acétamido-4 phényl-4 pipéridine.

a) Chlorhydrate d'acétamido-4 phényl-4 benzyl-1 pipéridine.

On ajoute goutte à goutte 260 ml d'acide sulfurique à 95 % à 69 g de benzyl-1 hydroxy-4 phényl-4 pipéridine en suspension dans 300 ml d'acétonitrile en maintenant la température entre 25 et 30°C. Le mélange réactionnel est alors agité à température ambiante pendant 4 h, puis successivement versé dans la glace et neutralisé avec une solution d'hydroxyde de sodium à 30 %.

Le précipité est séparé par filtration, lavé à l'eau puis séché dans l'acétone.

m = 58 g

F = 180,6-182°C

b) Chlorhydrate d'acétamido-4 phényl-4 pipéridine.

A 58 g du produit préparé précédemment, mis en solution dans 600 ml de méthanol, on ajoute de l'éther saturé en acide chlorhydrique jusqu'à pH = 1. On hydrogène ensuite à pression atmosphérique et température ambiante en présence de 6 g de palladium sur charbon à 10 %. Lorsque le volume théorique d'hydrogène est absorbé, on sépare le catalyseur par filtration, concentre le filtrat sous vide et recristallise le résidu dans l'éthanol.

m = 45 g

F = 286,5-288°C.

B) Préparation du composé 71.

a) N-[méthanesulfonyloxy-4 (dichloro-3,4 phényl)-2 butyl] N-méthyl benzamide.

Ce composé est préparé selon l'exemple 1, étape e).

F = 100-102°C.

b) Composé 71

1,4 g d'acétamido-4 phényl-4 pipéridine et 1,4 g du mésylate précédent sont chauffés à 80°C dans 3 ml de DMF pendant 2 h. On ajoute de la glace et extrait au dichlorométhane. La phase organique est décantée et successivement lavée à l'eau puis avec une solution saturée de NaCl et séchée sur $MgSO_4$. On concentre sous vide et le résidu est chromatographié sur gel de silice, éluant : dichlorométhane/méthanol (97/3 ; v/v).

La concentration des fractions du produit pur fournit un résidu qui est repris dans le méthanol. L'addition d'éther saturé d'acide chlorhydrique fournit le chlorhydrate.

m = 0,8 g.

RMN : 3H à 2

$$(s,\ CH_3-\underset{\underset{O}{\|}}{C}-)\ ;$$

18 H entre 2,10 et 3,90 (m, N-$CH_3$, tous les $CH_2$, CH-$C_6H_5$) ; 13 H entre 7,00 et 7,80 (m, H aromatiques) ; 1 H à 8,20

$$(s,\ NH-\underset{\underset{O}{\|}}{C}-).$$

EXEMPLE 72

Chlorhydrate de N-méthyl N-[(phényl-4 acétylamino-4 pipéridinyl)-4 (dichloro-3,4 phényl)-2 butyl] benzamide (-).

Etape 1

Tétrahydropyrannyl-2 oxyéthyl)-α dichloro-3,4 benzèneacétonitrile.

Ce composé est préparé selon l'exemple 1 a).

Etape 2

(Tétrahydropyrannyl-2 oxyéthyl)-β dichloro-3,4 benzèneéthanamine.

Ce composé est préparé selon l'exemple 1 b).

Etape 3

Hydroxyéthyl-β dichloro-3,4 benzèneéthanamine.

81 g du produit obtenu précédemment selon l'étape 2 sont dissous dans 38 ml de méthanol.

On ajoute 80 ml d'une solution saturée d'éther chlorhydrique en maintenant la température entre 20 et 25°C. On agite pendant 30 min à température ambiante, puis concentre à sec. On dissout le résidu dans 250 ml d'eau, lave 2 fois à l'éther, basifie avec une solution de NaOH, extrait au dichlorométhane. Après séchage sur $MgSO_4$, on concentre à sec, reprend dans 800 ml d'éther isopropylique, sépare un insoluble par filtration sur Célite, concentre sous vide à environ 300 ml, amorce avec des cristaux d'aminoalcool, agite pendant une nuit.

On filtre, rince à l'éther isopropylique puis au pentane. On obtient 30,2 g du produit attendu.

F = 90-91°C.

Etape 4

Hydroxyéthyl-β dichloro-3,4 benzèneéthanamine (+).

A une solution bouillante de 29 g d'acide D (-) tartrique dans 800 ml de méthanol, on ajoute une solution de 44,7 g de produit obtenu selon l'étape 3 précédente dans 300 ml de méthanol.

On laisse revenir à température ambiante et agite pendant 4 h. On filtre, rince à l'éthanol puis à l'éther. On obtient 34,1 g de tartrate.

On recristallise dans 1,75 l de méthanol pour obtenir 26,6 g de tartrate.

$[\alpha]_D^{25}$ = -4,2 (c = 1, $H_2O$)

Le tartrate est repris dans 120 ml d'eau. On basifie avec une solution de NaOH, extrait 2 fois au dichlorométhane, sèche sur $MgSO_4$, concentre à sec. On reprend dans un peu d'éther isopropylique, ajoute du pentane, filtre pour obtenir 15,4 g de produit.

F = 79-80°C.

$[\alpha]_D^{25}$ = +9,4 (c = 1, MeOH).

Etape 5

N-[hydroxy-4 (dichloro-3,4 phényl)-2 butyl] carbamate d'éthyle.

15 g de produit obtenu selon l'étape 4 précédente sont dissous dans 200 ml de dichlorométhane. On ajoute 9,9 ml de triéthylamine.

On refroidit à 0°C et ajoute goutte à goutte à cette température une solution de 6,3 ml de chloroformiate d'éthyle dans 30 ml de dichlorométhane. Après 15 min, on lave à l'eau puis avec de l'HCl dilué, puis avec une solution aqueuse saturée de $NaHCO_3$. Après séchage sur $MgSO_4$, on concentre à sec pour obtenir 20 g de produit sous forme d'huile.

Etape 6

Chlorhydrate de N-méthyl hydroxyéthyl-β dichloro-3,4 benzèneéthanamine (+).

A 5,1 g d'hydrure de lithium/aluminium en suspension dans 60 ml de THF anhydre, on ajoute une solution de 20 g de produit obtenu selon l'étape 5 précédente dans 150 ml de THF anhydre. On chauffe à reflux pendant 1 h. On hydrolyse avec 20 ml d'eau, filtre le minéral, concentre à sec. L'huile obtenue est dissoute dans 100 ml d'acétone. On ajoute de l'éther saturé d'acide chlorhydrique jusqu'à pH = 1, puis de l'éther jusqu'au trouble. On agite pendant 1 h, on filtre les cristaux, rince avec un peu d'acétone, puis d'éther pour obtenir 11 g du produit attendu.

F = 129°C.

$[\alpha]_D^{25}$ = +8,4 (c = 1, MeOH).

Etape 7

N-[Hydroxy-4 (dichloro-3,4 phényl)-2 butyl]-N-méthylbenzamide (-).

A 8,1 g de produit obtenu selon l'étape 6 précédente, en suspension dans 120 ml de dichlorométhane, on ajoute 8,4 ml de triéthylamine. On refroidit à 0°C et on ajoute goutte à goutte une solution de 3,4 ml de chlorure de benzoyle dans 35 ml de dichlorométhane. Après 15 min, on lave à l'eau, puis avec de l'HCl dilué, puis une solution aqueuse de

$NaHCO_3$. On sèche sur $MgSO_4$, concentre à sec. On obtient un solide qui est repris dans l'éther et filtré.
m = 9,0 g
F = 129°C.
$[\alpha]_D^{25}$ = -19 (c = 1, MeOH).

Etape 8

N-[Méthanesulfonyloxy-4 (dichloro-3,4 phényl)-2 butyl] N-méthylbenzamide (-).

A 10,5 g de produit obtenu selon l'étape 7 précédente, en solution dans 120 ml de dichlorométhane, on ajoute 4,8 ml de triéthylamine. On refroidit à 0°C et on ajoute goutte à goutte 2,7 ml de chlorure de méthanesulfonyle. Après 15 min, on lave 2 fois à l'eau puis avec une solution aqueuse saturée en NaCl. On sèche sur $MgSO_4$, on concentre à sec pour obtenir une mousse.
$[\alpha]_D^{25}$ = -2,3 (c = 1, $CHCl_3$).

Etape 9

Composé 72

22,7 g de chlorhydrate de phényl-4 acétylamino-4 pipéridine sont dissous dans 20 ml d'eau. On ajoute 10 ml d'une solution d'hydroxyde de sodium concentrée. On extrait 2 fois au dichlorométhane, on sèche sur $MgSO_4$. La solution obtenue est ajoutée au produit obtenu dans l'étape 8. On concentre à sec, on ajoute 30 ml de DMF et chauffe à 70°C pendant 1 h 30. On coule la solution très lentement sur 30 ml d'eau + glace. On filtre le précipité, on le reprend plusieurs fois dans l'eau et on l'essore. On purifie par chromatographie sur silice : élution : dichlorométhane pur, puis dichlorométhane avec addition de méthanol jusqu'à 10 %.

Chlorhydrate : la base est dissoute dans l'acétone. On ajoute de l'éther saturé d'acide chlorhydrique jusqu'à pH = 1. On coule la solution sur de l'éther isopropylique, filtre, sèche.
m = 11 g
$[\alpha]_D^{25}$ = -29,5 (c = 1, MeOH).
RMN : 3 H à 1,85

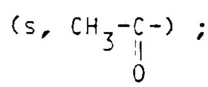

18 H entre 2,00 et 3,75. (m, N - $CH_3$, tous les $CH_2$, CH - $C_6H_5$), 13 H entre 6,80 et 7,70 (m, H aromatiques) ; 1 H à 8,10

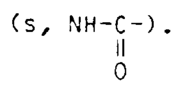

EXEMPLE 73

Chlorhydrate de N-méthyl N-[(phényl-4 acétylamino-4 pipéridinyl)-4 (dichloro-3,4 phényl)-2 butyl] benzamide (+).

L'énantiomère (+) est préparé selon le même mode opératoire que pour l'énantiomère (-) décrit à l'exemple 72 ci-dessus en remplaçant, à l'étape 4, l'acide D (-) tartrique par l'acide L (+) tartrique.
$[\alpha]_D^{25}$ = +30,6 (c = 1, MeOH).
RMN : 3 H à 1,85

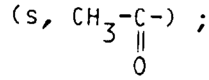

18 H entre 2,00 et 3,75
(m, N-$CH_3$, tous les $CH_2$, CH-$C_6H_5$) ; 13 H entre 6,80 et 7,70 (m, H aromatiques) ; 1 H à 8,10

(s, NH-C-).
||
O

EXEMPLE 74

Chlorhydrate de N-[(dichloro-2,4 phényl)-2 (hydroxy-4 phényl-4 pipéridinyl)-4 butyl] N-méthyl thiophène-2 carboxamide (-).

Ce composé est préparé en procédant selon l'exemple 72 ci-dessus.

$[\alpha]_D^{25}$ = -51,0 (c = 1, MeOH).

EXEMPLE 75

Chlorhydrate de N-[(dichloro-2,4 phényl)-2 (hydroxy-4 phényl-4 pipéridinyl)-4 butyl] N-méthyl thiophène-2 carboxamide (+).

Ce composé est préparé en procédant selon les exemples 72 et 73 ci-dessus.

$[\alpha]_D^{25}$ = +52,7 (c = 1, MeOH).

Les alcools synthétisés selon l'exemple 1 d) ci-dessus ou selon l'exemple 42 sont des intermédiaires clés pour la préparation des composés (I).

Le tableau A ci-dessous décrit différents alcools utiles pour la préparation des composés (I).

## TABLEAU A

Intermédiaires de synthèse

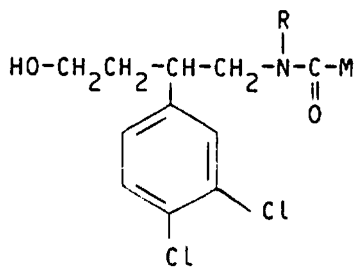

| n° produit | M | R | Spectre de RMN |
|---|---|---|---|
| (a) | 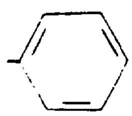 | $CH_3$ | 6,8-7,8 (m, 8 H) ; 4,5 (se, 1 H) ; 2,6-4 (m, 8 H) ; 1,3-2,1 (m, 2 H). |
| (b) | 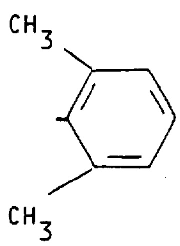 | $CH_3$ | 6,8-7,6 (m, 6 H) ; 3-4,2 (m, 5 H) ; 2,4 (s, 3 H) ; 1,4-2,2 (m, 8 H). |
| (c) | 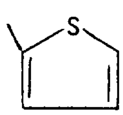 | $CH_3$ | 6,8-7,8 (m, 6 H) ; 4,4 (t, 1 H) ; 2,6-4 (m, 8 H) ; 1,4-1,9 (se, 2 H). |
| (d) | 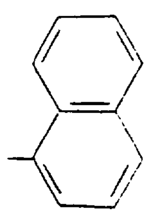 | $CH_3$ | 1,3 (m, 2 H) ; 2,6-5 (m, 9 H) ; 8,2-6,2 (m, 10 H) ; 5-2,6 (m, 9 H) ; 1,3 (m, 2 H). |

## Revendications

**Revendications pour les Etats contractants suivants : AT, BE, CH, DE, DK, FR, GB, IT, LI, LU, NL, SE**

**1.** Un composé de formule :

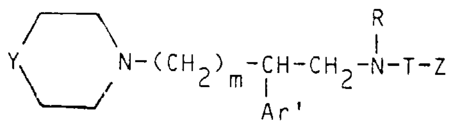

(I)

dans laquelle :

- Y représente - soit un groupe Cy-N dans lequel

  . Cy représente un phényle non substitué ou substitué une ou plusieurs fois par l'un des substituants choisis parmi : l'hydrogène, un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidinyle ou un groupe pyridyle ;

  - soit un groupe

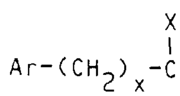

  dans lequel

  . Ar représente un phényle non substitué ou substitué une ou plusieurs fois par l'un des substituants choisis parmi : l'hydrogène, un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;

  . x est zéro ou un ;

  . X représente un hydroxyle, un alcoxy en $C_1$-$C_4$ ; un hydroxyalkyle dans lequel l'alkyle est en $C_1$-$C_3$ ; un acyloxy en $C_1$-$C_4$ ; un phénacyloxy ; un carboxy ; un carbalcoxy en $C_1$-$C_4$ ; un cyano ; un aminoalkylène dans lequel l'alkylène est en $C_1$-$C_3$ ; un groupe -N-$(X_1)_2$ dans lequel les groupes $X_1$ représentent indépendamment l'hydrogène, un alkyle en $C_1$-$C_4$ ; un groupe

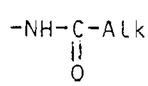

  dans lequel Alk représente un alkyle en $C_1$-$C_6$ ; un groupe

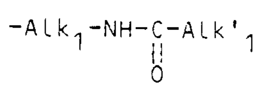

  dans lequel Alk1 est un alkylène en $C_1$-$C_3$ et $Alk'_1$ est un alkyle en $C_1$-$C_3$ ; un acyle en $C_1$-$C_4$ ; un groupe -S-$X_2$ dans lequel $X_2$ représente l'hydrogène ou un groupe alkyle en $C_1$-$C_4$ ;
  ou bien X forme avec l'atome de carbone auquel il est lié et avec l'atome de carbone voisin dans l'hétérocycle une double liaison ;

- m est 2 ou 3 ;
- Ar' représente un phényle non substitué ou substitué une ou plusieurs fois par l'un des substituants choisis parmi : l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ; un indolyle ; un indolyle N substitué par un alkyle en $C_1$-$C_3$ ;
- R représente l'hydrogène, un alkyle en $C_1$-$C_6$ ;
- T représente un groupe choisi parmi

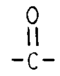

et

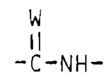

W étant un atome d'oxygène ou de soufre, et

- Z représente soit l'hydrogène, soit M ou OM lorsque T représente le groupe

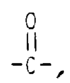

soit M lorsque T représente le groupe

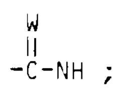

M représente un alkyle en $C_1$-$C_6$ ; un phénylalkyle dans lequel l'alkyle est en $C_1$-$C_3$, éventuellement substitué sur le cycle aromatique par un halogène, un trifluorométhyle, un alkyle en $C_1$-$C_4$, un hydroxyle, un alcoxy en $C_1$-$C_4$ ; un pyridylalkyle dans lequel l'alkyle est en $C_1$-$C_3$ ; un naphtylalkyle dans lequel l'alkyle est en $C_1$-$C_3$, éventuellement substitué sur le cycle naphtyle par un halogène, un trifluorométhyle, un alkyle en $C_1$-$C_4$, un hydroxyle, un alcoxy en $C_1$-$C_4$ ; un pyridylthioalkyle dans lequel l'alkyle est en $C_1$-$C_3$ ; un styryle ;
un groupe phényle, non substitué ou substitué par un ou plusieurs substituants choisis parmi F; Cl ; Br ; I ; CN ; OH ; $NH_2$ ; NH-CO-$NH_2$ ; $NO_2$, $CONH_2$ ; $CF_3$ ; alkyle en $C_1$-$C_{10}$ ; alcényle contenant 2 à 10 atomes de carbone ; alcynyle contenant 2 à 10 atomes de carbone ; cycloalkyle contenant 3 à 8 atomes de carbone ; bicycloalkyle contenant 4 à 11 atomes de carbone ; hydroxyalkyle contenant 1 à 5 atomes de carbone ; alcoxy contenant 1 à 10 atomes de carbone ; alcoxyalkyle contenant 2 à 10 atomes de carbone ; alcoxyalcoxyalkyle contenant jusqu'à 10 atomes de carbone ; alcoxyalcoxy contenant de 2 à 10 atomes de carbone ; alcényloxy contenant 2 à 10 atomes de carbone ; alcényloxyalkyl contenant jusqu'à 10 atomes de carbone ; alcynyloxy contenant de 2 à 10 atomes de carbone ; alcynyloxyalkyle contenant de 3 à 10 atomes de carbone ; cycloalcoxy contenant 3 à 8 atomes de carbone ; alkylthio contenant de 1 à 10 atomes de carbone ; alkylthioalkyle contenant de 2 à 10 atomes de carbone ; acylamino contenant de 1 à 7 atomes de carbone ; acylaminoalkyle contenant de 2 à 8 atomes de carbone ; acyloxy contenant de 1 à 6 atomes de carbone ; alcoxycarbonyle contenant de 2 à 5 atomes de carbone ; cycloalcoxycarbonyle contenant de 4 à 8 atomes de carbone ; alkylaminocarbonylamino contenant de 2 à 4 atomes de carbone ; dialkylaminocarbonylamino contenant de 3 à 7 atomes de carbone ; (pyrrolidino-1)-carbonylamino ; (pipéridino-1)carbonylamino ; cycloalkylaminocarbonylamino contenant de 4 à 8 atomes de carbone ; alkylaminocarbonylaminoalkyle contenant de 3 à 9 atomes de carbone ; dialkylaminocarbonylaminoalkyle contenant de 4 à 11 atomes de carbone ; (pyrrolidino-1)carbonylaminoéthyle (pipéridino-1)-carbonylaminoéthyle ; cycloalkylaminocarbonylaminoalkyle contenant de 5 à 12 atomes de carbone ; alcoxycarbonylaminoalkyle contenant de 3 à 12 atomes de carbone ; cycloalcoxycarbonylaminoalkyle contenant de 5 à 12 atomes de carbone ; carbamoylalkyle contenant de 2 à 5 atomes de carbone ; alkylaminocarbonylalkyle contenant de 3 à 9 atomes de carbone, dialkylaminocarbonylalkyle contenant de 4 à 11 atomes de carbone ; (pyrrolidino-1)carbonylméthyle, (pipéridino-1)carbonylméthyle, (pipéridino-1)carbonyléthyle ; cycloalkylaminocarbonylalkyle contenant de 5 à 12 atomes de carbone ; alkylaminocarbonylalcoxy contenant de 3 à 10 atomes de carbone ; dialkylaminocarbonylalcoxy contenant de 4 à 10 atomes de carbone ; (pipéridinyl-1)carbonylméthoxy ; cycloalkylaminocarbonylalcoxy contenant de 5 à 11 atomes de carbone ;

un groupe 1- ou 2-naphtyle ; un groupe 1-, 2-, 3-, 4-, 5-, 6-, 7- indényle, dont une ou plusieurs liaisons peuvent être hydrogénées, lesdits groupes naphtyle ou indényle pouvant être non substitués ou contenir éventuellement un ou plusieurs substituants tels que : le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$ ; un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en $C_1$-$C_4$, ou un de ses sels avec des acides minéraux ou organiques.

**2.** Composé selon la revendication 1, dans lequel Ar' est un groupe dichloro-3,4 phényle ou un de ses sels avec des acides minéraux ou organiques.

**3.** Composé selon l'une des revendications 1 ou 2, dans lequel X est un hydroxyle, un acétyloxy ou un groupe

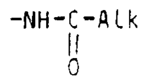

dans lequel Alk représente un alkyle en $C_1$-$C_6$ ou un de ses sels avec des acides minéraux ou organiques.

**4.** Composé selon l'une quelconque des revendications 1 à 3, dans lequel R est un méthyle ou un de ses sels minéraux ou organiques.

**5.** Composé selon l'une quelconque des revendications 1 à 4, dans lequel T est un groupe -C = O ou un de ses sels minéraux ou organiques.

**6.** Composé selon l'une quelconque des revendications 1 à 5, dans lequel T est un groupe -C = O et Z est un groupe thiényle ou un de ses sels minéraux ou organiques.

**7.** Composé selon l'une quelconque des revendications 1 à 5, dans lequel T est un groupe -C = O et Z un groupe phényle, éventuellement disubstitué par un halogène, tel que le chlore ou un de ses sels minéraux ou organiques.

**8.** Composé selon l'une quelconque des revendications 1 à 7, caractérisé en ce qu'il est le N-méthyl [(phényl-4 acétylamino-4 pipéridinyl)-4 (dichloro-3,4 phényl)-2 butyl] benzamide sous forme du racémate ou l'un de ses sels avec des acides minéraux ou organiques.

**9.** N-méthyl [(phényl-4 acétylamino-4 pipéridinyl)-4 (dichloro-3,4 phényl)-2 butyl]benzamide (-) et ses sels avec des acides minéraux ou organiques.

**10.** N-méthyl [(phényl-4 acétylamino-4 pipéridinyl)-4 (dichloro-3,4 phényl)-2 butyl] benzamide (+) et ses sels avec des acides minéraux ou organiques.

**11.** Procédé pour la préparation de composés selon l'une quelconque des revendications 1 à 10, caractérisé en ce que

- a) on traite une amine libre de formule :

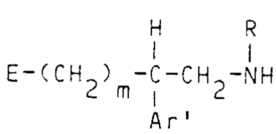

(II)

dans laquelle m, Ar' et R sont tels que définis précédemment dans la revendication 1 et E représente un groupe O-protecteur tel que par exemple tétrahydropyranyl-2 oxy ou un groupe

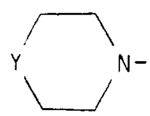

dans lequel Y est tel que défini précédemment dans la revendication 1 étant entendu que
lorsque Y représente le groupe

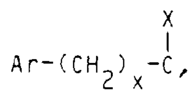

où X est un hydroxyle, cet hydroxyle est protégé, ou une amine de formule :

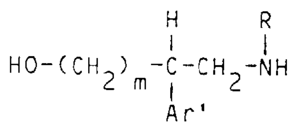

(II''')

dans laquelle m, Ar', et R sont tels que définis précédemment, ladite amine de formule (II''') étant éventuellement sous forme optiquement pure ;

- soit avec un dérivé fonctionnel d'un acide de formule :

  HO - CO - Z (III)

  dans laquelle Z est tel que défini précédemment dans la revendication 1, lorsqu'on doit préparer un composé de formule (I) où T est -CO-,
- soit avec un iso(thio)cyanate de formule :

  W = C = N - Z (III')

  dans laquelle W et Z sont tels que définis précédemment dans la revendication 1, lorsqu'on doit préparer un composé de formule (I) où T est -C(W)-NH-,
  pour former le composé de formule :

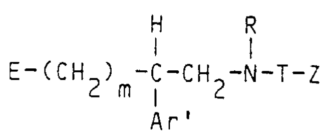

(IV)

- b) puis, lorsque E représente le tétrahydropyranyloxy, on élimine le groupe tétrahydropyranyle par action d'un acide, ladite déprotection pouvant être réalisée directement sur le composé de formule (II) pour fournir le composé de formule (II''') qui est alors traité avec l'un des composés de formule (III) ou (III'),
- c) on traite l'alcanolamine N-substituée ainsi obtenue de formule :

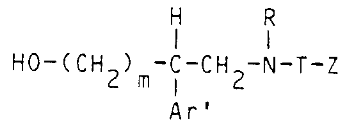

(V)

avec le chlorure de méthanesulfonyle

- d) on fait réagir le mésylate ainsi obtenu de formule :

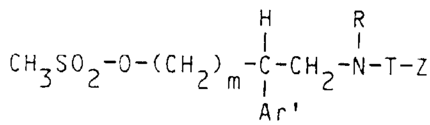

(VI)

avec une amine secondaire de formule :

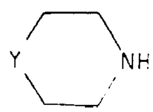

(VII)

dans laquelle Y est tel que défini précédemment dans la revendication 1 ; et

- e) après déprotection éventuelle de l'hydroxyle représenté par X, on transforme éventuellement le produit ainsi obtenu en un de ses sels.

**12.** Composition pharmaceutique contenant en tant que principe actif un composé de formule (I) selon l'une quelconque des revendications 1 à 10.

**13.** Composition pharmaceutique selon la revendication 12, sous forme d'unité de dosage, dans laquelle le principe actif est mélangé à au moins un excipient pharmaceutique.

**Revendications pour l'Etat contractant suivant : GR**

**1.** Un composé de formule :

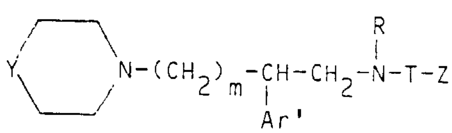

(I)

dans laquelle :

- Y représente - soit un groupe Cy-N dans lequel

  . Cy représente un phényle non substitué ou substitué une ou plusieurs fois par l'un des substituants choisis parmi : l'hydrogène, un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidinyle ou un groupe pyridyle ;

  - soit un groupe

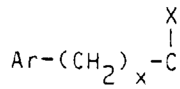

dans lequel

. Ar représente un phényle non substitué ou substitué une ou plusieurs fois par l'un des substituants choisis parmi : l'hydrogène, un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;
. x est zéro ou un ;
. X représente un hydroxyle, un alcoxy en $C_1$-$C_4$ ; un hydroxyalkyle dans lequel l'alkyle est en $C_1$-$C_3$ ; un acyloxy en $C_1$-$C_4$ ; un phénacyloxy ; un carboxy ; un carbalcoxy en $C_1$-$C_4$ ; un cyano ; un aminoalkylène dans lequel l'alkylène est en $C_1$-$C_3$ ; un groupe -N-$(X_1)_2$ dans lequel les groupes $X_1$ représentent indépendamment l'hydrogène, un alkyle en $C_1$-$C_4$ ; un groupe

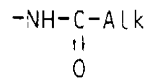

dans lequel Alk représente un alkyle en $C_1$-$C_6$ ; un groupe

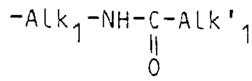

dans lequel $Alk_1$ est un alkylène en $C_1$-$C_3$ et $Alk'_1$ est un alkyle en $C_1$-$C_3$ ; un acyle en $C_1$-$C_4$ ; un groupe -S-$X_2$ dans lequel $X_2$ représente l'hydrogène ou un groupe alkyle en $C_1$-$C_4$ ; ou bien X forme avec l'atome de carbone auquel il est lié et avec l'atome de carbone voisin dans l'hétérocycle une double liaison ;

- m est 2 ou 3 ;

  - Ar' représente un phényle non substitué ou substitué une ou plusieurs fois par l'un des substituants choisis parmi : l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ; un indolyle ; un indolyle N substitué par un alkyle en $C_1$-$C_3$ ;

- R représente l'hydrogène, un alkyle en $C_1$-$C_6$ ;
- T représente un groupe choisi parmi

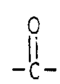 et 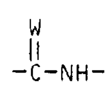

W étant un atome d'oxygène ou de soufre, et
- Z représente soit l'hydrogène, soit M ou OM lorsque T représente le groupe

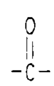,

soit M lorsque T représente le groupe

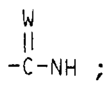

M représente un alkyle en $C_1$-$C_6$ ; un phénylalkyle dans lequel l'alkyle est en $C_1$-$C_3$, éventuellement substitué sur le cycle aromatique par un halogène, un trifluorométhyle, un alkyle en $C_1$-$C_4$, un hydroxyle, un alcoxy en $C_1$-$C_4$ ; un pyridylalkyle dans lequel l'alkyle est en $C_1$-$C_3$ ; un naphtylalkyle dans lequel l'alkyle est en $C_1$-$C_3$, éventuellement substitué sur le cycle naphtyle par un halogène, un trifluorométhyle, un alkyle en $C_1$-$C_4$, un hydroxyle, un alcoxy en $C_1$-$C_4$ ; un pyridylthioalkyle dans lequel l'alkyle est en $C_1$-$C_3$ ; un styryle ; un groupe phényle, non substitué ou substitué par un ou plusieurs substituants choisis parmi F; Cl ; Br ; I ; CN ; OH ; $NH_2$ ; NH-CO-$NH_2$ ; $NO_2$, $CONH_2$ ; $CF_3$ ; alkyle en $C_1$-$C_{10}$ ; alcényle contenant 2 à 10 atomes de carbone ; alcynyle contenant 2 à 10 atomes de carbone ; cycloalkyle contenant 3 à 8 atomes de carbone ; bicycloalkyle contenant 4 à 11 atomes de carbone ; hydroxyalkyle contenant 1 à 5 atomes de carbone ; alcoxy contenant 1 à 10 atomes de carbone ; alcoxyalkyle contenant 2 à 10 atomes de carbone ; alcoxyalcoxyalkyle contenant jusqu'à 10 atomes de carbone ; alcoxyalcoxy contenant de 2 à 10 atomes de carbone ; alcényloxy contenant 2 à 10 atomes de carbone ; alcényloxyalkyl contenant jusqu'à 10 atomes de carbone ; alcynyloxy contenant de 2 à 10 atomes de carbone ; alcynyloxyalkyle contenant de 3 à 10 atomes de carbone ; cycloalcoxy contenant 3 à 8 atomes de carbone ; alkylthio contenant de 1 à 10 atomes de carbone ; alkylthioalkyle contenant de 2 à 10 atomes de carbone ; acylamino contenant de 1 à 7 atomes de carbone ; acylaminoalkyle contenant de 2 à 8 atomes de carbone ; acyloxy contenant de 1 à 6 atomes de carbone ; alcoxycarbonyle contenant de 2 à 5 atomes de carbone ; cycloalcoxycarbonyle contenant de 4 à 8 atomes de carbone ; alkylaminocarbonylamino contenant de 2 à 4 atomes de carbone ; dialkylaminocarbonylamino contenant de 3 à 7 atomes de carbone ; (pyrrolidino-1)-carbonylamino ; (pipéridino-1)carbonylamino ; cycloalkylaminocarbonylamino contenant de 4 à 8 atomes de carbone ; alkylaminocarbonylaminoalkyle contenant de 3 à 9 atomes de carbone ; dialkylaminocarbonylaminoalkyle contenant de 4 à 11 atomes de carbone ; (pyrrolidino-1) carbonylaminoéthyle , (pipéridino-1)-carbonylaminoéthyle ; cycloalkylaminocarbonylaminoalkyle contenant de 5 à 12 atomes de carbone ; alcoxycarbonylaminoalkyle contenant de 3 à 12 atomes de carbone ; cycloalcoxycarbonylaminoalkyle contenant de 5 à 12 atomes de carbone ; carbamoylalkyle contenant de 2 à 5 atomes de carbone ; alkylaminocarbonylalkyle contenant de 3 à 9 atomes de carbone, dialkylaminocarbonylalkyle contenant de 4 à 11 atomes de carbone ; (pyrrolidino-1)carbonylméthyle, (pipéridino-1)carbonylméthyle, (pipéridino-1)carbonyléthyle ; cycloalkylaminocarbonylalkyle contenant de 5 à 12 atomes de carbone ; alkylaminocarbonylalcoxy contenant de 3 à 10 atomes de carbone ; dialkylaminocarbonylalcoxy contenant de 4 à 10 atomes de carbone ; (pipéridinyl-1)carbonylméthoxy ; cycloalkylaminocarbonylalcoxy contenant de 5 à 11 atomes de carbone ;
un groupe 1- ou 2-naphtyle ; un groupe 1-, 2-, 3-, 4-, 5-, 6-, 7- indényle, dont une ou plusieurs liaisons peuvent être hydrogénées, lesdits groupes naphtyle ou indényle pouvant être non substitués ou contenir éventuellement un ou plusieurs substituants tels que : le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$ ; un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en $C_1$-$C_4$, ou un de ses sels avec des acides minéraux ou organiques.

2. Composé selon la revendication 1, dans lequel Ar' est un groupe dichloro-3,4 phényle ou un de ses sels avec des acides minéraux ou organiques.

3. Composé selon l'une des revendications 1 ou 2, dans lequel X est un hydroxyle, un acétyloxy ou un groupe

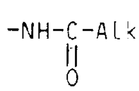

dans lequel Alk représente un alkyle en $C_1$-$C_6$ ou un de ses sels avec des acides minéraux ou organiques.

**4.** Composé selon l'une quelconque des revendications 1 à 3, dans lequel R est un méthyle ou un de ses sels minéraux ou organiques.

**5.** Composé selon l'une quelconque des revendications 1 à 4, dans lequel T est un groupe -C = O ou un de ses sels minéraux ou organiques.

**6.** Composé selon l'une quelconque des revendications 1 à 5, dans lequel T est un groupe -C = O et Z est un groupe thiényle ou un de ses sels minéraux ou organiques.

**7.** Composé selon l'une quelconque des revendications 1 à 5, dans lequel T est un groupe -C = O et Z un groupe phényle, éventuellement disubstitué par un halogène, tel que le chlore ou un de ses sels minéraux ou organiques.

**8.** Composé selon l'une quelconque des revendications 1 à 7, caractérisé en ce qu'il est le N-méthyl [(phényl-4 acétylamino-4 pipéridinyl)-4 (dichloro-3,4 phényl)-2 butyl] benzamide sous forme du racémate ou l'un de ses sels avec des acides minéraux ou organiques.

**9.** N-méthyl [(phényl-4 acétylamino-4 pipéridinyl)-4 (dichloro-3,4 phényl)-2 butyl] benzamide (-) et ses sels avec des acides minéraux ou organiques.

**10.** N-méthyl [(phényl-4 acétylamino-4 pipéridinyl)-4 (dichloro-3,4 phényl)-2 butyl] benzamide (+) et ses sels avec des acides minéraux ou organiques.

**11.** Procédé pour la préparation de composés selon l'une quelconque des revendications 1 à 10, caractérisé en ce que

- a) on traite une amine libre de formule :

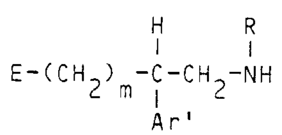

(II)

dans laquelle m, Ar' et R sont tels que définis précédemment dans la revendication 1 et E représente un groupe O-protecteur tel que par exemple tétrahydropyranyl-2 oxy ou un groupe

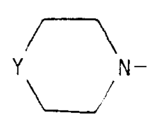

dans lequel Y est tel que défini précédemment dans la revendication 1 étant entendu que lorsque Y représente le groupe

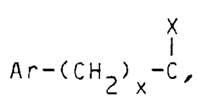

où X est un hydroxyle, cet hydroxyle est protégé, ou une amine de formule :

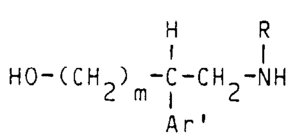

(II''')

dans laquelle m, Ar', et R sont tels que définis précédemment, ladite amine de formule (II''') étant éventuellement sous forme optiquement pure ;

- soit avec un dérivé fonctionnel d'un acide de formule :

$$HO - CO - Z \qquad (III)$$

  dans laquelle Z est tel que défini précédemment dans la revendication 1, lorsqu'on doit préparer un composé de formule (I) où T est -CO-,
- soit avec un iso(thio)cyanate de formule :

$$W = C = N - Z \qquad (III')$$

  dans laquelle W et Z sont tels que définis précédemment dans la revendication 1, lorsqu'on doit préparer un composé de formule (I) où T est -C(W)-NH-,
  pour former le composé de formule :

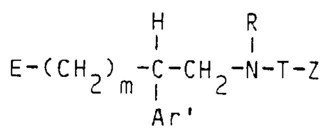

(IV)

- b) puis, lorsque E représente le tétrahydropyranyloxy, on élimine le groupe tétrahydropyranyle par action d'un acide, ladite déprotection pouvant être réalisée directement sur le composé de formule (II) pour fournir le composé de formule (II''') qui est alors traité avec l'un des composés de formule (III) ou (III'),
- c) on traite l'alcanolamine N-substituée ainsi obtenue de formule :

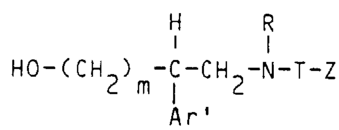

(V)

  avec le chlorure de méthanesulfonyle
- d) on fait réagir le mésylate ainsi obtenu de formule :

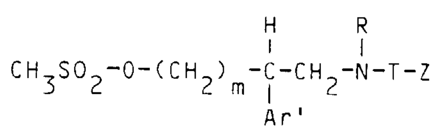

(VI)

  avec une amine secondaire de formule :

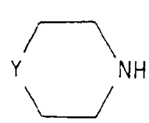

(VII)

  dans laquelle Y est tel que défini précédemment dans la revendication 1 ; et
- e) après déprotection éventuelle de l'hydroxyle représenté par X, on transforme éventuellement le produit

ainsi obtenu en un de ses sels.

**12.** Procédé pour la préparation d'une composition pharmaceutique, caractérisé en ce que l'on mélange, en tant que principe actif, un composé selon l'une quelconque des revendication 1 à 10, avec un véhicule pharmaceutiquement acceptable.

**Revendications pour l'Etat contractant suivant : ES**

**1.** Procédé pour la préparation de composés de formule :

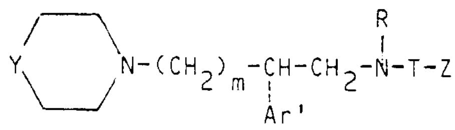 (I)

dans laquelle :

- Y représente - soit un groupe Cy-N dans lequel

  . Cy représente un phényle non substitué ou substitué une ou plusieurs fois par l'un des substituants choisis parmi : l'hydrogène, un atome d'halogène, un hydroxyle, un alcoxy en $C_1$-$C_4$, un alkyle en $C_1$-$C_4$, un trifluorométhyle, lesdits substituants étant identiques ou différents ; un groupe cycloalkyle en $C_3$-$C_7$ ; un groupe pyrimidinyle ou un groupe pyridyle ;

  - soit un groupe

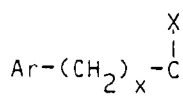

dans lequel

  . Ar représente un phényle non substitué ou substitué une ou plusieurs fois par l'un des substituants choisis parmi : l'hydrogène, un atome d'halogène, un hydroxyle, un alcoxy en $C_1$-$C_4$, un trifluorométhyle, un alkyle en $C_1$-$C_4$, lesdits substituants étant identiques ou différents ; un groupe pyridyle ; un groupe thiényle ;
  . x est zéro ou un ;
  . X représente un hydroxyle, un alcoxy en $C_1$-$C_4$ ; un hydroxyalkyle dans lequel l'alkyle est en $C_1$-$C_3$ ; un acyloxy en $C_1$-$C_4$ ; un phénacyloxy ; un carboxy ; un carbalcoxy en $C_1$-$C_4$ ; un cyano ; un aminoalkylène dans lequel l'alkylène est en $C_1$-$C_3$ ; un groupe -N-$(X_1)_2$ dans lequel les groupes $X_1$ représentent indépendamment l'hydrogène, un alkyle en $C_1$-$C_4$ ; un groupe

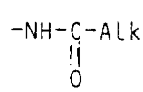

dans lequel Alk représente un alkyle en $C_1$-$C_6$ ; un groupe

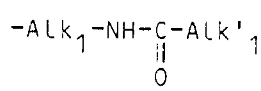

dans lequel $Alk_1$ est un alkylène en $C_1$-$C_3$ et $Alk'_1$ est un alkyle en $C_1$-$C_3$ ; un acyle en $C_1$ -$C_4$ ; un groupe -S-$X_2$ dans lequel $X_2$ représente l'hydrogène ou un groupe alkyle en $C_1$-$C_4$ ;
ou bien X forme avec l'atome de carbone auquel il est lié et avec l'atome de carbone voisin dans l'hétérocycle une double liaison ;

- m est 2 ou 3 ;
- Ar' représente un phényle non substitué ou substitué une ou plusieurs fois par l'un des substituants choisis parmi : l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ; un indolyle ; un indolyle N substitué par un alkyle en $C_1$-$C_3$ ;
- R représente l'hydrogène, un alkyle en $C_1$-$C_6$ ;
- T représente un groupe choisi parmi

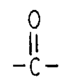 et 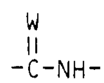

W étant un atome d'oxygène ou de soufre, et
- Z représente soit l'hydrogène, soit M ou OM lorsque T représente le groupe

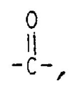

soit M lorsque T représente le groupe

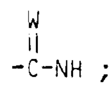

M représente un alkyle en $C_1$-$C_6$ ; un phénylalkyle dans lequel l'alkyle est en $C_1$-$C_3$, éventuellement substitué sur le cycle aromatique par un halogène, un trifluorométhyle, un alkyle en $C_1$-$C_4$, un hydroxyle, un alcoxy en $C_1$-$C_4$ ; un pyridylalkyle dans lequel l'alkyle est en $C_1$-$C_3$ ; un naphtylalkyle dans lequel l'alkyle est en $C_1$-$C_3$, éventuellement substitué sur le cycle naphtyle par un halogène, un trifluorométhyle, un alkyle en $C_1$-$C_4$, un hydroxyle, un alcoxy en $C_1$-$C_4$ ; un pyridylthioalkyle dans lequel l'alkyle est en $C_1$-$C_3$ ; un styryle ;
un groupe phényle, non substitué ou substitué par un ou plusieurs substituants choisis parmi F; Cl ; Br ; I ; CN ; OH ; $NH_2$ ; NH-CO-$NH_2$; $NO_2$, $CONH_2$ ; $CF_3$ ; alkyle en $C_1$-$C_{10}$ ; alcényle contenant 2 à 10 atomes de carbone ; alcynyle contenant 2 à 10 atomes de carbone ; cycloalkyle contenant 3 à 8 atomes de carbone ; bicycloalkyle contenant 4 à 11 atomes de carbone ; hydroxyalkyle contenant 1 à 5 atomes de carbone ; alcoxy contenant 1 à 10 atomes de carbone ; alcoxyalkyle contenant 2 à 10 atomes de carbone ; alcoxyalcoxyalkyle contenant jusqu'à 10 atomes de carbone ; alcoxyalcoxy contenant de 2 à 10 atomes de carbone ; alcényloxy contenant 2 à 10 atomes de carbone ; alcényloxyalkyl contenant jusqu'à 10 atomes de carbone ; alcynyloxy contenant de 2 à 10 atomes de carbone ; alcynyloxyalkyle contenant de 3 à 10 atomes de carbone ; cycloalcoxy contenant 3 à 8 atomes de carbone ; alkylthio contenant de 1 à 10 atomes de carbone ; alkylthioalkyle contenant de 2 à 10 atomes de carbone ; acylamino contenant de 1 à 7 atomes de carbone ; acylaminoalkyle contenant de 2 à 8 atomes de carbone ; acyloxy contenant de 1 à 6 atomes de carbone ; alcoxycarbonyle contenant de 2 à 5 atomes de carbone ; cycloalcoxycarbonyle contenant de 4 à 8 atomes de carbone ; alkylaminocarbonylamino contenant de 2 à 4 atomes de carbone ; dialkylaminocarbonylamino contenant de 3 à 7 atomes de carbone ; (pyrrolidino-1)carbonylamino ; (pipéridino-1)carbonylamino ; cycloalkylaminocarbonylamino contenant de 4 à 8 atomes de carbone ; alkylaminocarbonylaminoalkyle contenant de 3 à 9 atomes de carbone ; dialkylaminocarbonylaminoalkyle contenant de 4 à 11 atomes de carbone ; (pyrrolidino-1) carbonylaminoéthyle ; ( pipéridino-1)-carbonylaminoéthyle ; cycloalkylaminocarbonylaminoalkyle contenant de 5 à 12 atomes de carbone ; alcoxycarbonylaminoalkyle contenant de 3 à 12 atomes de carbone ; cycloalcoxycarbonylaminoalkyle contenant de 5 à 12 atomes de carbone ; carbamoylalkyle contenant de 2 à 5 atomes de carbone ; alkylaminocarbonylalkyle contenant de 3 à 9 atomes de carbone, dialkylaminocarbonylalkyle contenant de 4 à 11 atomes de carbone ; (pyrrolidino-1)carbonylméthyle, (pipéridino-1)carbonylméthyle, (pipéridino-1)carbonyléthyle ; cycloalkylaminocarbonylalkyle contenant de 5 à 12 atomes de carbone ; alkylaminocarbonylalcoxy contenant de 3 à 10 atomes de carbone ; dialkylaminocarbonylalcoxy contenant de 4 à 10 atomes de carbone ; (piperidinyl-1)carbonylméthoxy ; cycloalkylaminocarbonylalcoxy contenant de 5 à 11 atomes de carbone ;
un groupe 1- ou 2-naphtyle ; un groupe 1-, 2-, 3-, 4-, 5-, 6-, 7- indényle, dont une ou plusieurs liaisons peuvent être hydrogénées, lesdits groupes naphtyle ou indényle pouvant être non substitués ou contenir éventuellement un ou plusieurs substituants tels que : le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$ ; un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en $C_1$-$C_4$, ou un de ses sels avec des acides minéraux ou organiques,

caractérisé en ce que :

- a) on traite une amine libre de formule :

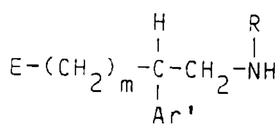

(II)

dans laquelle m, Ar' et R sont tels que définis précédemment et E représente un groupe O-protecteur tel que par exemple tétrahydropyranyl-2 oxy ou un groupe

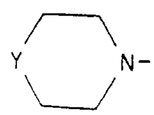

dans lequel Y est tel que défini précédemment étant entendu que lorsque Y représente le groupe

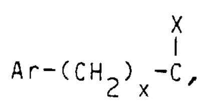

où X est un hydroxyle, cet hydroxyle est protégé, ou une amine de formule :

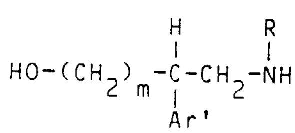

(II''')

dans laquelle m, Ar', et R sont tels que définis précédemment, ladite amine de formule (II''') étant éventuellement sous forme optiquement pure ;

- soit avec un dérivé fonctionnel d'un acide de formule :

HO - CO - Z (III)

dans laquelle Z est tel que défini précédemment, lorsqu'on doit préparer un composé de formule (I) où T est -CO-,

- soit avec un iso(thio)cyanate de formule :

W = C = N - Z (III')

dans laquelle W et Z sont tels que définis précédemment, lorsqu'on doit préparer un composé de formule (I) où T est -C(W)-NH-, pour former le composé de formule :

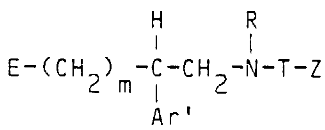

(IV)

- b) puis, lorsque E représente le tétrahydropyranyloxy, on élimine le groupe tétrahydropyranyle par action d'un acide, ladite déprotection pouvant être réalisée directement sur le composé de formule (II) pour fournir le composé de formule (II''') qui est alors traité avec l'un des composés de formule (III) ou (III') ;
- c) on traite l'alcanolamine N-substituée ainsi obtenue de formule :

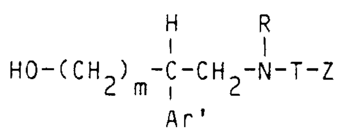

(V)

avec le chlorure de méthanesulfonyle

- d) on fait réagir le mésylate ainsi obtenu de formule :

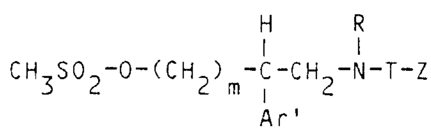

(VI)

avec une amine secondaire de formule :

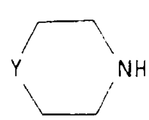

(VII)

dans laquelle Y est tel que défini précédemment ; et

- e) après déprotection éventuelle de l'hydroxyle représenté par X, on transforme éventuellement le produit ainsi obtenu en un de ses sels.

**2.** Procédé selon la revendication 1, caractérisé en ce que l'on utilise une amine libre de formule (II) dans laquelle E, m et R sont tels que définis dans la revendication 1 et Ar' est le groupe dichloro-3,4 phényle.

**3.** Procédé selon l'une des revendications 1 ou 2, caractérisé en ce que l'on utilise une amine libre de formule (II)

dans laquelle :

- Ar', m et R sont tels que définis dans l'une des revendications 1 ou 2 ;
- E représente le groupe :

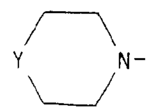

dans lequel Y est un groupe

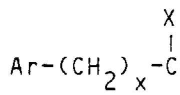

dans lequel :

Ar et x sont tels que définis dans la revendication 1 et X est un groupe hydroxyle, acétoxy ou un groupe

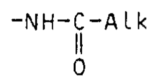

dans lequel Alk représente un alkyle en $C_1$-$C_6$.

**4.** Procédé selon l'une quelconque des revendications 1 à 3, caractérisé en ce que l'on utilise une amine de formule (II) dans laquelle :

- E, m et Ar' sont tels que définis dans l'une quelconque des revendications 1 à 3,
- R est le groupe méthyle.

**5.** Procédé selon l'une quelconque des revendications 1 à 4, caractérisé en ce que l'on utilise un dérivé fonctionnel d'un acide de formule (III) dans laquelle Z est tel que défini dans la revendication 1.

**6.** Procédé selon la revendication 5, caractérisé en ce que Z est un groupe thiényle.

**7.** Procédé selon la revendication 5, caractérisé en ce que Z est un groupe phényle, éventuellement disubstitué par un halogène, tel que le chlore.

**8.** Procédé selon la revendication 1, caractérisé en ce que

1) à l'étape a), on fait réagir une amine de formule II dans laquelle :

- E est le groupe tétrahydropyranyloxy ;
- m est égal à 2 ;
- Ar' est le groupe dichloro-3,4 phényle ;
- R est le groupe méthyle
  avec un composé de formule III dans laquelle
- Z est le groupe phényle et

2) à l'étape d), on utilise une amine secondaire de formule (VII) dans laquelle Y est le groupe phényl-4 acétylamino pour former le N-méthyl [(phényl-4 acétylamino-4 pipéridinyl)-4 (dichloro-3,4 phényl)-2 butyl] benzamide.

**9.** Procédé selon l'une des revendications 1 à 2, caractérisé en ce que :

1) on utilise une amine de formule II dans laquelle :

- E est le groupe tétrahydropyranyloxy ;
- m est égal à 2 ;
- Ar' est le groupe dichloro-3,4 phényle ;
- R est le groupe méthyle

2) on déprotège cette amine de formule II pour former le composé hydroxylé correspondant de formule (II''') ;
3) on procède à la résolution de l'amine de formule (II''') par réaction avec un acide optiquement pur et
4) on fait réagir le composé optiquement pur résultant avec un composé de formule (III) dans laquelle Z est le groupe phényle ;
5) on procède ensuite aux étapes c), d) et e) telles que définies dans la revendication 1 pour former les énantiomères ci-après : le N-méthyl [(phényl-4 acétylamino-4 pipéridinyl)-4 (dichloro-3,4 phényl)-2 butyl] benzamide (-) et ses sels avec des acides minéraux ou organiques ou le N-méthyl [(phényl-4 acétylamino-4 pipéridinyl)-4 (dichloro-3,4 phényl)-2 butyl] benzamide (+) et ses sels avec des acides minéraux ou organiques.

**10.** Procédé pour la préparation d'une composition pharmaceutique, caractérisé en ce que l'on mélange, en tant que principe actif, un composé obtenu par le procédé selon l'une quelconque des revendications 1 à 9, avec un véhicule pharmaceutiquement acceptable.

## Patentansprüche

**Patentansprüche für folgende Vertragsstaaten : AT, BE, CH, DE, DK, FR, GB, IT, LI, LU, NL, SE**

**1.** Verbindungen der Formel

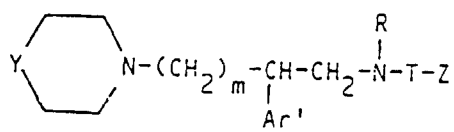

(I),

worin bedeuten:

- Y

  - entweder eine Gruppe Cy-N,
    worin Cy eine unsubstituierte Phenylgruppe, eine Phenylgruppe, die ein- oder mehrfach mit einem der folgenden Substituenten substituiert ist: Wasserstoff, Halogen, Hydroxy, $C_{1-4}$-Alkoxy, $C_{1-4}$-Alkyl und Trifluormethyl, wobei die Substituenten identisch oder voneinander verschieden sind, eine $C_{3-7}$-Cycloalkylgruppe, eine Pyrimidinylgruppe oder eine Pyridylgruppe bedeutet;

  - oder ein Gruppe

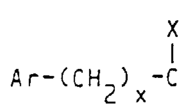

,

worin bedeuten:

Ar eine unsubstituierte Phenylgruppe, eine Phenylgruppe, die ein- oder mehrfach mit einem der folgenden Substituenten substituiert ist: Wasserstoff, Halogen, Hydroxy, $C_{1-4}$-Alkoxy, Trifluormethyl, $C_{1-4}$-Alkyl, wobei die Substituenten identisch oder voneinander verschieden sind, eine Pyridylgruppe oder eine Thienylgruppe;

x Null oder 1;

X Hydroxy, $C_{1-4}$-Alkoxy, Hydroxyalkyl, wobei die Alkylgruppe 1 bis 3 Kohlenstoffatome aufweist, $C_{1-4}$-Acyloxy, Phenacyloxy, Carboxy, $C_{1-4}$-Carbalkoxy, Cyano, Aminoalkylen, wobei die Alkylengruppe 1 bis 3 Kohlenstoffatome aufweist, eine Gruppe -N-$(X_1)_2$, worin die Gruppen $X_1$ unabhängig voneinander Wasserstoff oder $C_{1-4}$-Alkyl bedeuten, eine Gruppe

$$-NH-\underset{\underset{O}{\|}}{C}-Alk \quad ,$$

worin Alk $C_{1-6}$-Alkyl bedeutet, eine Gruppe

$$-Alk_1-NH-\underset{\underset{O}{\|}}{C}-Alk'_1 \quad ,$$

worin $Alk_1$ $C_{1-3}$-Alkylen und $Alk_1'$ $C_{1-3}$-Alkyl bedeutet, $C_{1-4}$-Acyl, -S-$X_2$, worin $X_2$ Wasserstoff oder $C_{1-4}$-Alkyl bedeutet;
oder X bildet mit dem Kohlenstoffatom, an das es gebunden ist, und dem benachbarten Kohlenstoffatom im Heterocyclus eine Doppelbindung;

- m 2 oder 3;
- Ar' eine unsubstituierte Phenylgruppe, eine Phenylgruppe, die ein- oder mehrfach mit einem der folgenden Substituenten substituiert ist: Wasserstoff, Halogen, vorzugsweise Chlor oder Fluor, Trifluormethyl, $C_{1-4}$-Alkoxy, $C_{1-4}$-Alkyl, wobei die Substituenten identisch oder voneinander verschieden sind, eine Thienylgruppe, eine Benzothienylgruppe, eine Naphthylgruppe, eine Indolylgruppe, eine Indolylgruppe, die mit $C_{1-3}$-Alkyl N-substituiert ist;
- R Wasserstoff, $C_{1-6}$-Alkyl;
- T eine Gruppe, die ausgewählt ist unter:

$$-\overset{\overset{O}{\|}}{C}- \quad \text{und} \quad -\overset{\overset{W}{\|}}{C}-NH-$$

wobei W Sauerstoff oder Schwefel bedeutet;

- Z entweder Wasserstoff oder M oder OM, wenn T die Gruppe

$$-\overset{\overset{O}{\|}}{C}-$$

bedeutet, oder M, wenn T die Gruppe

$$-\overset{\overset{W}{\|}}{C}-NH$$

bedeutet;
M bedeutet: eine $C_{1-6}$-Alkylgruppe; eine Phenylalkylgruppe, worin die Alkylgruppe 1 bis 3 Kohlenstoffatome

aufweist, welche gegebenenfalls am aromatischen Ring mit Halogen, Trifluormethyl, $C_{1-4}$-Alkyl, Hydroxy, $C_{1-4}$-Alkoxy substituiert ist; eine Pyridylalkylgruppe, worin die Alkylgruppe 1 bis 3 Kohlenstoffatome aufweist; eine Naphthylalkylgruppe, worin die Alkylgruppe 1 bis 3 Kohlenstoffatome aufweist, welche gegebenenfalls am Naphthylring mit Halogen, Trifluormethyl, $C_{1-4}$-Alkyl, Hydroxy, $C_{1-4}$-Alkoxy substituiert ist; eine Pyridylthioalkylgruppe, worin die Alkylgruppe 1 bis 3 Kohlenstoffatome aufweist; eine Styrylgruppe;
eine Phenylgruppe, die unsubstituiert vorliegt oder ein- oder mehrfach mit einem der folgenden Substituenten substituiert ist: F; Cl; Br; I; CN; OH; $NH_2$; NH-CO-$NH_2$; $NO_2$; $CONH_2$; $CF_3$; $C_{1-10}$-Alkyl; Alkenyl mit 2 bis 10 Kohlenstoffatomen; Alkinyl mit 2 bis 10 Kohlenstoffatomen; Cycloalkyl mit 3 bis 8 Kohlenstoffatomen; Bicycloalkyl mit 4 bis 11 Kohlenstoffatomen; Hydroxyalkyl mit 1 bis 5 Kohlenstoffatomen; Alkoxy mit 1 bis 10 Kohlenstoffatomen; Alkoxyalkyl mit 2 bis 10 Kohlenstoffatomen; Alkoxyalkoxyalkyl mit bis zu 10 Kohlenstoffatomen; Alkoxyalkoxy mit 2 bis 10 Kohlenstoffatomen; Alkenyloxy mit 2 bis 10 Kohlenstoffatomen; Alkenyloxyalkyl mit bis zu 10 Kohlenstoffatomen; Alkinyloxy mit 2 bis 10 Kohlenstoffatomen; Alkinyloxyalkyl mit 3 bis 10 Kohlenstoffatomen; Cycloalkoxy mit 3 bis 8 Kohlenstoffatomen; Alkylthio mit 1 bis 10 Kohlenstoffatomen; Alkylthioalkyl mit 2 bis 10 Kohlenstoffatomen; Acylamino mit 1 bis 7 Kohlenstoffatomen; Acylaminoalkyl mit 2 bis 8 Kohlenstoffatomen; Acyloxy mit 1 bis 6 Kohlenstoffatomen; Alkoxycarbonyl mit 2 bis 5 Kohlenstoffatomen; Cycloalkoxycarbonyl mit 4 bis 8 Kohlenstoffatomen; Alkylaminocarbonylamino mit 2 bis 4 Kohlenstoffatomen; Dialkylaminocarbonylamino mit 3 bis 7 Kohlenstoffatomen; (1-Pyrrolidino)-carbonylamino; (1-Piperidino)carbonylamino; Cycloalkylaminocarbonylamino mit 4 bis 8 Kohlenstoffatomen; Alkylaminocarbonylaminoalkyl mit 3 bis 9 Kohlenstoffatomen; Dialkylaminocarbonylaminoalkyl mit 4 bis 11 Kohlenstoffatomen; (1-Pyrrolidino)-carbonylaminoethyl; (1-Piperidino)carbonylaminoethyl; Cycloalkylaminocarbonylaminoalkyl mit 5 bis 12 Kohlenstoffatomen; Alkoxycarbonylaminoalkyl mit 3 bis 12 Kohlenstoffatomen; Cycloalkoxycarbonylaminoalkyl mit 5 bis 12 Kohlenstoffatomen; Carbamoylalkyl mit 2 bis 5 Kohlenstoffatomen; Alkylaminocarbonylalkyl mit 3 bis 9 Kohlenstoffatomen; Dialkylaminocarbonylalkyl mit 4 bis 11 Kohlenstoffatomen; (1-Pyrrolidino)-carbonylmethyl; (1-Piperidino)-carbonylmethyl; (1-Piperidino)-carbonylethyl; Cycloalkylaminocarbonylalkyl mit 5 bis 12 Kohlenstoffatomen; Alkylaminocarbonylalkoxy mit 3 bis 10 Kohlenstoffatomen; Dialkylaminocarbonylalkoxy mit 4 bis 10 Kohlenstoffatomen; (1-Piperidinyl)carbonylmethoxy; Cycloalkylaminocarbonylalkoxy mit 5 bis 11 Kohlenstoffatomen;
eine 1- oder 2-Naphthylgruppe; eine 1-, 2-, 3-, 4-, 5-, 6- oder 7-Indenylgruppe, wobei eine oder mehrere der Doppelbindungen hydriert sein können, wobei die Naphthylgruppen oder Indenylgruppen unsubstituiert vorliegen können oder gegebenenfalls einen oder mehrere Substituenten aufweisen können, beispielsweise: Alkyl, Phenyl, Cyano, Hydroxyalkyl, Hydroxy, Oxo, Alkylcarbonylamino, Alkoxycarbonyl, Thioalkyl, worin die Alkylgruppen 1 bis 4 Kohlenstoffatome aufweisen; eine Pyridylgruppe; eine Thiadiazolylgruppe; eine Indolylgruppe; eine Indazolylgruppe; eine Imidazolylgruppe; eine Benzimidazolylgruppe; eine Benzotriazolylgruppe; eine Benzofuranylgruppe; eine Benzothienylgruppe; eine Benzothiazolylgruppe; eine Benzisothiazolylgruppe; eine Chinolylgruppe; eine Isochinolylgruppe; eine Benzoxazolylgruppe; eine Benzisoxazolylgruppe; eine Benzoxazinylgruppe; eine Benzodioxinylgruppe; eine Isoxazolylgruppe; eine Benzopyranylgruppe; eine Thiazolylgruppe; eine Thienylgruppe; eine Furylgruppe; eine Pyranylgruppe; eine Chromenylgruppe; eine Isobenzofuranylgruppe; eine Pyrrolylgruppe; eine Pyrazolylgruppe; eine Pyrazinylgruppe; eine Pyrimidinylgruppe; eine Pyridazinylgruppe; eine Indolizinylgruppe; eine Phthalazinylgruppe; eine Chinazolinylgruppe; eine Acridinylgruppe; eine Isothiazolylgruppe; eine Isochromanylgruppe; eine Chromanylgruppe, wobei eine oder mehrere der Doppelbindungen hydriert sein können, wobei die Gruppen unsubstituiert vorliegen können oder gegebenenfalls einen oder mehrere Substituenten aufweisen können, beispielsweise: Alkyl, Phenyl, Cyano, Hydroxyalkyl, Hydroxy, Alkylcarbonylamino, Alkoxycarbonyl, Thioalkyl, worin die Alkylgruppen 1 bis 4 Kohlenstoffatome aufweisen; oder eines ihrer Salze mit anorganischen oder organischen Säuren.

2. Verbindungen nach Anspruch 1, worin Ar' eine 3,4-Dichlorphenylgruppe bedeutet, oder eines ihrer Salze mit anorganischen oder organischen Säuren.

3. Verbindungen nach einem der Ansprüche 1 oder 2, worin X Hydroxy, Acetyloxy oder eine Gruppe

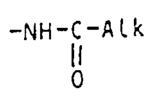

bedeutet, worin Alk eine $C_{1-6}$-Alkylgruppe ist, oder eines ihrer Salze mit anorganischen oder organischen Säuren.

4. Verbindungen nach einem der Ansprüche 1 bis 3, worin R Methyl bedeutet, oder eines ihrer Salze mit anorganischen oder organischen Säuren.

**5.** Verbindungen nach einem der Ansprüche 1 bis 4, worin T die Gruppe -C=O bedeutet, oder eines ihrer Salze mit anorganischen oder organischen Säuren.

**6.** Verbindungen nach einem der Ansprüche 1 bis 5, worin T die Gruppe -C=O bedeutet und Z eine Thienylgruppe ist, oder eines ihrer Salze mit anorganischen oder organischen Säuren.

**7.** Verbindungen nach einem der Ansprüche 1 bis 5, worin T die Gruppe -C=O bedeutet und Z eine Phenylgruppe ist, die gegebenenfalls mit einem Halogen wie Chlor zweifach substituiert ist, oder eines ihrer Salze mit anorganischen oder organischen Säuren.

**8.** Verbindungen nach einem der Ansprüche 1 bis 7, welche N-Methyl-[4-(4-Phenyl-4-acetylamino-piperidinyl)-2-(3,4-dichlor-phenyl)-butyl]benzamid in Form des Racemats sind oder eines seiner Salze mit anorganischen oder organischen Säuren.

**9.** (-) N-Methyl-[4-(4-Phenyl-4-acetylamino-piperidinyl)-2-(3,4-dichlor-phenyl)-butyl]benzamid und seine Salze mit anorganischen oder organischen Säuren.

**10.** (+) N-Methyl-[4-(4-Phenyl-4-acetylamino-piperidinyl)-2-(3,4-dichlor-phenyl)-butyl]benzamid und seine Salze mit anorganischen oder organischen Säuren.

**11.** Verfahren zur Herstellung der Verbindungen nach einem der Ansprüche 1 bis 10, dadurch gekennzeichnet, daß

(a) ein freies Amin der Formel

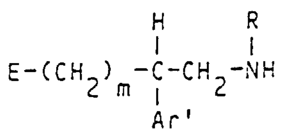

(II),

worin m, Ar' und R die in Anspruch 1 angegebenen Bedeutungen aufweisen und E eine O-Schutzgruppe ist, wie beispielsweise 2-Tetrahydropyranyl-oxy oder eine Gruppe

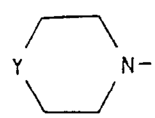

,

worin Y die in Anspruch 1 angegebene Bedeutung aufweist, mit der Maßgabe, daß, wenn Y die Gruppe

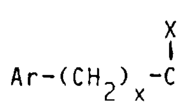

bedeutet, worin X eine Hydroxygruppe ist, diese Hydroxygruppe geschützt ist,
oder ein Amin der Formel

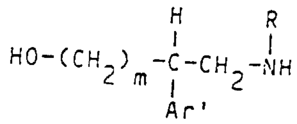

(II''')

worin m, Ar' und R die oben angegebenen Bedeutungen aufweisen und das Amin der Formel (II''') gegebenenfalls in optisch reiner Form vorliegt,

- entweder mit einem funktionellen Säurederivat der Formel:

  HO - CO - Z (III),

  worin Z die in Anspruch 1 angegebene Bedeutung aufweist, wenn eine Verbindung der Formel (I) hergestellt werden soll, worin T -CO- bedeutet,

- oder mit einem Iso(thio)cyanat der Formel

  W = C = N - Z (III'),

  worin W und Z die oben angegebenen Bedeutungen aufweisen, wenn eine Verbindung der Formel (I) hergestellt werden soll, worin T -C(W)-NH- bedeutet, wodurch die Verbindungen der Formel

(IV);

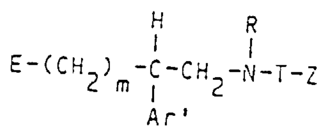

  gebildet werden, umgesetzt wird;

(b) wenn E Tetrahydropyranyloxy bedeutet, anschließend die Tetrahydropyranylgruppe durch Einwirkung einer Säure entfernt wird, wobei die Entfernung der Schutzgruppe direkt an der Verbindung der Formel (II) durchgeführt werden kann, wodurch die Verbindung (II''') erhalten wird, die dann mit einer der Verbindungen (III) oder (III') umgesetzt wird;

(c) das so hergestellte N-substituierte Alkanolamin der Formel:

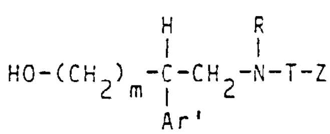

(V)

mit Methansulfonylchlorid umgesetzt wird,

(d) das so hergestellte Mesylat der Formel:

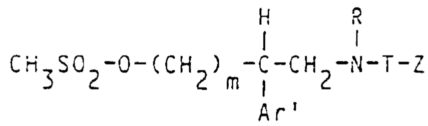

(VI)

mit einem sekundären Amin der Formel

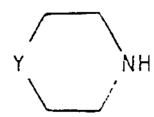

(VII)

umgesetzt wird, worin Y die in Anspruch 1 angegebene Bedeutung aufweist; und

(e) gegebenenfalls nach Entfernen der Schutzgruppe von der durch X dargestellten Hydroxygruppe, das so hergestellt Produkt gegebenenfalls in eines seiner Salze übergeführt wird.

**12.** Pharmazeutische Zusammensetzung, die als Hauptwirkstoff eine Verbindung der Formel (I) nach einem der Ansprüche 1 bis 10 enthält.

**13.** Pharmazeutische Zusammensetzung nach Anspruch 12 in Form einer Dosierungseinheit, worin der Hauptwirkstoff mit mindestens einem pharmazeutischen Hilfsstoff vermischt ist.

**Patentansprüche für folgenden Vertragsstaat : GR**

**1.** Verbindungen der Formel

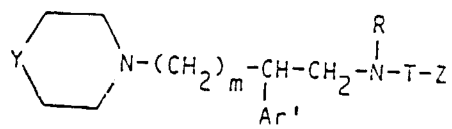

(I),

worin bedeuten:

- Y

  - entweder eine Gruppe Cy-N,
    worin Cy eine unsubstituierte Phenylgruppe oder eine Phenylgruppe, die ein- oder mehrfach mit einem der folgenden Substituenten substituiert ist: Wasserstoff, Halogen, Hydroxy, $C_{1-4}$-Alkoxy, $C_{1-4}$-Alkyl und Trifluormethyl, wobei die Substituenten identisch oder voneinander verschieden sind, eine $C_{3-7}$-Cycloalkylgruppe, eine Pyrimidinylgruppe oder eine Pyridylgruppe bedeutet;

  - oder ein Gruppe

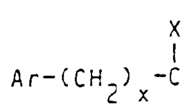

,

worin bedeuten:

Ar eine unsubstituierte Phenylgruppe, eine Phenylgruppe, die ein- oder mehrfach mit einem der folgenden Substituenten substituiert ist: Wasserstoff, Halogen, Hydroxy, $C_{1-4}$-Alkoxy, Trifluormethyl, $C_{1-4}$-Alkyl, wobei die Substituenten identisch oder voneinander verschieden sind, eine Pyridylgruppe oder eine Thienylgruppe;

x Null oder 1;

X Hydroxy, $C_{1-4}$-Alkoxy, Hydroxyalkyl, wobei die Alkylgruppe 1 bis 3 Kohlenstoffatome aufweist, $C_{1-4}$-Acyloxy, Phenacyloxy, Carboxy, $C_{1-4}$-Carbalkoxy, Cyano, Aminoalkylen, wobei die Alkylengruppe 1 bis 3 Kohlenstoffatome aufweist, eine Gruppe $-N-(X_1)_2$, worin die Gruppen $X_1$ unabhängig voneinander Wasserstoff oder $C_{1-4}$-Alkyl bedeuten, eine Gruppe

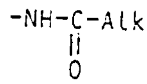 ,

worin Alk $C_{1-6}$-Alkyl bedeutet, eine Gruppe

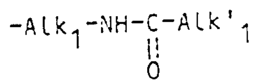 ,

worin $Alk_1$ $C_{1-3}$-Alkylen und $Alk_1'$ $C_{1-3}$-Alkyl bedeutet, $C_{1-4}$-Acyl, $-S-X_2$, worin $X_2$ Wasserstoff oder $C_{1-4}$-Alkyl bedeutet;
oder X bildet mit dem Kohlenstoffatom, an das es gebunden ist, und dem benachbarten Kohlenstoffatom im Heterocyclus eine Doppelbindung;

- m 2 oder 3;

- Ar' eine unsubstituierte Phenylgruppe, eine Phenylgruppe, die ein- oder mehrfach mit einem der folgenden Substituenten substituiert ist: Wasserstoff, Halogen, vorzugsweise Chlor oder Fluor, Trifluormethyl, $C_{1-4}$-Alkoxy, $C_{1-4}$-Alkyl, wobei die Substituenten identisch oder voneinander verschieden sind, eine Thienylgruppe, eine Benzothienylgruppe, eine Naphthylgruppe, eine Indolylgruppe, eine Indolylgruppe, die mit $C_{1-3}$-Alkyl N-substituiert ist;

- R Wasserstoff, $C_{1-6}$-Alkyl;

- T eine Gruppe, die ausgewählt ist unter:

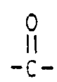 und 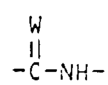

wobei W Sauerstoff oder Schwefel bedeutet;

- Z entweder Wasserstoff oder M oder OM, wenn T die Gruppe

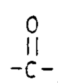

bedeutet, oder M, wenn T die Gruppe

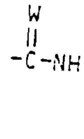

bedeutet;

M bedeutet: eine $C_{1-6}$-Alkylgruppe; eine Phenylalkylgruppe, worin die Alkylgruppe 1 bis 3 Kohlenstoffatome aufweist, welche gegebenenfalls am aromatischen Ring mit Halogen, Trifluormethyl, $C_{1-4}$-Alkyl, Hydroxy, $C_{1-4}$-Alkoxy substituiert ist; eine Pyridylalkylgruppe, worin die Alkylgruppe 1 bis 3 Kohlenstoffatome aufweist; eine Naphthylalkylgruppe, worin die Alkylgruppe 1 bis 3 Kohlenstoffatome aufweist, welche gegebenenfalls am Naphthylring mit Halogen, Trifluormethyl, $C_{1-4}$-Alkyl, Hydroxy, $C_{1-4}$-Alkoxy substituiert ist; eine Pyridylthioalkylgruppe, worin die Alkylgruppe 1 bis 3 Kohlenstoffatome aufweist; eine Styrylgruppe;

eine Phenylgruppe, die unsubstituiert vorliegt oder ein- oder mehrfach mit einem der folgenden Substituenten substituiert ist: F; Cl; Br; I; CN; OH; $NH_2$; $NH-CO-NH_2$; $NO_2$; $CONH_2$; $CF_3$; $C_{1-10}$-Alkyl; Alkenyl mit 2 bis 10 Kohlenstoffatomen; Alkinyl mit 2 bis 10 Kohlenstoffatomen; Cycloalkyl mit 3 bis 8 Kohlenstoffatomen; Bicycloalkyl mit 4 bis 11 Kohlenstoffatomen; Hydroxyalkyl mit 1 bis 5 Kohlenstoffatomen; Alkoxy mit 1 bis 10 Kohlenstoffatomen; Alkoxyalkyl mit 2 bis 10 Kohlenstoffatomen; Alkoxyalkoxyalkyl mit bis zu 10 Kohlenstoffatomen; Alkoxyalkoxy mit 2 bis 10 Kohlenstoffatomen; Alkenyloxy mit 2 bis 10 Kohlenstoffatomen; Alkenyloxyalkyl mit bis zu 10 Kohlenstoffatomen; Alkinyloxy mit 2 bis 10 Kohlenstoffatomen; Alkinyloxyalkyl mit 3 bis 10 Kohlenstoffatomen; Cycloalkoxy mit 3 bis 8 Kohlenstoffatomen; Alkylthio mit 1 bis 10 Kohlenstoffatomen; Alkylthioalkyl mit 2 bis 10 Kohlenstoffatomen; Acylamino mit 1 bis 7 Kohlenstoffatomen; Acylaminoalkyl mit 2 bis 8 Kohlenstoffatomen; Acyloxy mit 1 bis 6 Kohlenstoffatomen; Alkoxycarbonyl mit 2 bis 5 Kohlenstoffatomen; Cycloalkoxycarbonyl mit 4 bis 8 Kohlenstoffatomen; Alkylaminocarbonylamino mit 2 bis 4 Kohlenstoffatomen; Dialkylaminocarbonylamino mit 3 bis 7 Kohlenstoffatomen; (1-Pyrrolidino)-carbonylamino; (1-Piperidino)-carbonylamino; Cycloalkylaminocarbonylamino mit 4 bis 8 Kohlenstoffatomen; Alkylaminocarbonylaminoalkyl mit 3 bis 9 Kohlenstoffatomen; Dialkylaminocarbonylaminoalkyl mit 4 bis 11 Kohlenstoffatomen; (1-Pyrrolidino)-carbonylaminoethyl; (1-Piperidino)-carbonylaminoethyl; Cycloalkylaminocarbonylaminoalkyl mit 5 bis 12 Kohlenstoffatomen; Alkoxycarbonylaminoalkyl mit 3 bis 12 Kohlenstoffatomen; Cycloalkoxycarbonylaminoalkyl mit 5 bis 12 Kohlenstoffatomen; Carbamoylalkyl mit 2 bis 5 Kohlenstoffatomen; Alkylaminocarbonylalkyl mit 3 bis 9 Kohlenstoffatomen; Dialkylaminocarbonylalkyl mit 4 bis 11 Kohlenstoffatomen; (1-Pyrrolidino)-carbonylmethyl; (1-Piperidino)-carbonylmethyl; (1-Piperidino)-carbonylethyl; Cycloalkylaminocarbonylalkyl mit 5 bis 12 Kohlenstoffatomen; Alkylaminocarbonylalkoxy mit 3 bis 10 Kohlenstoffatomen; Dialkylaminocarbonylalkoxy mit 4 bis 10 Kohlenstoffatomen; (1-Piperidinyl)carbonylmethoxy; Cycloalkylaminocarbonylalkoxy mit 5 bis 11 Kohlenstoffatomen;

eine 1- oder 2-Naphthylgruppe; eine 1-, 2-, 3-, 4-, 5-, 6- oder 7-Indenylgruppe, wobei eine oder mehrere der Doppelbindungen hydriert sein können, wobei die Naphthylgruppen oder Indenylgruppen unsubstituiert vorliegen können oder gegebenenfalls einen oder mehrere Substituenten aufweisen können, beispielsweise: Alkyl, Phenyl, Cyano, Hydroxyalkyl, Hydroxy, Oxo, Alkylcarbonylamino, Alkoxycarbonyl, Thioalkyl, worin die Alkylgruppen 1 bis 4 Kohlenstoffatome aufweisen; eine Pyridylgruppe; eine Thiadiazolylgruppe; eine Indolylgruppe; eine Indazolylgruppe; eine Imidazolylgruppe; eine Benzimidazolylgruppe; eine Benzotriazolylgruppe; eine Benzofuranylgruppe; eine Benzothienylgruppe; eine Benzothiazolylgruppe; eine Benzisothiazolylgruppe; eine Chinolylgruppe; eine Isochinolylgruppe; eine Benzoxazolylgruppe; eine Benzisoxazolylgruppe; eine Benzoxazinylgruppe; eine Benzodioxinylgruppe; eine Isoxazolylgruppe; eine Benzopyranylgruppe; eine Thiazolylgruppe; eine Thienylgruppe; eine Furylgruppe; eine Pyranylgruppe; eine Chromenylgruppe; eine Isobenzofuranylgruppe; eine Pyrrolylgruppe; eine Pyrazolylgruppe; eine Pyrazinylgruppe; eine Pyrimidinylgruppe; eine Pyridazinylgruppe; eine Indolizinylgruppe; eine Phthalazinylgruppe; eine Chinazolinylgruppe; eine Acridinylgruppe; eine Isothiazolylgruppe; eine Isochromanylgruppe; eine Chromanylgruppe, wobei eine oder mehrere der Doppelbindungen hydriert sein können, wobei die Gruppen unsubstituiert vorliegen können oder gegebenenfalls einen oder mehrere Substituenten aufweisen können, beispielsweise: Alkyl, Phenyl, Cyano, Hydroxyalkyl, Hydroxy, Alkylcarbonylamino, Alkoxycarbonyl, Thioalkyl, worin die Alkylgruppen 1 bis 4 Kohlenstoffatome aufweisen; oder eines ihrer Salze mit anorganischen oder organischen Säuren.

**2.** Verbindungen nach Anspruch 1, worin Ar' eine 3,4-Dichlorphenylgruppe bedeutet, oder eines ihrer Salze mit anorganischen oder organischen Säuren.

**3.** Verbindungen nach einem der Ansprüche 1 oder 2, worin X Hydroxy, Acetyloxy oder eine Gruppe

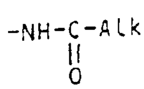

bedeutet, worin Alk eine $C_{1-6}$-Alkylgruppe ist, oder eines ihrer Salze mit anorganischen oder organischen Säuren.

4. Verbindungen nach einem der Ansprüche 1 bis 3, worin R Methyl bedeutet, oder eines ihrer Salze mit anorganischen oder organischen Säuren.

5. Verbindungen nach einem der Ansprüche 1 bis 4, worin T die Gruppe -C=O bedeutet, oder eines ihrer Salze mit anorganischen oder organischen Säuren.

6. Verbindungen nach einem der Ansprüche 1 bis 5, worin T die Gruppe -C=O bedeutet und Z eine Thienylgruppe ist, oder eines ihrer Salze mit anorganischen oder organischen Säuren.

7. Verbindungen nach einem der Ansprüche 1 bis 5, worin T die Gruppe -C=O bedeutet und Z eine Phenylgruppe ist, die gegebenenfalls mit einem Halogen wie Chlor zweifach substituiert ist, oder eines ihrer Salze mit anorganischen oder organischen Säuren.

8. Verbindungen nach einem der Ansprüche 1 bis 7, welche N-Methyl-[4-(4-Phenyl-4-acetylamino-piperidinyl)-2-(3,4-dichlor-phenyl)-butyl]benzamid in Form des Racemats sind oder eines seiner Salze mit anorganischen oder organischen Säuren.

9. (-) N-Methyl-[4-(4-Phenyl-4-acetylamino-piperidinyl)-2-(3,4-dichlor-phenyl)-butyl]benzamid und seine Salze mit anorganischen oder organischen Säuren.

10. (+) N-Methyl-[4-(4-Phenyl-4-acetylamino-piperidinyl)-2-(3,4-dichlor-phenyl)-butyl]benzamid und seine Salze mit anorganischen oder organischen Säuren.

11. Verfahren zur Herstellung der Verbindungen nach einem der Ansprüche 1 bis 10, dadurch gekennzeichnet, daß

(a) ein freies Amin der Formel

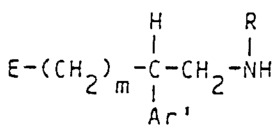

(II),

worin m, Ar' und R die in Anspruch 1 angegebenen Bedeutungen aufweisen und E eine O-Schutzgruppe ist, wie beispielsweise 2-Tetrahydropyranyl-oxy oder eine Gruppe

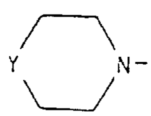

,

worin Y die in Anspruch 1 angegebene Bedeutung aufweist, mit der Maßgabe, daß, wenn Y die Gruppe

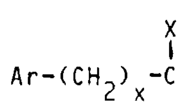

bedeutet, worin X eine Hydroxygruppe ist, diese Hydroxygruppe geschützt ist,
oder ein Amin der Formel

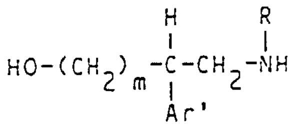

(II''')

worin m, Ar' und R die oben angegebenen Bedeutungen aufweisen und das Amin der Formel (II''') gegebenenfalls in optisch reiner Form vorliegt,

- entweder mit einem funktionellen Säurederivat der Formel:

$$HO - CO - Z \qquad (III)$$

  worin Z die in Anspruch 1 angegebene Bedeutung aufweist, wenn eine Verbindung der Formel (I) hergestellt werden soll, worin T -CO- bedeutet,

- oder mit einem Iso(thio)cyanat der Formel

$$W = C = N - Z \qquad (III'),$$

  worin W und Z die oben angegebenen Bedeutungen aufweisen, wenn eine Verbindung der Formel der Formel (I) hergestellt werden soll, worin T -C(W)-NH- bedeutet, wodurch die Verbindungen der Formel

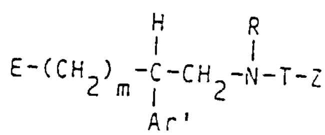

(IV);

  gebildet werden, umgesetzt wird;

(b) wenn E Tetrahydropyranyloxy bedeutet, anschließend die Tetrahydropyranylgruppe durch Einwirkung einer Säure entfernt wird, wobei die Entfernung der Schutzgruppe direkt an der Verbindung der Formel (II) durchgeführt werden kann, wodurch die Verbindung (II''') erhalten wird, die dann mit einer der Verbindungen (III) oder (III') umgesetzt wird;

(c) das so hergestellte N-substituierte Alkanolamin der Formel:

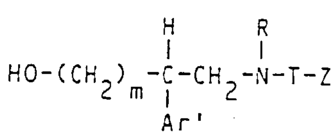

(V)

mit Methansulfonylchlorid umgesetzt wird,

(d) das so hergestellte Mesylat der Formel:

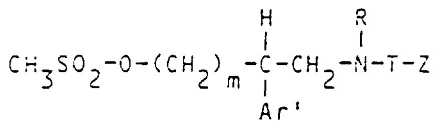

(VI)

mit einem sekundären Amin der Formel

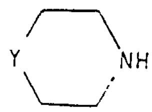

(VII)

umgesetzt wird, worin Y die in Anspruch 1 angegebene Bedeutung aufweist; und

(e) gegebenenfalls nach Entfernen der Schutzgruppe von der durch X dargestellten Hydroxygruppe, das so hergestellt Produkt gegebenenfalls in eines seiner Salze übergeführt wird.

**12.** Verfahren zur Herstellung einer pharmazeutischen Zusammensetzung, dadurch gekennzeichnet, daß eine Verbindung nach einem der Ansprüche 1 bis 10 als Hauptwirkstoff mit einem pharmazeutisch akzeptablen Träger vermischt wird.

**Patentansprüche für folgenden Vertragsstaat : ES**

**1.** Verfahren zur Herstellung der Verbindungen der Formel

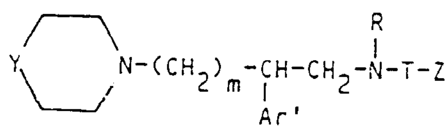

(I),

worin bedeuten:

- Y
  - entweder eine Gruppe Cy-N,
    worin Cy eine unsubstituierte Phenylgruppe, eine Phenylgruppe, die ein- oder mehrfach mit einem der folgenden Substituenten substituiert ist: Wasserstoff, Halogen, Hydroxy, $C_{1-4}$-Alkoxy, $C_{1-4}$-Alkyl und Trifluormethyl, wobei die Substituenten identisch oder voneinander verschieden sind, eine $C_{3-7}$-Cycloalkylgruppe, eine Pyrimidinylgruppe oder eine Pyridylgruppe bedeutet;
  - oder ein Gruppe

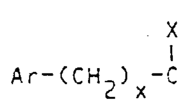

,

worin bedeuten:

Ar eine unsubstituierte Phenylgruppe, eine Phenylgruppe, die ein- oder mehrfach mit einem der folgenden Substituenten substituiert ist: Wasserstoff, Halogen, Hydroxy, $C_{1-4}$-Alkoxy, Trifluormethyl, $C_{1-4}$-Alkyl, wobei die Substituenten identisch oder voneinander verschieden sind, eine Pyridylgruppe oder eine Thienylgruppe;

x Null oder 1;

X Hydroxy, $C_{1-4}$-Alkoxy, Hydroxyalkyl, wobei die Alkylgruppe 1 bis 3 Kohlenstoffatome aufweist, $C_{1-4}$-Acyloxy, Phenacyloxy, Carboxy, $C_{1-4}$-Carbalkoxy, Cyano, Aminoalkylen, wobei die Alkylengruppe 1 bis 3 Kohlenstoffatome aufweist, eine Gruppe $-N-(X_1)_2$, worin die Gruppen $X_1$ unabhängig voneinander Wasserstoff und $C_{1-4}$-Alkyl bedeuten, eine Gruppe

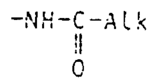 ,

worin Alk $C_{1-6}$-Alkyl bedeutet, eine Gruppe

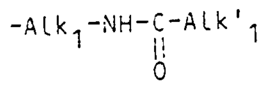 ,

worin $Alk_1$ $C_{1-3}$-Alkylen und $Alk_1'$ $C_{1-3}$Alkyl bedeutet, $C_{1-4}$-Acyl, $-S-X_2$, worin $X_2$ Wasserstoff oder $C_{1-4}$-Alkyl bedeutet;
oder X bildet mit dem Kohlenstoffatom, an das es gebunden ist, und dem benachbarten Kohlenstoffatom im Heterocyclus eine Doppelbindung;

- m 2 oder 3;
- Ar' eine unsubstituierte Phenylgruppe, eine Phenylgruppe, die ein- oder mehrfach mit einem der folgenden Substituenten substituiert ist: Wasserstoff, Halogen, vorzugsweise Chlor oder Fluor, Trifluormethyl, $C_{1-4}$-Alkoxy, $C_{1-4}$-Alkyl, wobei die Substituenten identisch oder voneinander verschieden sind, eine Thienylgruppe, eine Benzothienylgruppe, eine Naphthylgruppe, eine Indolylgruppe, eine Indolylgruppe, die mit $C_{1-3}$-Alkyl N-substituiert ist;
- R Wasserstoff, $C_{1-6}$-Alkyl;
- T eine Gruppe, die ausgewählt ist unter:

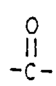 und 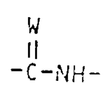

wobei W Sauerstoff oder Schwefel bedeutet;

- Z entweder Wasserstoff oder M oder OM, wenn T die Gruppe

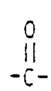

bedeutet, oder M, wenn T die Gruppe

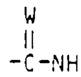

bedeutet;

M bedeutet: eine $C_{1-6}$-Alkylgruppe; eine Phenylalkylgruppe, worin die Alkylgruppe 1 bis 3 Kohlenstoffatome aufweist, welche gegebenenfalls am aromatischen Ring mit Halogen, Trifluormethyl, $C_{1-4}$-Alkyl, Hydroxy, $C_{1-4}$-Alkoxy substituiert ist; eine Pyridylalkylgruppe, worin die Alkylgruppe 1 bis 3 Kohlenstoffatome aufweist; eine Naphthylalkylgruppe, worin die Alkylgruppe 1 bis 3 Kohlenstoffatome aufweist, welche gegebenenfalls am Naphthylring mit Halogen, Trifluormethyl, $C_{1-4}$-Alkyl, Hydroxy, $C_{1-4}$-Alkoxy substituiert ist; eine Pyridylthioalkylgruppe, worin die Alkylgruppe 1 bis 3 Kohlenstoffatome aufweist; eine Styrylgruppe;

eine Phenylgruppe, die unsubstituiert vorliegt oder ein- oder mehrfach mit einem der folgenden Substituenten substituiert ist: F; Cl; Br; I; CN; OH; $NH_2$; NH-CO-$NH_2$; $NO_2$; $CONH_2$; $CF_3$; $C_{1-10}$-Alkyl; Alkenyl mit 2 bis 10 Kohlenstoffatomen; Alkinyl mit 2 bis 10 Kohlenstoffatomen; Cycloalkyl mit 3 bis 8 Kohlenstoffatomen; Bicycloalkyl mit 4 bis 11 Kohlenstoffatomen; Hydroxyalkyl mit 1 bis 5 Kohlenstoffatomen; Alkoxy mit 1 bis 10 Kohlenstoffatomen; Alkoxyalkyl mit 2 bis 10 Kohlenstoffatomen; Alkoxyalkoxyalkyl mit bis zu 10 Kohlenstoffatomen; Alkoxyalkoxy mit 2 bis 10 Kohlenstoffatomen; Alkenyloxy mit 2 bis 10 Kohlenstoffatomen; Alkenyloxyalkyl mit bis zu 10 Kohlenstoffatomen; Alkinyloxy mit 2 bis 10 Kohlenstoffatomen; Alkinyloxyalkyl mit 3 bis 10 Kohlenstoffatomen; Cycloalkoxy mit 3 bis 8 Kohlenstoffatomen; Alkylthio mit 1 bis 10 Kohlenstoffatomen; Alkylthioalkyl mit 2 bis 10 Kohlenstoffatomen; Acylamino mit 1 bis 7 Kohlenstoffatomen; Acylaminoalkyl mit 2 bis 8 Kohlenstoffatomen; Acyloxy mit 1 bis 6 Kohlenstoffatomen; Alkoxycarbonyl mit 2 bis 5 Kohlenstoffatomen; Cycloalkoxycarbonyl mit 4 bis 8 Kohlenstoffatomen; Alkylaminocarbonylamino mit 2 bis 4 Kohlenstoffatomen; Dialkylaminocarbonylamino mit 3 bis 7 Kohlenstoffatomen; (1-Pyrrolidino)-carbonylamino; (1-Piperidino)-carbonylamino; Cycloalkylaminocarbonylamino mit 4 bis 8 Kohlenstoffatomen; Alkylaminocarbonylaminoalkyl mit 3 bis 9 Kohlenstoffatomen; Dialkylaminocarbonylaminoalkyl mit 4 bis 11 Kohlenstoffatomen; (1-Pyrrolidino)-carbonylaminoethyl; (1-Piperidino)-carbonylaminoethyl; Cycloalkylaminocarbonylaminoalkyl mit 5 bis 12 Kohlenstoffatomen; Alkoxycarbonylaminoalkyl mit 3 bis 12 Kohlenstoffatomen; Cycloalkoxycarbonylaminoalkyl mit 5 bis 12 Kohlenstoffatomen; Carbamoylalkyl mit 2 bis 5 Kohlenstoffatomen; Alkylaminocarbonylalkyl mit 3 bis 9 Kohlenstoffatomen; Dialkylaminocarbonylalkyl mit 4 bis 11 Kohlenstoffatomen; (1-Pyrrolidino)-carbonylmethyl; (1-Piperidino)-carbonylmethyl; (1-Piperidino)-carbonylethyl; Cycloalkylaminocarbonylalkyl mit 5 bis 12 Kohlenstoffatomen; Alkylaminocarbonylalkoxy mit 3 bis 10 Kohlenstoffatomen; Dialkylaminocarbonylalkoxy mit 4 bis 10 Kohlenstoffatomen; (1-Piperidinyl)carbonylmethoxy; Cycloalkylaminocarbonylalkoxy mit 5 bis 11 Kohlenstoffatomen;

eine 1- oder 2-Naphthylgruppe; eine 1-, 2-, 3-, 4-, 5-, 6- oder 7-Indenylgruppe, wobei eine oder mehrere der Doppelbindungen hydriert sein können, wobei die Naphthylgruppen oder Indenylgruppen unsubstituiert vorliegen können oder gegebenenfalls einen oder mehrere Substituenten aufweisen können, beispielsweise: Alkyl, Phenyl, Cyano, Hydroxyalkyl, Hydroxy, Oxo, Alkylcarbonylamino, Alkoxycarbonyl, Thioalkyl, worin die Alkylgruppen 1 bis 4 Kohlenstoffatome aufweisen; eine Pyridylgruppe; eine Thiadiazolylgruppe; eine Indolylgruppe; eine Indazolylgruppe; eine Imidazolylgruppe; eine Benzimidazolylgruppe; eine Benzotriazolylgruppe; eine Benzofuranylgruppe; eine Benzothienylgruppe; eine Benzothiazolylgruppe; eine Benzisothiazolylgruppe; eine Chinolylgruppe; eine Isochinolylgruppe; eine Benzoxazolylgruppe; eine Benzisoxazolylgruppe; eine Benzoxazinylgruppe; eine Benzodioxinylgruppe; eine Isoxazolylgruppe; eine Benzopyranylgruppe; eine Thiazolylgruppe; eine Thienylgruppe; eine Furylgruppe; eine Pyranylgruppe; eine Chromenylgruppe; eine Isobenzofuranylgruppe; eine Pyrrolylgruppe; eine Pyrazolylgruppe; eine Pyrazinylgruppe; eine Pyrimidinylgruppe; eine Pyridazinylgruppe; eine Indolizinylgruppe; eine Phthalazinylgruppe; eine Chinazolinylgruppe; eine Acridinylgruppe; eine Isothiazolylgruppe; eine Isochromanylgruppe; eine Chromanylgruppe, wobei eine oder mehrere der Doppelbindungen hydriert sein können, wobei die Gruppen unsubstituiert vorliegen können oder gegebenenfalls einen oder mehrere Substituenten aufweisen können, beispielsweise: Alkyl, Phenyl, Cyano, Hydroxyalkyl, Hydroxy, Alkylcarbonylamino, Alkoxycarbonyl, Thioalkyl, worin die Alkylgruppen 1 bis 4 Kohlenstoffatome aufweisen; oder eines ihrer Salze mit anorganischen oder organischen Säuren,

dadurch gekennzeichnet, daß

(a) ein freies Amin der Formel

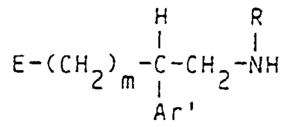 (II),

worin m, Ar' und R die in Anspruch 1 angegebenen Bedeutungen aufweisen und E eine O-Schutzgruppe ist, wie beispielsweise 2-Tetrahydropyranyl-oxy oder eine Gruppe

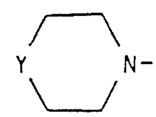

worin Y die in Anspruch 1 angegebene Bedeutung aufweist, mit der Maßgabe, daß, wenn Y die Gruppe

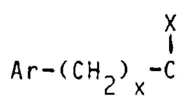

bedeutet, worin X eine Hydroxygruppe ist, diese Hydroxygruppe geschützt ist,
oder ein Amin der Formel

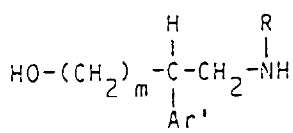 (II''')

worin m, Ar' und R die oben angegebenen Bedeutungen aufweisen und das Amin der Formel (II''') gegebenenfalls in optisch reiner Form vorliegt,

- entweder mit einem funktionellen Säurederivat der Formel:

  HO - CO - Z (III),

  worin Z die in Anspruch 1 angegebene Bedeutung aufweist, wenn eine Verbindung der Formel (I) hergestellt werden soll, worin T -CO- bedeutet,

- oder mit einem Iso(thio)cyanat der Formel

  W = C = N - Z (III'),

  worin W und Z die oben angegebenen Bedeutungen aufweisen, wenn eine Verbindung der Formel der Formel (I) hergestellt werden soll, worin T -C(W)-NH- bedeutet, wodurch die Verbindungen der Formel

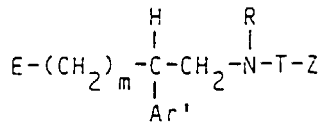

(IV);

gebildet werden, umgesetzt wird;

(b) wenn E Tetrahydropyranyloxy bedeutet, anschließend die Tetrahydropyranylgruppe durch Einwirkung einer Säure entfernt wird, wobei die Entfernung der Schutzgruppe direkt an der Verbindung der Formel (II) durchgeführt werden kann, wodurch die Verbindung (II''') erhalten wird, die dann mit einer der Verbindungen (III) oder (III') umgesetzt wird;

(c) das so hergestellte N-substituierte Alkanolamin der Formel:

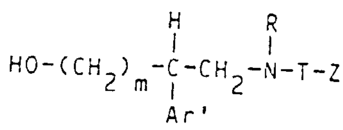

(V)

mit Methansulfonylchlorid umgesetzt wird,

(d) das so hergestellte Mesylat der Formel:

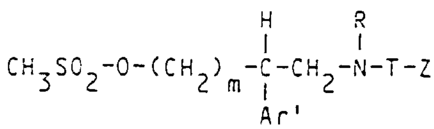

(VI)

mit einem sekundären Amin der Formel

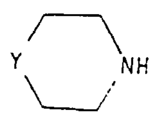

(VII)

umgesetzt wird, worin Y die in Anspruch 1 angegebene Bedeutung aufweist; und

(e) gegebenenfalls nach Entfernen der Schutzgruppe von der durch X dargestellten Hydroxygruppe, das so hergestellt Produkt gegebenenfalls in eines seiner Salze übergeführt wird.

**2.** Verfahren nach Anspruch 1, dadurch gekennzeichnet, daß ein freies Amin der Formel (II) verwendet wird, worin E, m und R die in Anspruch 1 angegebenen Bedeutungen aufweisen und Ar' die 3,4-Dichlor-phenylgruppe bedeutet.

**3.** Verfahren nach einem der Ansprüche 1 oder 2, dadurch gekennzeichnet, daß ein freies Amin der Formel (II) verwendet wird, worin bedeuten:

- Ar', m und R die in Anspruch 1 oder 2 angegebenen Bedeutungen, und
- E die Gruppe

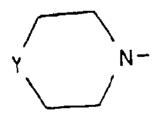

wobei Y eine Gruppe

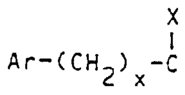

ist, worin bedeuten: Ar und x die in Anspruch 1 angegebenen Bedeutungen und X Hydroxy, Acetyloxy oder eine Gruppe

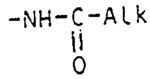 ,

worin Alk eine $C_{1-6}$-Alkylgruppe ist.

**4.** Verfahren nach einem der Ansprüche 1 bis 3, dadurch gekennzeichnet, daß ein freies Amin der Formel (II) verwendet wird, worin bedeuten:

- E, m und Ar' die in Anspruch 1 bis 3 angegebenen Bedeutungen, und
- R Methyl.

**5.** Verfahren nach einem der Ansprüche 1 bis 4, dadurch gekennzeichnet, daß ein funktionelles Derivat einer Säure der Formel (III) verwendet wird, worin Z die in Anspruch 1 angegebenen Bedeutungen aufweist.

**6.** Verfahren nach Anspruch 5, dadurch gekennzeichnet, daß Z eine Thienylgruppe ist.

**7.** Verfahren nach Anspruch 5, dadurch gekennzeichnet, daß Z eine Phenylgruppe bedeutet, die gegebenenfalls mit einem Halogen wie Chlor zweifach substituiert ist.

**8.** Verfahren nach Anspruch 1, dadurch gekennzeichnet, daß (1) in Schritt a) ein Amin der Formel (II), worin bedeuten:

- E Tetrahydropyranyloxy,
- m 2,
- Ar' 3,4-Dichlorphenyl, und
- R Methyl,

mit einer Verbindung der Formel (III) umgesetzt wird, worin Z Phenyl bedeutet, und
(2) in Schritt d) ein sekundäres Amin der Formel (VII) verwendet wird, worin Y die Gruppe 4-Phenyl-acetylamino bedeutet, wodurch N-Methyl-[4-(4-Phenyl-4-acetylaminopiperidinyl)-2-(3,4-dichlor-phenyl)-butyl]benzamid hergestellt wird.

**9.** Verfahren nach einem der Ansprüche 1 oder 2, dadurch gekennzeichnet, daß

(1) ein Amin der Formel (II) verwendet wird, worin bedeuten:

- E Tetrahydropyranyloxy,
- m 2,
- Ar' 3,4-Dichlorphenyl, und
- R Methyl,

(2) die Schutzgruppe dieses Amins der Formel (II) zur Bildung der entsprechenden Hydroxyverbindung der Formel (II''') entfernt wird,
(3) das Amin der Formel (II''') durch Umsetzung mit einer optisch reinen Säure aufgetrennt wird,
(4) die resultierende optisch reine Verbindung mit einer Verbindung der Formel (III) umgesetzt wird, worin Z Phenyl bedeutet, und
(5) dann die in Anspruch 1 definierten Schritte c), d) und e) durchgeführt werden, wodurch die folgenden Enantiomere gebildet werden: (-) N-Methyl-[4-(4-Phenyl-4-acetylamino-piperidinyl)-2- (3,4-dichlor-phenyl)-butyl]benzamid und seine Salze mit anorganischen oder organischen Säuren oder (+) N-Methyl-[4-(4-Phenyl-4-acetylamino-piperidinyl)-2-(3,4-dichlor-phenyl)-butyl]benzamid und seine Salze mit anorganischen oder organischen Säuren.

**10.** Verfahren zur Herstellung von pharmazeutischen Zusammensetzungen, dadurch gekennzeichnet, daß eine nach dem Verfahren nach einem der Ansprüche 1 bis 9 hergestellte Verbindung mit einem pharmazeutischen akzeptablen Träger vermischt wird.

**Claims**

**Claims for the following Contracting States : AT, BE, CH, DE, DK, FR, GB, IT, LI, LU, NL, SE**

**1.** A compound of formula:

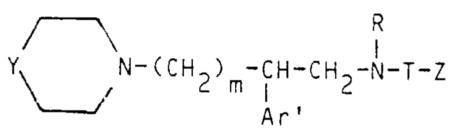

(I)

in which:

- Y represents - either a group Cy-N in which

  . Cy represents a phenyl, unsubstituted or substituted one or more times with one of the substituents selected from:
  hydrogen, a halogen atom, a hydroxyl, a $C_1$-$C_4$ alkoxy, a $C_1$-$C_4$ alkyl, a trifluoromethyl, the said substituents being identical or different; a $C_3$-$C_7$ cycloalkyl group; a pyrimidinyl group or a pyridyl group;

  - or a group

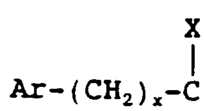

in which

  . Ar represents a phenyl, unsubstituted or substituted one or more times with one of the substituents selected from:
  hydrogen, a halogen atom, a hydroxyl, a $C_1$-$C_4$ alkoxy, a trifluoromethyl, a $C_1$-$C_4$ alkyl, the said substituents being identical or different; a pyridyl group; a thienyl group;
  . x is zero or one;
  . X represents a hydroxyl, a $C_1$-$C_4$ alkoxy; a hydroxyalkyl in which the alkyl is a $C_1$-$C_3$ group; a $C_1$-$C_4$ acyloxy; a phenacyloxy; a carboxyl; a $C_1$-$C_4$ carbalkoxy; a cyano; an aminoalkylene in which the alkylene is a $C_1$-$C_3$ group; a group -N-$(X_1)_2$ in which the groups $X_1$ independently represent hydrogen, a $C_1$-$C_4$

alkyl; a group

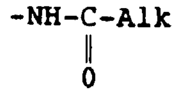

in which Alk represents a $C_1$-$C_6$ alkyl;
a group

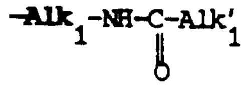

in which $Alk_1$ is a $C_1$-$C_3$ alkylene and $Alk'_1$ is a $C_1$-$C_3$ alkyl; a $C_1$-$C_4$ acyl; a group -S-$X_2$ in which $X_2$ represents hydrogen or a $C_1$-$C_4$ alkyl group; or alternatively X forms a double bond with the carbon atom to which it is bonded and with the adjacent carbon atom in the heterocycle;

- m is 2 or 3;
- Ar' represents a phenyl, unsubstituted or substituted one or more times with one of the substituents selected from: hydrogen, a halogen atom, preferably a chlorine or fluorine atom, a trifluoromethyl, a $C_1$-$C_4$ alkoxy, a $C_1$-$C_4$ alkyl, the said substituents being identical or different; a thienyl; a benzothienyl; a naphthyl; an indolyl; an indolyl N-substituted with a $C_1$-$C_3$ alkyl;
- R represents hydrogen, a $C_1$-$C_6$ alkyl;
- T represents a group selected from

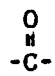 and 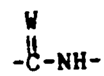

W being an oxygen or sulphur atom, and
- Z represents either hydrogen, or M or OM when T represents a

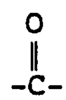

group, or M when T represents a group

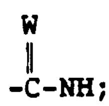

M represents a $C_1$-$C_6$ alkyl; a phenylalkyl in which the alkyl is a $C_1$-$C_3$ group, optionally substituted on the aromatic ring with a halogen, a trifluoromethyl, a $C_1$-$C_4$ alkyl, a hydroxyl, a $C_1$-$C_4$ alkoxy; a pyridyl alkyl in which the alkyl is a $C_1$-$C_3$ group; a naphthylalkyl in which the alkyl is a $C_1$-$C_3$ group, optionally substituted on the naphthyl ringsystem with a halogen, a trifluoromethyl, a $C_1$-$C_4$ alkyl, a hydroxyl, a $C_1$-$C_4$ alkoxy; a pyridylthioalkyl in which the alkyl is a $C_1$-$C_3$ group; a styryl; a phenyl group which is unsubstituted or substituted with one or more substituants selected from F; Cl; Br; I; CN; OH; $NH_2$; NH-CO-$NH_2$; $NO_2$, $CONH_2$; $CF_3$, $C_1$-$C_{10}$ alkyl; alkenyl containing from 2 to 10 carbon atoms; alkynyl containing from 2 to 10 carbon atoms; cycloalkyl containing from 3 to 8 carbon atoms; bicycloalkyl containing from 4 to 11 carbon atoms; hydroxyalkyl containing from 1 to 5 carbon atoms; alkoxy containing from 1 to 10 carbon atoms; alkoxyalkyl containing from 2 to 10 carbon atoms; alkoxyalkoxyalkyl containing up to 10 carbon atoms; alkoxyalkoxy containing from 2 to 10 carbon atoms; alkenyloxy containing from 2 to 10 carbon atoms; alkenyloxyalkyl containing up to 10 carbon atoms; alkynyloxy containing from 2 to 10 carbon atoms; alkynyloxyalkyl containing from 3 to 10 carbon atoms; cycloalkoxy containing from 3 to 8 carbon atoms; alkylthio containing from 1 to 10 carbon atoms; alkylthioalkyl containing from 2 to 10 carbon atoms; acylamino containing from 1 to 7 carbon atoms; acylaminoalkyl containing from 2 to 8 carbon atoms; acyloxy containing from 1 to 6 carbon atoms; alkoxycarbonyl containing from 2 to 5 carbon atoms; cycloalkoxycarbonyl containing from 4 to 8 carbon atoms; alkylaminocarbonylamino containing from 2 to 4 carbon atoms; dialkylaminocarbonylamino containing from 3 to 7 carbon atoms; pyrrolidinocarbonylamino; piperidinocarbonylamino; cycloalkylaminocarbonylamino containing from 4 to 8 carbon atoms; alkylaminocarbonylaminoalkyl containing from 3 to 9 carbon atoms; dialkylaminocarbonylaminoalkyl containing from 4 to 11 carbon atoms; pyrrolidinocarbonylaminoethyl, piperidinocarbonylaminoethyl; cycloalkylaminocarbonylaminoalkyl containing from 5 to 12 carbon atoms; alkoxycarbonylaminoalkyl containing from 3 to 12 carbon atoms; cycloalkoxycarbonylaminoalkyl containing from 5 to 12 carbon atoms; carbamoylalkyl containing from 2 to 5 carbon atoms; alkylaminocarbonylalkyl containing from 3 to 9 carbon atoms; dialkylaminocarbonylalkyl containing from 4 to 11 carbon atoms;
pyrrolidinocarbonylmethyl, piperidinocarbonylmethyl, piperidinocarbonylethyl; cycloalkylaminocarbonylalkyl containing from 5 to 12 carbon atoms;
alkylaminocarbonylalkoxy containing from 3 to 10 carbon atoms;
dialkylaminocarbonylalkoxy containing from 4 to 10 carbon atoms; (1-piperidinyl)carbonylmethoxy; cyclocloalkylaminocarbonylalkoxy containing from 5 to 11 carbon atoms; a 1- or 2-naphthyl group; a 1-, 2-, 3-, 4-, 5-, 6-, 7-indenyl group; in which one or bonds may be hydrogenated, it being possible for the said naphthyl or indenyl groups to be unsubstituted or optionally to contain one or more substituents such as alkyl, phenyl, cyano, hydroxyalkyl, hydroxyl, oxo, alkylcarbonylamino, alkoxycarbonyl and thioalkyl, in which the alkyls are $C_1$-$C_4$ groups; a pyridyl, thiadiazolyl, indolyl, indazolyl, imidazolyl, benzimidazolyl, benzotriazolyl, benzofuranyl, benzothienyl, benzothiazolyl, benzisothiazolyl, quinolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl, chromanyl group, of which one or more double bonds may be hydrogenated, it being possible for the said groups to be unsubstituted or optionally to contain one or more substituents such as alkyl, phenyl, cyano, hydroxyalkyl, hydroxyl, alkylcarbonylamino, alkoxycarbonyl and thioalkyl groups, in which groups the alkyls are $C_1$-$C_4$ groups;

or one of its salts with inorganic or organic acids.

**2.** Compound according to Claim 1, in which Ar' is a 3,4-dichlorophenyl group, or one of its salts with inorganic or organic acids.

**3.** Compound according to one of Claims 1 or 2, in which X is a hydroxyl, an acetyloxy or a group

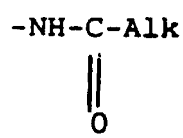

in which Alk represents a $C_1$-$C_6$ alkyl, or one of its salts with inorganic or organic acids.

**4.** Compound according to any one of claims 1 to 3, in which R is a methyl, or one of its inorganic or organic salts.

**5.** Compound according to any one of claims 1 to 4, in which T is a -C=O group, or one of its inorganic or organic salts.

**6.** Compound according to any one of claims 1 to 5, in which T is a -C=O group and Z is a thienyl group, or one of its inorganic or organic salts.

**7.** Compound according to any one of claims 1 to 5, in which T is a -C=O group and Z is a phenyl group, optionally disubstituted with a halogen such as chlorine, or one of its inorganic or organic salts.

**8.** Compound according to any one of claims 1 to 7, characterized in that it is the N-methyl-[4-(4-phenyl-4-acetylamino-piperidyl)-2-(3,4-dichlorophenyl)butyl]benzamide under the racemic form or one of its salts with organic or mineral acids.

9. (-)-N-methyl-[4-(4-phenyl-4-acetylaminopiperidyl)-2-(3,4-dichlorophenyl)butyl]benzamide and its salts with organic or mineral acids.

10. (+)-N-methyl-[4-(4-phenyl-4-acetylaminopiperidyl)-2-(3,4-dichlorophenyl)butyl]benzamide and its salts with organic or mineral acids.

11. Process for the preparation of compounds according to any one of claims 1 to 10, characterized in that

- a) a free amine of formula:

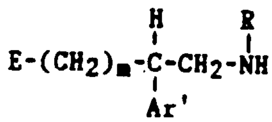 (II)

in which m, Ar' and R are as defined above in claim 1 and E represents an O-protecting group such as, for example, tetrahydro-2-pyranyloxy or a group

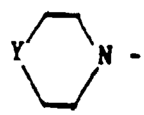

in which Y is defined as above in claim 1, on the understanding that when Y represents a group

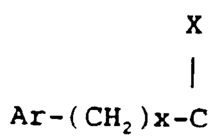

where X is a hydroxyl, this hydroxyl is protected, or an amine of formula:

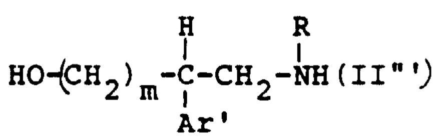

(II''')

in which m, Ar' and R are as above defined, said amine of formula (II''') being optionally under an optically pure form, is treated

- either with a functional derivative of an acid of formula

HO-CO-Z (III)

in which Z is as defined above in claim 1, when a compound of formula (I) in which T is -CO- is to be prepared,
- or with an iso(thio)cyanate of formula:

W=C=N-Z (III')

in which W and Z are as defined above in claim 1, when a compound of formula (I) in which T is -C(W)-NH- is to be prepared,
to form the compound of formula:

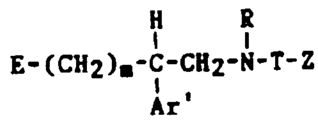

(IV)

- b) then, when E represents tetrahydropyranyloxy, the tetrahydropyranyl group is removed by the action of an acid, said deprotection being optionally carried out directly on the compound of formula (II) in order to yield a compound of formula (II''') which is then treated with one of the compounds of formula (III) or (III');
- c) the N-substituted alkanolamine thereby obtained, of formula:

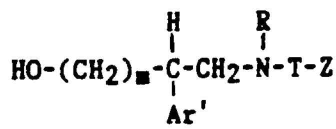

(V)

is treated with methanesulphonyl chloride
- d) the mesylate thereby obtained, of formula:

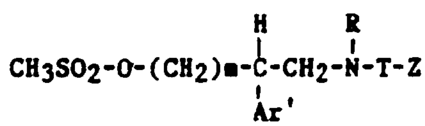

(VI)

is reacted with a secondary amine of formula:

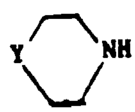

(VII)

in which Y is as defined above in claim 1; and
- e) after deprotection, where appropriate, of the hydroxyl represented by X, the product thereby obtained is optionally converted to one of its salts.

**12.** Pharmaceutical composition containing as active principle a compound of formula (I) according to any one of claims 1 to 10.

**13.** Pharmaceutical composition according to claim 12, in the form of a dosage unit, in which the active principle is mixed with at least one pharmaceutical excipient.

**Claims for the following Contracting State : GR**

**1.** A compound of formula:

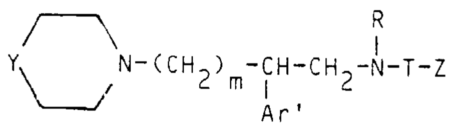 (I)

in which:

- Y represents - either a group Cy-N in which

  . Cy represents a phenyl, unsubstituted or substituted one or more times with one of the substituents selected from:
  hydrogen, a halogen atom, a hydroxyl, a $C_1$-$C_4$ alkoxy, a $C_1$-$C_4$ alkyl, a trifluoromethyl, the said substituents being identical or different; a $C_3$-$C_7$ cycloalkyl group; a pyrimidinyl group or a pyridyl group;

  - or a group

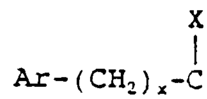

  in which

  . Ar represents a phenyl, unsubstituted or substituted one or more times with one of the substituents selected from:
  hydrogen, a halogen atom, a hydroxyl, a $C_1$-$C_4$ alkoxy, a trifluoromethyl, a $C_1$-$C_4$ alkyl, the said substituents being identical or different; a pyridyl group; a thienyl group;
  . x is zero or one;
  . X represents a hydroxyl, a $C_1$-$C_4$ alkoxy; a hydroxyalkyl in which the alkyl is a $C_1$-$C_3$ group; a $C_1$-$C_4$ acyloxy; a phenacyloxy; a carboxyl; a $C_1$-$C_4$ carbalkoxy; a cyano; an aminoalkylene in which the alkylene is a $C_1$-$C_3$ group; a group $-N-(X_1)_2$ in which the groups $X_1$ independently represent hydrogen, a $C_1$-$C_4$ alkyl; a group

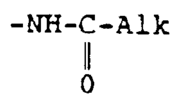

  in which Alk represents a $C_1$-$C_6$ alkyl;
  a group

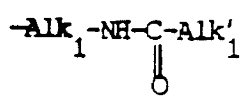

  in which $Alk_1$ is a $C_1$-$C_3$ alkylene and $Alk'_1$ is a $C_1$-$C_3$ alkyl; a $C_1$-$C_4$ acyl; a group $-S-X_2$ in which $X_2$ represents hydrogen or a $C_1$-$C_4$ alkyl group; or alternatively X forms a double bond with the carbon atom to which it is bonded and with the adjacent carbon atom in the heterocycle;

- m is 2 or 3;
- Ar' represents a phenyl, unsubstituted or substituted one or more times with one of the substituents selected from: hydrogen, a halogen atom, preferably a chlorine or fluorine atom, a trifluoromethyl, a $C_1$-$C_4$ alkoxy, a $C_1$-$C_4$ alkyl, the said substituents being identical or different; a thienyl; a benzothienyl; a naphthyl; an indolyl;

an indolyl N-substituted with a $C_1$-$C_3$ alkyl;

- R represents hydrogen, a $C_1$-$C_6$ alkyl;
- T represents a group selected from

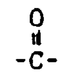

and

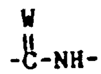

W being an oxygen or sulphur atom, and

- Z represents either hydrogen, or M or OM when T represents a

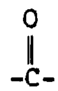

group, or M when T represents a group

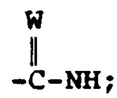

M represents a $C_1$-$C_6$ alkyl; a phenylalkyl in which the alkyl is a $C_1$-$C_3$ group, optionally substituted on the aromatic ring with a halogen, a trifluoromethyl, a $C_1$-$C_4$ alkyl, a hydroxyl, a $C_1$-$C_4$ alkoxy; a pyridyl-alkyl in which the alkyl is a $C_1$-$C_3$ group; a naphthylalkyl in which the alkyl is a $C_1$-$C_3$ group, optionally substituted on the naphthyl ringsystem with a halogen, a trifluoromethyl, a $C_1$-$C_4$ alkyl, a hydroxyl, a $C_1$-$C_4$ alkoxy; a pyridylthioalkyl in which the alkyl is a $C_1$-$C_3$ group; a styryl; a phenyl group which is unsubstituted or substituted with one or more substituants selected from F; Cl; Br; I; CN; OH; $NH_2$; NH-CO-$NH_2$; $NO_2$, $CONH_2$; $CF_3$, $C_1$-$C_{10}$ alkyl; alkenyl containing from 2 to 10 carbon atoms; alkynyl containing from 2 to 10 carbon atoms; cycloalkyl containing from 3 to 8 carbon atoms; bicycloalkyl containing from 4 to 11 carbon atoms; hydroxyalkyl containing from 1 to 5 carbon atoms; alkoxy containing from 1 to 10 carbon atoms; alkoxyalkyl containing from 2 to 10 carbon atoms; alkoxyalkoxyalkyl containing up to 10 carbon atoms; alkoxyalkoxy containing from 2 to 10 carbon atoms; alkenyloxy containing from 2 to 10 carbon atoms; alkenyloxyalkyl containing up to 10 carbon atoms; alkynyloxy containing from 2 to 10 carbon atoms; alkynyloxyalkyl containing from 3 to 10 carbon atoms; cycloalkoxy containing from 3 to 8 carbon atoms; alkylthio containing from I to 10 carbon atoms; alkylthioalkyl containing from 2 to 10 carbon atoms; acylamino containing from 1 to 7 carbon atoms; acylaminoalkyl containing from 2 to 8 carbon atoms; acyloxy containing from 1 to 6 carbon atoms; alkoxycarbonyl containing from 2 to 5 carbon atoms; cycloalkoxycarbonyl containing from 4 to 8 carbon atoms; alkylaminocarbonylamino containing from 2 to 4 carbon atoms; dialkylaminocarbonylamino containing from 3 to 7 carbon atoms; pyrrolidinocarbonylamino; piperidinocarbonylamino; cycloalkylaminocarbonylamino containing from 4 to 8 carbon atoms; alkylaminocarbonylaminoalkyl containing from 3 to 9 carbon atoms; dialkylaminocarbonylaminoalkyl containing from 4 to 11 carbon atoms; pyrrolidinocarbonylaminoethyl, piperidinocarbonylaminoethyl; cycloalkylaminocarbonylaminoalkyl containing from 5 to 12 carbon atoms; alkoxycarbonylaminoalkyl containing from 3 to 12 carbon atoms; cycloalkoxycarbonylaminoalkyl containing from 5 to 12 carbon atoms; carbamoylalkyl containing from 2 to 5 carbon atoms; alkylaminocarbonylalkyl containing from 3 to 9 carbon atoms; dialkylaminocarbonylalkyl containing from 4 to 11 carbon atoms;
pyrrolidinocarbonylmethyl, piperidinocarbonylmethyl, piperidinocarbonylethyl;
cycloalkylaminocarbonylalkyl containing from 5 to 12 carbon atoms;
alkylaminocarbonylalkoxy containing from 3 to 10 carbon atoms; dialkylaminocarbonylalkoxy containing from 4 to 10 carbon atoms; (1-piperidinyl)carbonylmethoxy; cycloalkylaminocarbonylalkoxy containing from 5 to 11 carbon atoms; a 1- or 2-naphthyl group; a 1-, 2-, 3-, 4-, 5-, 6-, 7-indenyl group; in which one or bonds may be hydrogenated, it being possible for the said naphthyl or indenyl groups to be unsubstituted or optionally to contain one or more substituents such as alkyl, phenyl, cyano, hydroxyalkyl, hydroxyl, oxo, alkylcarbonylamino, alkoxycarbonyl and thioalkyl, in which the alkyls are $C_1$-$C_4$ groups; a pyridyl, thiadiazolyl, indolyl, indazolyl, imidazolyl, benzimidazolyl, benzotriazolyl, benzofuranyl, benzothienyl, benzothiazolyl, benzisothiazolyl, quinolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl, chromanyl group, of which one or more double bonds may be hydrogenated, it being possible for the said groups to be unsubstituted or optionally to contain one or more substituents such as alkyl, phenyl, cyano, hydroxyalkyl, hydroxyl, alkylcarbonylamino, alkoxycarbonyl and thioalkyl groups, in which groups the alkyls are $C_1$-$C_4$ groups;

or one of its salts with inorganic or organic acids.

**2.** Compound according to Claim 1, in which Ar' is a 3,4-dichlorophenyl group, or one of its salts with inorganic or organic acids.

**3.** Compound according to one of Claims 1 or 2, in which X is a hydroxyl, an acetyloxy or a group

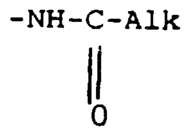

in which Alk represents a $C_1$-$C_6$ alkyl, or one of its salts with inorganic or organic acids.

**4.** Compound according to any one of claims 1 to 3, in which R is a methyl, or one of its inorganic or organic salts.

**5.** Compound according to any one of claims 1 to 4, in which T is a -C=O group, or one of its inorganic or organic salts.

**6.** Compound according to any one of claims 1 to 5, in which T is a -C=O group and Z is a thienyl group, or one of its inorganic or organic salts.

**7.** Compound according to any one of claims 1 to 5, in which T is a -C=O group and Z is a phenyl group, optionally disubstituted with a halogen such as chlorine, or one of its inorganic or organic salts.

**8.** Compound according to any one of claims 1 to 7, characterized in that it is the N-methyl-[4-(4-phenyl-4-acetylamino-piperidyl)-2-(3,4-dichlorophenyl)butyl]benzamide under the racemic form or one of its salts with organic or mineral acids.

**9.** (-)-N-methyl-[4-(4-phenyl-4-acetylaminopiperidyl)-2-(3,4-dichlorophenyl)butyl]benzamide and its salts with organic or mineral acids.

**10.** (+)-N-methyl-[4-(4-phenyl-4-acetylaminopiperidyl)-2-(3,4-dichlorophenyl)butyl]benzamide and its salts with organic or mineral acids.

**11.** Process for the preparation of compounds according to any one of claims 1 to 10, characterized in that

- a) a free amine of formula:

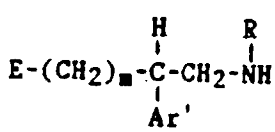

(II)

in which m, Ar' and R are as defined above in claim 1 and E represents an O-protecting group such as, for example, tetrahydro-2-pyranyloxy or a group

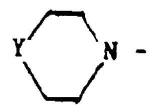

in which Y is defined as above in claim 1, on the understanding that when Y represents a group

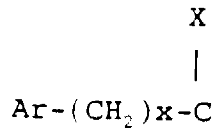

where X is a hydroxyl, this hydroxyl is protected, or an amine of formula:

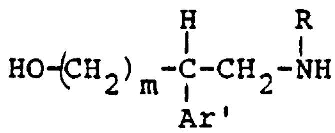 (II''')

in which m, Ar' and R are as above defined, said amine of formula (II''') being optionally under an optically pure form, is treated

- either with a functional derivative of an acid of formula

  HO-CO-Z (III)

  in which Z is as defined above in claim 1, when a compound of formula (I) in which T is -CO- is to be prepared,
- or with an iso(thio)cyanate of formula:

  W=C=N-Z (III')

  in which W and Z are as defined above in claim 1, when a compound of formula (I) in which T is -C(W)-NH- is to be prepared,
  to form the compound of formula:

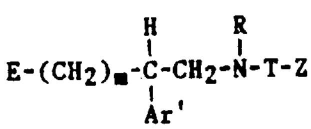 (IV)

- b) then, when E represents tetrahydropyranyloxy, the tetrahydropyranyl group is removed by the action of an acid, said deprotection being optionally carried out directly on the compound of formula (II) in order to yield a compound of formula (II''') which is then treated with one of the compounds of formula (III) or (III');
- c) the N-substituted alkanolamine thereby obtained, of formula:

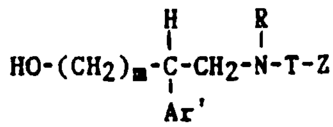

(V)

is treated with methanesulphonyl chloride

- d) the mesylate thereby obtained, of formula:

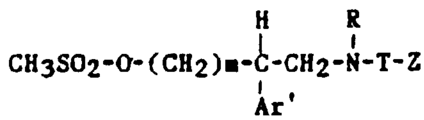

(VI)

is reacted with a secondary amine of formula:

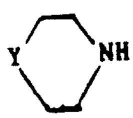

(VII)

in which Y is as defined above in claim 1; and

- e) after deprotection, where appropriate, of the hydroxyl represented by X, the product thereby obtained is optionally converted to one of its salts.

**12.** Process for the preparation of a pharmaceutical composition, characterized in that a compound according to anyone of claims 1 to 10, as active principle, is mixed with a pharmaceutically acceptable vehicle.

**Claims for the following Contracting State : ES**

**1.** A process for the preparation of compounds of formula:

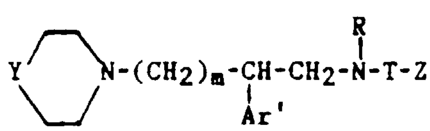

(I)

in which:

- Y represents - either a group Cy-N in which

  . Cy represents a phenyl, unsubstituted or substituted one or more times with one of the substituents selected from:
  hydrogen, a halogen atom, a hydroxyl, a $C_1$-$C_4$ alkoxy, a $C_1$-$C_4$ alkyl, a trifluoromethyl, the said substituents being identical or different; a $C_3$-$C_7$ cycloalkyl group; a pyrimidinyl group or a pyridyl group;

  - or a group

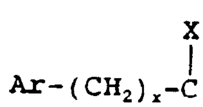

in which

. Ar represents a phenyl, unsubstituted or substituted one or more times with one of the substituents selected from: hydrogen, a halogen atom, a hydroxyl, a $C_1$-$C_4$ alkoxy, a trifluoromethyl, a $C_1$-$C_4$ alkyl, the said substituents being identical or different; a pyridyl group; a thienyl group;

. x is zero or one;

. X represents a hydroxyl, a $C_1$-$C_4$ alkoxy; a hydroxyalkyl in which the alkyl is a $C_1$-$C_3$ group; a $C_1$-$C_4$ acyloxy; a phenacyloxy; a carboxyl; a $C_1$-$C_4$ carbalkoxy; a cyano; an aminoalkylene in which the alkylene is a $C_1$-$C_3$ group; a group -N-$(X_1)_2$ in which the groups $X_1$ independently represent hydrogen, a $C_1$-$C_4$ alkyl; a group

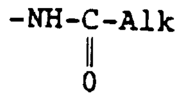

in which Alk represents a $C_1$-$C_6$ alkyl;
a group

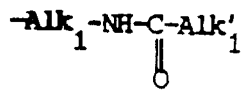

in which $Alk_1$ is a $C_1$-$C_3$ alkylene and $Alk'_1$ is a $C_1$-$C_3$ alkyl; a $C_1$-$C_4$ acyl; a group -S-$X_2$ in which $X_2$ represents hydrogen or a $C_1$-$C_4$ alkyl group; or alternatively X forms a double bond with the carbon atom to which it is bonded and with the adjacent carbon atom in the heterocycle;

- m is 2 or 3;
- Ar' represents a phenyl, unsubstituted or substituted one or more times with one of the substituents selected from: hydrogen, a halogen atom, preferably a chlorine or fluorine atom, a trifluoromethyl, a $C_1$-$C_4$ alkoxy, a $C_1$-$C_4$ alkyl, the said substituents being identical or different; a thienyl; a benzothienyl; a naphthyl; an indolyl; an indolyl N-substituted with a $C_1$-$C_3$ alkyl;
- R represents hydrogen, a $C_1$-$C_6$ alkyl;
- T represents a group selected from

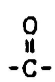 and 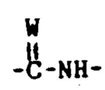

W being an oxygen or sulphur atom, and

- Z represents either hydrogen, or M or OM when T represents a

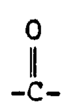

group, or M when T represents a group

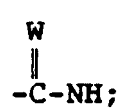

M represents a $C_1$-$C_6$ alkyl; a phenylalkyl in which the alkyl is a $C_1$-$C_3$ group, optionally substituted on the aromatic ring with a halogen, a trifluoromethyl, a $C_1$-$C_4$ alkyl, a hydroxyl, a $C_1$-$C_4$ alkoxy; a pyridyl-alkyl in which the alkyl is a $C_1$-$C_3$ group; a naphthylalkyl in which the alkyl is a $C_1$-$C_3$ group, optionally substituted on the naphthyl ringsystem with a halogen, a trifluoromethyl, a $C_1$-$C_4$ alkyl, a hydroxyl, a $C_1$-$C_4$ alkoxy; a pyridylthioalkyl in which the alkyl is a $C_1$-$C_3$ group; a styryl; a phenyl group which is unsubstituted or substituted with one or more substituants selected from F; Cl; Br; I; CN; OH; $NH_2$; NH-CO-$NH_2$; $NO_2$, $CONH_2$; $CF_3$, $C_1$-$C_{10}$ alkyl; alkenyl containing from 2 to 10 carbon atoms; alkynyl containing from 2 to 10 carbon atoms; cycloalkyl containing from 3 to 8 carbon atoms; bicycloalkyl containing from 4 to 11 carbon atoms; hydroxyalkyl containing from 1 to 5 carbon atoms; alkoxy containing from 1 to 10 carbon atoms; alkoxyalkyl containing from 2 to 10 carbon atoms; alkoxyalkoxyalkyl containing up to 10 carbon atoms; alkoxyalkoxy containing from 2 to 10 carbon atoms; alkenyloxy containing from 2 to 10 carbon atoms; alkenyloxyalkyl containing up to 10 carbon atoms; alkynyloxy containing from 2 to 10 carbon atoms; alkynyloxyalkyl containing from 3 to 10 carbon atoms; cycloalkoxy containing from 3 to 8 carbon atoms; alkylthio containing from 1 to 10 carbon atoms; alkylthioalkyl containing from 2 to 10 carbon atoms; acylamino containing from 1 to 7 carbon atoms; acylaminoalkyl containing from 2 to 8 carbon atoms; acyloxy containing from 1 to 6 carbon atoms; alkoxycarbonyl containing from 2 to 5 carbon atoms; cycloalkoxycarbonyl containing from 4 to 8 carbon atoms; alkylaminocarbonylamino containing from 2 to 4 carbon atoms; dialkylaminocarbonylamino containing from 3 to 7 carbon atoms; pyrrolidinocarbonylamino; piperidinocarbonylamino; cycloalkylaminocarbonylamino containing from 4 to 8 carbon atoms; alkylaminocarbonylaminoalkyl containing from 3 to 9 carbon atoms; dialkylaminocarbonylaminoalkyl containing from 4 to 11 carbon atoms; pyrrolidinocarbonylaminoethyl, piperidinocarbonylaminoethyl; cycloalkylaminocarbonylaminoalkyl containing from 5 to 12 carbon atoms; alkoxycarbonylaminoalkyl containing from 3 to 12 carbon atoms; cycloalkoxycarbonylaminoalkyl containing from 5 to 12 carbon atoms; carbamoylalkyl containing from 2 to 5 carbon atoms; alkylaminocarbonylalkyl containing from 3 to 9 carbon atoms; dialkylamiocarbonylalkyl containing from 4 to 11 carbon atoms; pyrrolidinocarbonylmethyl, piperidinocarbonylmethyl, piperidinocarbonylethyl; cycloalkylaminocarbonylalkyl containing from 5 to 12 carbon atoms; akylaminocarbonylalkoxy containing from 3 to 10 carbon atoms; dialkylaminocarbonylalkoxy containing from 4 to 10 carbon atoms; (1-piperidinyl)carbonylmethoxy; cycloalkylaminocarbonylalkoxy containing from 5 to 11 carbon atoms; a 1- or 2-naphthyl group; a 1-, 2-, 3-, 4-, 5-, 6-, 7-indenyl group; in which one or bonds may be hydrogenated, it being possible for the said naphthyl or indenyl groups to be unsubstituted or optionally to contain one or more substituents such as alkyl, phenyl, cyano, hydroxyalkyl, hydroxyl, oxo, alkylcarbonylamino, alkoxycarbonyl and thioalkyl, in which the alkyls are $C_1$-$C_4$ groups; a pyridyl, thiadiazolyl, indolyl, indazolyl, imidazolyl, benzimidazolyl, benzotriazolyl, benzofuranyl, benzothienyl, benzothiazolyl, benzisothiazolyl, quinolyl, isoquinolyl, benzoxazolyl, benzisoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl, chromanyl group, of which one or more double bonds may be hydrogenated, it being possible for the said groups to be unsubstituted or optionally to contain one or more substituents such as alkyl, phenyl, cyano, hydroxyalkyl, hydroxyl, alkylcarbonylamino, alkoxycarbonyl and thioalkyl groups, in which groups the alkyls are $C_1$-$C_4$ groups;

or one of its salts with inorganic or organic acids, characterized in that

- a) a free amine of formula:

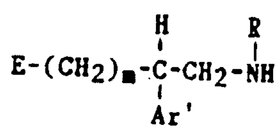

(II)

in which m, Ar' and R are as defined above and E represents an O-protecting group such as, for example, tetrahydro-2-pyranyloxy or a group

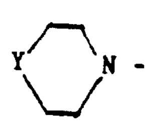

in which Y is defined as above, on the understanding that when Y represents a group

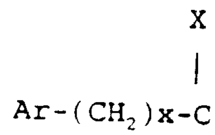

where X is a hydroxyl, this hydroxyl is protected, or an amine of formula:

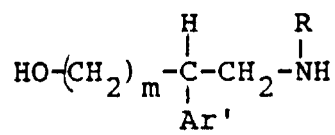

(II''')

in which m, Ar' and R are as above defined, said amine of formula (II''') being optionally under an optically pure form, is treated

- either with a functional derivative of an acid of formula

  HO-CO-Z (III)

  in which Z is as defined above, when a compound of formula (I) in which T is -CO- is to be prepared,
- or with an iso(thio)cyanate of formula:

  W=C=N-Z (III')

  in which W and Z are as defined above, when a compound of formula (I) in which T is -C(W)-NH- is to be prepared,
  to form the compound of formula:

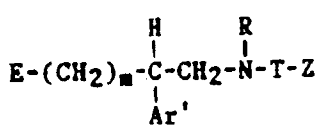

(IV)

- b) then, when E represents tetrahydropyranyloxy, the tetrahydropyranyl group is removed by the action of an acid, said deprotection being optionally carried out directly on the compound of formula (II) in order to yield a compound of formula (II''') which is then treated with one of the compounds of formula (III) or (III');
- c) the N-substituted alkanolamine thereby obtained, of formula:

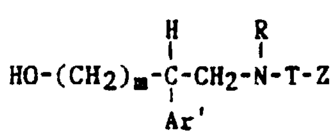

(V)

  is treated with methanesulphonyl chloride
- d) the mesylate thereby obtained, of formula:

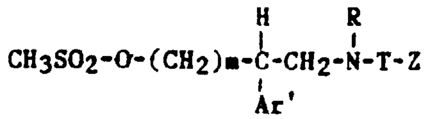

(VI)

is reacted with a secondary amine of formula:

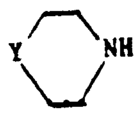

(VII)

in which Y is a defined above ; and

- e) after deprotection, where appropriate, of the hydroxyl represented by X, the product thereby obtained is optionally converted to one of its salts.

**2.** Process according to claim 1,characterized in that a free amine of formula (II), in which E, m and R are as defined in claim 1 and Ar' is 3,4-dichlorophenyl group, is used.

**3.** Process according to one of claims 1 and 2, characterized in that a free amine of formula (II), in which :

- Ar', m and R are as defined in one of claims 1 and 2,
- E represents the group :

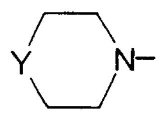

in which Y is a

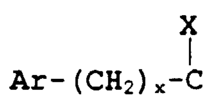

group in which : Ar and x are as defined in claim 1 and X is a hydroxyl, acetoxy group or a

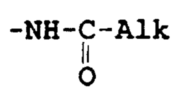

group in which Alk represents a $C_1$-$C_6$ alkyl, is used.

**4.** Process according to any one of claims 1 to 3, characterized in that a amine of formula (II), in which

- E, m and Ar' are as defined in any one of claims 1 to 3,
- R is the methyl group,

is used.

**5.** Process according to any one of claims 1 to 4, characterized in that a functional derivative of an acide of formula (III), in which Z is as defined in claim 1, is used.

**6.** Process according to claim 5, characterized in that Z is a thienyl group.

**7.** Process according to claim 5, characterized in that Z is a phenyl group, which is optionally disubstituted with a halogen such as chlorine.

**8.** Process according to claim 1, characterized in that

1) in step a), an amine of formula II in which

- E is the tetrahydropyranyloxy group;
- m is 2;
- Ar' is the 3,4-dichlorophenyl group;
- R is the methyl group,

is reacted with a compound of formula III in which

- Z is the phenyl group and

2) in step d), a secondary amine of formula (VII), in which Y is the 4-phenylacetylamino group, is used in order to form the N-methyl-[4-(4-phenyl-4-acetylaminopiperidyl)-2-(3,4-dichlorophenyl)butyl]-benzamide.

**9.** Process according to one of claims 1 and 2, characterized in that:

1) an amine of formula II in which

- E is the tetrahydropyranyloxy group;
- m is 2;
- Ar' is the 3,4-dichlorophenyl group;
- R is the methyl group,

is used;
2) this amine of formula II is deprotected in order to form the corresponding hydroxylated compound of formula (II''');
3) the resolution of the amine of formula (II''') is carried out by reaction with an optically pure acid and
4) the obtained optically pure compound is reacted with a compound of formula (III) in which Z is the phenyl group;
5) steps c), d) and e) as defined in claim 1 are carried out in order to form the following enantiomers: the (-)-N-methyl-[4-(4-phenyl-4-acetylaminopiperidyl)-2-(3,4-dichlorophenyl)butyl]benzamide and its salts with mineral or organic acids or the (+)-N-methyl-[4-(4-phenyl-4-acetylaminopiperidyl)-2-(3,4-dichlorophenyl)butyl] benzamide and its salts with mineral or organic acids.

**10.** Process for the preparation of a pharmaceutical composition, characterized in that a compound obtained by the process according to any one of claims 1 to 9, as active principle, is mixed with a pharmaceutically acceptable vehicle.